# EUROPEAN PATENT SPECIFICATION

(11) **EP 1 315 491 B1**
(45) Date of publication and mention of the grant of the patent: **09.08.2006**
(21) Application number: 01968486.9
(22) Date of filing: 05.09.2001
(51) Int. Cl.: A61K 31/00, A61K 31/13, A61K 31/275, A61K 31/4155, A61K 31/4439, A61K 31/454, A61K 31/495, A61K 31/496, A61K 31/5377, A61K 31/541, A61K 31/55, A61P 37/08

(54) **USE OF SUBSTITUTED PYRAZOLES FOR THE MANUFACTURE OF A MEDICAMENT FOR THE TREATMENT OF ALLERGIES**
VERWENDUNG VON SUBSTITUIERTEN PYRAZOLEN ZUR HERSTELLUNG EINES MEDIKAMENTS ZUR BEHANDLUNG VON ALLERGIEN
UTILISATION DE PYRAZOLES SUBSTITUES POUR L'OBTENTION D'UN MEDICAMENT DESTINE AU TRAITEMENT D'ALLERGIES

(30) Priority: 06.09.2000 US 230407 P; 10.08.2001 US 928122
(43) Date of publication of application: 04.06.2003
(73) Proprietor: Ortho-McNeil Pharmaceutical, Inc., Raritan, NJ 08869-0602 (US)
(72) Inventor: BREITENBUCHER, J., Guy, Escondido, CA 92026 (US); CAI, Hui, San Diego, CA 92130 (US); EDWARDS, James, P., San Diego, CA 92129 (US); GRICE, Cheryl, A., Carlsbad, CA 92009 (US); GU, Yin, San Diego, CA 92130 (US); GUSTIN, Darin, J., Bothell, WA 98021 (US); KARLSSON, Lars, La Jolla, CA 92037 (US); KHATUYA, Haripada, San Diego, CA 92129 (US); MEDUNA, Steven, P., San Diego, CA 92129 (US); PIO, Barbara, A., San Diego, CA 92111 (US); SUN, Siquan, San Diego, CA 92129 (US); TAYS, Kevin, L., Cardiff, CA 92007 (US); THURMOND, Robin, L., San Diego, CA 92115 (US); WEI, Jianmei, San Diego, CA 92129 (US)
(74) Representative: Fisher, Adrian John
(86) International application number: PCT/US2001/027479
(87) International publication number: WO 2002/020012

(56) References cited:
- EP-A- 0 254 241
- DATABASE CA [Online] CHEMICAL ABSTRACTS SERVICE, COLUMBUS, OHIO, US; NAKATSUKA, MASASHI ET AL: "Preparation of pyrazole derivatives as immunosuppressants" retrieved from STN Database accession no. 130:52417 XP002193692 -& WO 98 56785 A (SUMITOMO PHARMACEUTICALS CO., LTD., JAPAN) 17 December 1998 (1998-12-17)

## Description

### Field of the Invention

This invention relates to the use of substituted pyrazoles to the manufacture of a medicament for the treatment of an allergic condition.

### Background of the Invention

Atopic allergies afflict at least 20% of populations in developed countries and comprise a wide range of IgE-mediated diseases such as hay fever, asthma, atopic dermatitis, and food allergies. Exposure of an allergic subject to relevant allergens cross-links allergen specific IgE bound to mast cells, triggering degranulation and release of proinflammatory mediators, such as histamine and eicosanoids, which cause the weal-and-flare response on a skin test. Characteristically, this early response is followed by a prolonged late reaction in which inflammatory cells, particularly eosinophils and activated TH-2 CD4 T cells, are recruited to the site of allergen exposure. Inflammatory cytokines such as IL-4 and IL-5, both produced by TH-2 cells, are important for IgE production by B cells and for eosinophilia, respectively. Immunotherapies targeting CD4 T cells have been shown to be effective in reducing the production of IgE, the activation of proinflammatory cells, and the release of inflammatory mediators.

Current allergy therapies targeting CD4 T cells have met with mixed success. Desensitization with allergen extracts or vaccines is effective for many allergens, such as the *Hymenoptera* insect sting which can induce life-threatening allergic reactions. The mechanism may be either induction of T cell tolerance or the conversion of TH-2 to TH-1. However, such treatment requires a long-term treatment regime, frequent doctor visits and prior stabilization by other medications, and is associated with a certain morbidity rate and rare deaths. Alternatively, immunosuppressive drugs such as steroids which effectively stabilize ongoing allergy responses, are often associated with severe side effects.

The activation of CD4 T cells is a major factor in the initiation and maintenance of the allergic response. Allergens are taken up by specialized antigen presenting cells (APCs) such as dendritic cells and B cells. Protein allergens pass through the endosomal or lysosomal system where they are degraded by different proteases. These peptide fragments are bound by the MHC class II molecules which, at the cell surface, are heterotrimeric complexes consisting of two transmembrane glycoprotein chains (α and β) that form a binding scaffold for the third component, a peptide of 11-20 amino acids. The antigen-MHC class II molecule complex is recognized by CD4 T cells and leads to the activation of the T cell. Activated T cells in turn activate several other components of the immune system, such as B cells and macrophages, that are crucial for the body's response to pathogens, but also lead to the symptoms of allergies.

Class II molecules, like other transmembrane proteins, are translocated into the endoplasmic reticulum (ER) after synthesis, where they associate with a third protein, the invariant chain (Ii). The invariant chain molecule is a type II transmembrane protein that serves as a class II-specific chaperone, promoting the exit of class II-Ii complexes from the ER and preventing class II molecules from binding to peptides and unfolded proteins in the ER and in the secretory pathway. A targeting motif in the cytoplasmic tail of Ii directs the class II-Ii complexes from the secretory pathway into the endosomal system.

Before the MHC class II molecules can present antigen the li must be removed by a series of proteases that break down li. The resultant li peptide fragments, called class II-associated invariant chain peptides (CLIP), occupy the peptide binding groove of the class II molecule, and in most cases are not spontaneously released. The CLIP protects the class II binding pocket from collapsing both during intracellular transport and after li degradation in the endosomal system. Binding of antigenic peptides generated from endocytosed proteins requires an empty, and yet open binding site. The CLIP therefore must be released while the open binding site is stabilized to allow the binding of other peptides. Human Leukocyte Antigen - DM ('HLA-DM') mediates both of these functions, thus promoting the binding of antigenic peptides. After acquiring peptides, the class II molecules are transported to the cell surface via routes that are largely unknown.

In view of the above, inhibition of invariant chain proteolysis will prevent removal of li from the class II binding pocket, which in turn will specifically block antigen binding to the MHC class II molecule.

Cathepsin S ('CatS') is a cysteine protease expressed in lymphatic tissues. CatS mediates invariant chain proteolysis, which is a prerequisite for peptide loading of MHC class II molecules (Riese et al. (1996) Immunity 4:357). CatS has 50-60% homology with cathepsins L and K, but differs from them in that it has a broad pH optimum that extends to alkaline pH. CatS modulates antigen presentation in animal models, and inhibitors are effective in an asthma model (Riese et al. (1998) J. Clin. Invest. 101:2351). Mice deficient in cathepsin S have an impaired ability to present exogenous proteins by professional antigen presenting cells (Nakagawa et al. (1999) Immunity 10:207; Shi et al. (1999) Immunity 10:197).

Compounds that inhibit the proteolytic activity of human cathepsin S are expected to find utility in the treatment of chronic autoimmune diseases including, but not limited to, lupus and rheumatoid arthritis; and have potential utility in modulating the immune response to tissue transplantation. Methods of modulating autoimmunity with an agent that modulates cathepsin S activity, e.g., proteolysis of the li chain, as well as methods of treating a subject having an autoimmune disorder, methods of evaluating a treatment for its ability to modulate an immune response are described in WO 99/58153.

Compounds somewhat similar to those of the present invention are described in the following references.

Winters, et. al. (Winters, G.; Sala, A.; Barone, D.; Baldoli, E. *J. Med. Chem.* **1985**, *28*, 934-940; Singh, P.; Sharma, R. C. *Quant. Struct.-Act. Relat.* **1990**, *9*, 29-32; Winters, G.; Sala, A.; Barone, D. in US-4500525 (1985)) have described bicyclic pyrazoles of the type shown below. R never contains a heterocyclic ring and no protease inhibitor activity is ascribed to these molecules; they are described as α1-adrenergic receptor modulators.

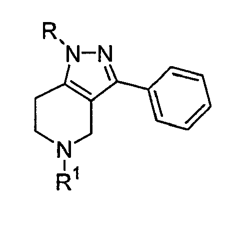

Shutske, et. al. claim the bicylic pyrazoles below. The pyridine ring is aromatic in their system (Shutske, G. M.; Kapples, K. J.; Tomer, J. D. US-5264576 (1993)). Although reference is made to R being a linker to a heterocycle, the claims specify only R = hydrogen. The compounds are referred to as serotonin reuptake inhibitors.

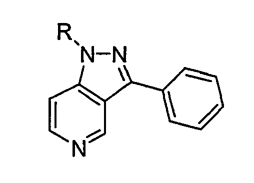

The compound 2-[4-[4-(3-methyl-5-phenyl-1H-pyrazol-1-yl)butyl]-1-piperazinyl]-pyrimidine is known from EP-382637, which describes pyrimidines having anxiolytic properties. This compound and analogs are further described in EP-502786 as cardiovascular and central nervous system agents. Pharmaceutical formulations with such compounds are disclosed in EP-655248 for use in the treatment of gastric secreation and as anti-ulcer agents. WO-9721439 describes medicaments with such compounds for treating obsessive-compulsive disorders, sleep apnea, sexual dysfunctions, emesis and motion sickness.

The compounds 5-methyl-3-phenyl-1-[4-(4-phenyl-1-piperazinyl)butyl]-1H-indazole and 5-bromo-3-(2-chlorophenyl)-1-[4-(4-phenyl-1-piperazinyl)butyl]-1H-indazole, in particular the hydrochloride salts thereof, are known from WO-9853940 and CA 122:314528, where these and similar compounds are described as kinase inhibitors in the former reference and possessing affinity for benzodiazepine receptors in the latter reference.

### Summary of the Invention

The present invention features the use of cathepsin S inhibitors for the manufacture of a medicament to treat allergic conditions, including atopic allergies. Examples of an allergic condition include hay fever, asthma, atopic dermatitis and food allergies. Allergens include dust, pollen, mold, and pet dander or pet hair.

In one aspect, the invention provides the use of a therapeutically effective amount of a pharmaceutical composition comprising a cathepsin S inhibitor for the manufacture of a medicament for treating a subject suffering from an allergic condition, in particular an atopic allergic condition.

In another aspect, the invention provides the use of a therapeutically effective amount of a pharmaceutical composition comprising a cathepsin S inhibitor for the manufacture of a medicament for treating a subject suffering from an IgE-mediated allergic condition, in particular an atopic allergic condition.

A third aspect of the invention provides the use for the manufacture of a medicament, of a cathepsin S inhibitor for treating an allergic condition, more in particular for treating IgE-mediated allergic conditions, still more in particular treating hay fever, asthma, atopic dermatitis or food allergies. The invention also features anti-allergic pharmaceutical compositions comprising as active ingredient an effective amount of a cathepsin S inhibitor, and a pharmaceutically acceptable carrier. The active ingredient can be formulated in any manner suitable for the particular allergic condition, including aerosol, oral and topical formulations and time-release formulations.

The present invention concerns the treatment of an allergic condition using one or more compounds which can be represented by formula (I): 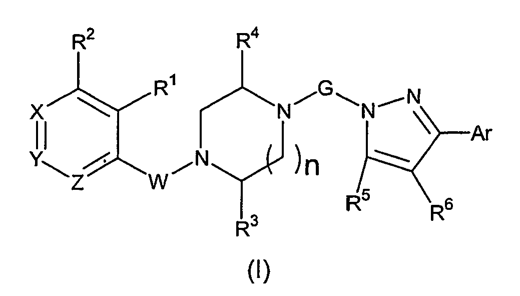 wherein:
- R¹: is hydrogen, azido, halogen, C₁₋₅ alkoxy, hydroxy, C₁₋₅ alkyl, C₂₋₅alkenyl, cyano, nitro, R⁷R⁸N, C₂₋₈ acyl, R⁹OC=O, R¹⁰R¹¹NC=O, or R¹⁰R¹¹NSO₂; or R¹ is taken together with W as described below;
- R²: is hydrogen, halogen, C₁₋₅ alkoxy, C₁₋₅ alkyl, C₂₋₅ alkenyl, C₁₋₅ haloalkyl, cyano, or R⁴⁸R⁴⁹N; alternatively, R¹ and R² can be taken together to form an optionally substituted 5- to 7- membered carbocyclic or heterocyclic ring, which ring may be unsaturated or aromatic;
- each: of R³ and R⁴ is independently hydrogen or C₁₋₅ alkyl;
- each: of R⁵ and R⁶ is independently hydrogen, C₁₋₅ alkyl, C₂₋₅ alkenyl, C₁₋₅ alkoxy, C₁₋₅ alkylthio, halogen, or a 4-7 membered carbocyclyl or heterocyclyl;
- alternatively,: R⁵ and R⁶ can be taken together to form an optionally substituted 5- to 7- membered carbocyclic or heterocyclic ring, which ring may be unsaturated or aromatic, and may be optionally substituted with between one and three substituents independently selected from halo, cyano, amino, nitro, R⁴⁰, R⁴⁰O-, R⁴⁰S-, R⁴⁰O(C ₁₋₅ alkylene)-, R⁴⁰O(C=O)-, R⁴⁰(C=O)-, R⁴⁰(C=S)-, R⁴⁰(C=O)O-, R⁴⁰O(C=O)(C=O)-, R⁴⁰SO₂, NHR⁶²(C=NH)-, NHR⁶²SO₂-, and NHR⁶²(C=O)-;
- R⁴⁰: is H, C ₁₋₅ alkyl, C₂₋₅ alkenyl, phenyl, benzyl, phenethyl, C ₁₋₅ heterocyclyl, (C ₁₋₅ heterocyclyl)C ₁₋₅ alkylene, amino, or mono- or di(C₁₋₅ alkyl)amino, or R⁵⁸OR⁵⁹-, wherein R⁵⁸ is H, C₁₋₅ alkyl, C ₂₋₅ alkenyl, phenyl, benzyl, phenethyl, C ₁₋₅ heterocyclyl, or (C ₁₋₅ heterocyclyl)C ₁₋₆ alkylene and R⁵⁹ is C ₁₋₅ alkylene, phenylene, or divalent C ₁₋₅ heterocyclyl; and
- R⁶²: can be H in addition to the values for R⁴⁰;
- R⁷: is hydrogen, C₁₋₅ alkyl, C₃₋₅ alkenyl, phenyl, naphthyl, C ₁₋₅ heterocyclyl, C₂₋₈ acyl, aroyl, R²⁷OC=O, R²⁸R²⁹NC=O, R²⁷SO, R²⁷SO₂, or R²⁸R²⁹NSO₂;
- R⁸: is hydrogen, C₁₋₅ alkyl, C₃₋₅ alkenyl, phenyl, or C ₁₋₅ heterocyclyl; alternatively, R⁷ and R⁸ can be taken together to form an optionally substituted 4- to 7- membered heterocyclic ring, which ring may be saturated, unsaturated or aromatic;
- R⁹: is C₁₋₅alkyl, phenyl, naphthyl, or C ₁₋₅ heterocyclyl;
- R²¹: is hydrogen, C₁₋₅ alkyl, C₃₋₅ alkenyl, phenyl, naphthyl, C ₁₋₅ heterocyclyl, C₂₋₈acyl, aroyl, R³⁰OC=O, R³¹R³²NC=O, R³⁰SO, R³⁰SO₂, or R³¹R³²NSO₂;
- R²²: is hydrogen, C₁₋₅alkyl, C₃₋₅ alkenyl, phenyl, or C ₁₋₅ heterocyclyl; alternatively, R²¹ and R²²can be taken together to form an optionally substituted 4- to 7-membered heterocyclic ring, which ring may be saturated, unsaturated or aromatic;
- each: of R²³, R²⁶, R²⁷, R³⁰, R³³, R⁴⁴, R⁴⁵, and R⁵⁰ is C₁₋₅ alkyl, phenyl, naphthyl, or C₁₋₅ heterocyclyl;
- R²⁴: is hydrogen, C₁₋₅ alkyl, C₃₋₅ alkenyl, phenyl, naphthyl, C ₁₋₅ heterocyclyl, C₂₋₈ acyl, aroyl, R³³OC=O, R³⁴R³⁵NC=O, R³³SO , R³³SO₂, or R³⁴R³⁵NSO₂;
- R²⁵: is hydrogen, C₁₋₅ alkyl, C₃₋₅ alkenyl, phenyl, or C ₁₋₅ heterocyclyl; alternatively, R²⁴ and R²⁵ can be taken together to form an optionally substituted 4- to 7- membered heterocyclic ring, which ring may be saturated, unsaturated or aromatic;
- each: of R¹⁰ and R¹¹ is independently hydrogen, C₁₋₅ alkyl, C₂₋₅ alkenyl, phenyl, or C ₁₋₅ heterocyclyl; alternatively, R¹⁰ and R¹¹ or can be taken together to form an optionally substituted 4- to 7- membered heterocyclic ring, which ring may be saturated, unsaturated or aromatic;
- each: of R²⁸, R²⁹, R³¹, R³², R³⁴, R³⁵, R⁴⁶, R⁴⁷, R⁵¹ and R⁵² is independently hydrogen, C₁₋₅ alkyl, phenyl, or C ₁₋₅ heterocyclyl; alternatively, R²⁸ and R²⁹, R³¹ and R³², R³⁴ and R³⁵, R⁴⁶ and R⁴⁷, or R⁵¹ and R⁵², independently, can be taken together to form an optionally substituted 4- to 7- membered heterocyclic ring, which ring may be saturated, unsaturated or aromatic;
- n: is 1 or 2;
- G: represents C₃₋₆ alkenediyl or C₃₋₆ alkanediyl, optionally substituted with hydroxy, halogen, C₁₋₅alkyl, C₁₋₅alkoxy, oxo, hydroximino, CO₂R⁶⁰, R⁶⁰R⁶¹NCO₂, (L)-C₁₋₄ alkylene-, (L)-C₁₋₅ alkoxy, N₃, or [(L)-C ₁₋₅ alkylene]amino;
- each: of R⁶⁰ and R⁶¹ is independently hydrogen, C₁₋₅ alkyl, C₃₋₅ alkenyl, phenyl, benzyl, phenethyl, or C ₁₋₅ heterocyclyl; alternatively R⁶⁰ and R⁶¹, can be taken together to form an optionally substituted 4- to 7- membered heterocyclic ring, which ring may be saturated, unsaturated or aromatic;
- L: is amino, mono- or di-C₁₋₅alkylamino, pyrrolidinyl, morpholinyl, piperidinyl homopiperidinyl, or piperazinyl, where available ring nitrogens may be optionally substituted with C₁₋₅ alkyl, benzyl, C₂₋₅acyl, C₁₋₅ alkylsulfonyl or C₁₋₅ alkyloxycarbonyl;
- X: is nitrogen or R¹²C;
- Y: is nitrogen or R¹³C;
- Z: is nitrogen or R⁴C;
- R¹²: is hydrogen, halogen, C₁₋₅ alkoxy, C₁₋₅ alkyl, C₂₋₅ alkenyl, cyano, nitro, R²¹R²²N, C₂₋₈acyl, C₁₋₅haloalkyl, C₁₋₅ heterocyclyl, (C ₁₋₅ heterocyclyl)C ₁₋₅ alkylene, R²³OC=O, R²³O(C=O)NH-, R²³SO, R²²NHCO-, R²²NH(C=O)NH-, R²³(C₁₋₄ alkylene)NHCO-, R²³SO₂, or R²³SO₂NH-;
- R¹³: is hydrogen, halogen, C₁₋₅ alkoxy, C₁₋₅ alkyl, C₂₋₅ alkenyl, cyano, nitro, R⁴²R⁴³N, C ₂₋₈ acyl, C₁₋₅ haloalkyl, C ₁₋₅ heterocyclyl, (C ₁₋₅ heterocyclyl)C ₁₋₅ alkylene, R⁴⁴OC=O, R⁴⁴O(C=O)NH-, R⁴⁴SO, R⁴³NHCO-, R⁴³NH(C=O)NH-, R⁴⁴(C ₁₋₄ alkylene)NHCO-, R⁴⁴SO₂, or R⁴⁴SO₂NH-;
- R¹⁴: is hydrogen, halogen, C₁₋₅ alkoxy, C₁₋₅ alkyl, C₂₋₅ alkenyl, cyano, nitro, R²⁴R²⁵N, C ₂₋₈ acyl, C₁₋₅ haloalkyl, C ₁₋₅ heterocyclyl, (C ₁₋₅ heterocyclyl)C ₁₋₅ alkylene, R²⁶OC=O, R²⁶O(C=O)NH-, R²⁶SO, R²⁵ NHCO-, R²⁵NH(C=O)NH-, R²⁶(C ₁₋₄ alkylene)NHCO-, R²⁶SO₂, or R²⁶SO₂NH-; alternatively, R¹² and R¹³ or R¹² and R² or R¹³ and R¹⁴ can be taken together to form an optionally substituted 5- to 6- membered carbocyclic or heterocyclic ring, which ring may be unsaturated or aromatic;
- Ar: represents a monocyclic or bicyclic aryl or heteroaryl ring, optionally substituted with between 1 and 3 substituents selected from halogen, C₁₋₅ alkoxy, C₁₋₅ alkyl, C₂₋₅ alkenyl, cyano, azido, nitro, R¹⁵R¹⁶N, R¹⁷SO₂, R¹⁷S R¹⁷SO, R¹⁷OC=O, R¹⁵R¹⁶ NC=O, C₁₋₅ haloalkyl, C₁₋₅ haloalkoxy, C₁₋₅ haloalkylthio, and C₁₋₅ alkylthio;
- R¹⁵: is hydrogen, C₁₋₅alkyl, C₃₋₅ alkenyl, phenyl, benzyl, C ₁₋₅ heterocyclyl, C ₂₋₈ acyl, aroyl, R⁵³OC=O, R⁵⁴R⁵⁵NC=O, R⁵³S, R⁵³SO, R⁵³SO₂, or R⁵⁴R⁵⁵NSO₂;
- R¹⁶: is hydrogen, C₁₋₅alkyl, C₃₋₅ alkenyl, phenyl, benzyl, or C ₁₋₅ heterocyclyl; alternatively, R¹⁵ and R¹⁶ can be taken together to form an optionally substituted 4- to 7- membered heterocyclic ring, which ring may be saturated, unsaturated or aromatic;
- each: of R¹⁷ and R⁵³ is C₁₋₅ alkyl, phenyl, or C ₁₋₅ heterocyclyl;
- each: of R⁵⁴ and R⁵⁵ is independently hydrogen, C₁₋₅ alkyl, C₂₋₅ alkenyl, phenyl, benzyl, or C₁₋₅ heterocyclyl; alternatively, R⁵⁴ and R⁵⁵ can be taken together to form an optionally substituted 4- to 7- membered heterocyclic ring, which ring may be saturated, unsaturated or aromatic;
- W: represents SO₂, C=O, CHR²⁰, or a covalent bond; or W and R¹, taken together with the 6-membered ring to which they are both attached, form one of the following two formulae: 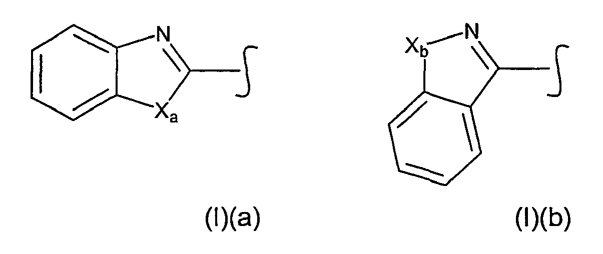 wherein Xₐ is O, S, or N; and X_{b} is O, S or SO₂
- R²⁰: is hydrogen, C₁₋₅ alkyl, phenyl, benzyl, naphthyl, or C ₁₋₅ heterocyclyl;
- R⁴²: is hydrogen, C₁₋₅ alkyl, C₃₋₅ alkenyl, phenyl, naphthyl, C ₁₋₅ heterocyclyl, C ₂₋₈ acyl, aroyl, R⁴⁵OC=O, R⁴⁶R⁴⁷NC=O, R⁴⁵SO, R⁴⁵SO₂, or R⁴⁶R⁴⁷NSO₂;
- R⁴³: is hydrogen, C₁₋₅ alkyl, C₃₋₅ alkenyl, phenyl, or C₁₋₅ heterocyclyl; alternatively, R⁴² and R⁴³can be taken together to form an optionally substituted 4- to 7- membered heterocyclic ring, which ring may be saturated, unsaturated or aromatic;
- R⁴⁴: is C₁₋₅alkyl, C₂₋₅ alkenyl, phenyl, naphthyl, or C₁₋₅ heterocyclyl;
- R⁴⁸: is hydrogen, C₁₋₅ alkyl, C₃₋₅ alkenyl, phenyl, naphthyl, C₁₋₅ heterocyclyl, C 2-8 acyl, aroyl, R⁵⁰OC=O, R⁵¹R⁵²NC=O, R⁵⁰SO, R⁵⁰SO₂, or R⁵¹R⁵²NSO₂;
- R⁴⁹: is hydrogen, C₁₋₅ alkyl, C₃₋₅ alkenyl, phenyl, or C ₁₋₅ heterocyclyl; alternatively, R⁴⁸ and R⁴⁹ can be taken together to form an optionally substituted 4- to 7- membered heterocyclic ring, which ring may be saturated, unsaturated or aromatic; and
wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅ alkyl, C₁₋₅ alkoxy, -COOH, C ₂₋₆ acyl, [di(C ₁₋₄ alkyl)amino]C ₂₋₅ alkylene, [di(C ₁₋₄ alkyl)amino] C ₂₋₅ alkyl-NH-CO-, and C ₁₋₅ haloalkoxy;
or a pharmaceutically acceptable salt, ester, or amide thereof, including a stereoisomeric form thereof.

The disclosed compounds are high-affinity inhibitors of the proteolytic activity of human cathepsin S. For use in medicine, the preparation of pharmaceutically acceptable salts of compounds of formula (I) may be desirable.

Certain compounds of the present invention may have one stereogenic atom and may exist as two enantiomers. Certain compounds of the present invention may have two or more stereogenic atoms and may further exist as diastereomers. It is to be understood by those skilled in the art that all such stereoisomers and mixtures thereof in any proportion are encompassed within the scope of the present invention.

Another aspect of the invention provides pharmaceutical anti-allergic compositions comprising a compound of formula (I) and a pharmaceutically acceptable carrier. A further embodiment of the invention is a process for making an anti-allergic pharmaceutical composition comprising mixing a disclosed compound as described above, with a suitable pharmaceutically acceptable carrier.

The invention also contemplates pharmaceutical compositions comprising more than one compound of formula (I) and compositions comprising a compound of formula (I) and another pharmaceutically active agent.

The invention features a method of treating allergic disorders or conditions mediated by the cathepsin S enzyme, in a subject in need thereof, comprising administering to the subject a therapeutically effective amount of any of the compounds or pharmaceutical compositions described above. If more than one active agent is administered, the therapeutically effective amount may be a jointly effective amount. The compounds described herein inhibit the protease activity of human cathepsin S, an enzyme involved in the immune response. In preferred embodiments, cathepsin S inhibition is selective. As such, the disclosed compounds and compositions are useful in the prevention, inhibition, or treatment of allergic conditions, particularly atopic allergic conditions.

Additional features and advantages of the invention will become apparent from the detailed description and examples below, and the appended claims.

### Brief Description of the Figures

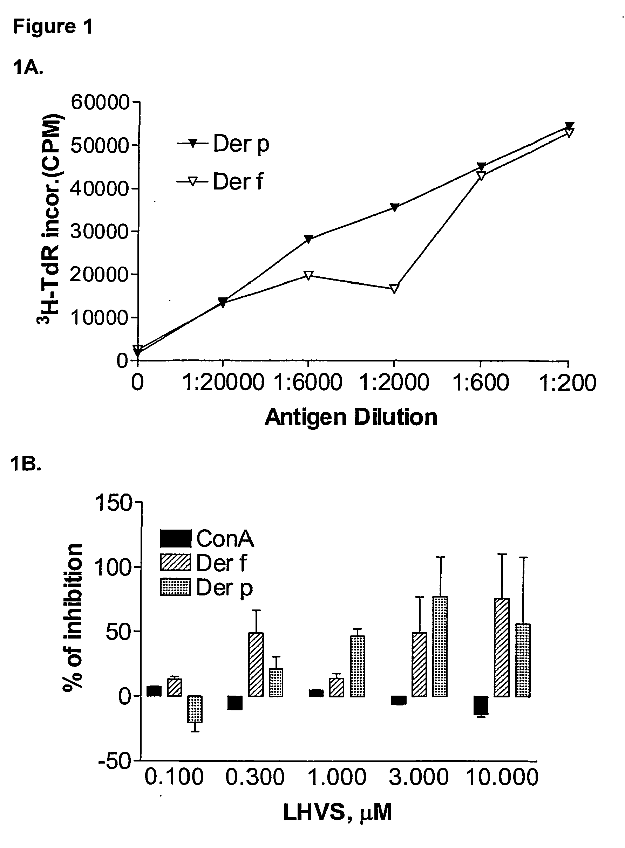

FIG. 1 shows the inhibition of human T cell proliferative responses to two species of dust mites, *Der p* and *Der f.* Top panel, FIG. 1A: Dilution curve for purified PBMC from an allergy donor were cultured with titrated doses of allergen extracts prepared from *Der p* and *Der f* for seven days. Proliferation of T cells was scored by measuring ³H-thymidine incorporation for 18 h at the end of culture. Bottom panel, FIG. 1B: Effect of titrated doses of LHVS on proliferative responses of T cells to dust mite extracts.

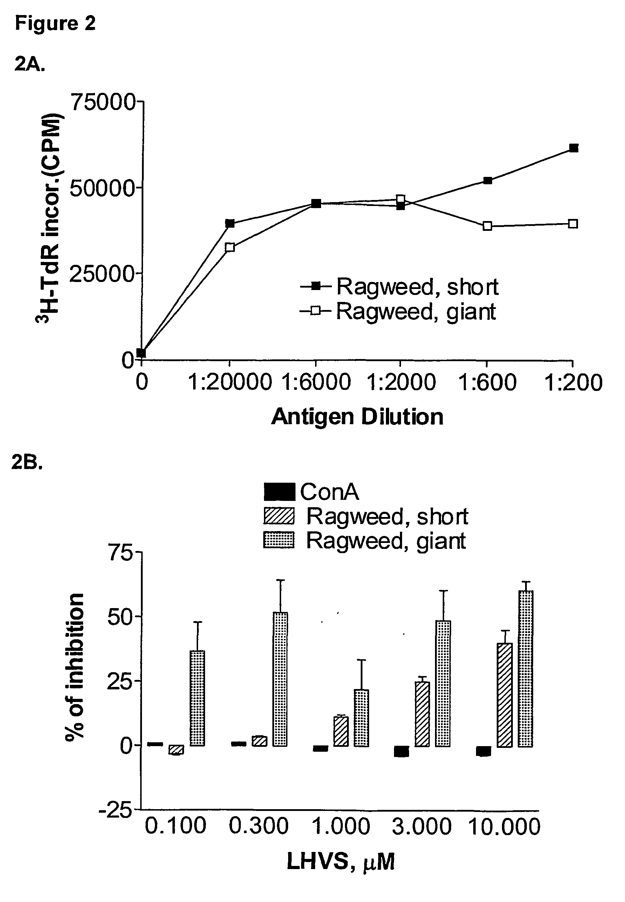

FIG. 2 is shows the inhibition of human T cell proliferative responses to ragweeds but not ConA by LHVS. Top panel, FIG. 2A: Dilution curve for purified PBMC from an allergy donor were cultured with titrated doses of allergen extracts prepared from Ragweed short and Ragweed giant for seven days. Proliferation of T cells was scored by measuring ³H-thymidine incorporation for 18 h at the end of culture. Bottom panel, FIG. 2B: Effect of titrated doses of LHVS on proliferative responses of T cells to ragweed extracts.

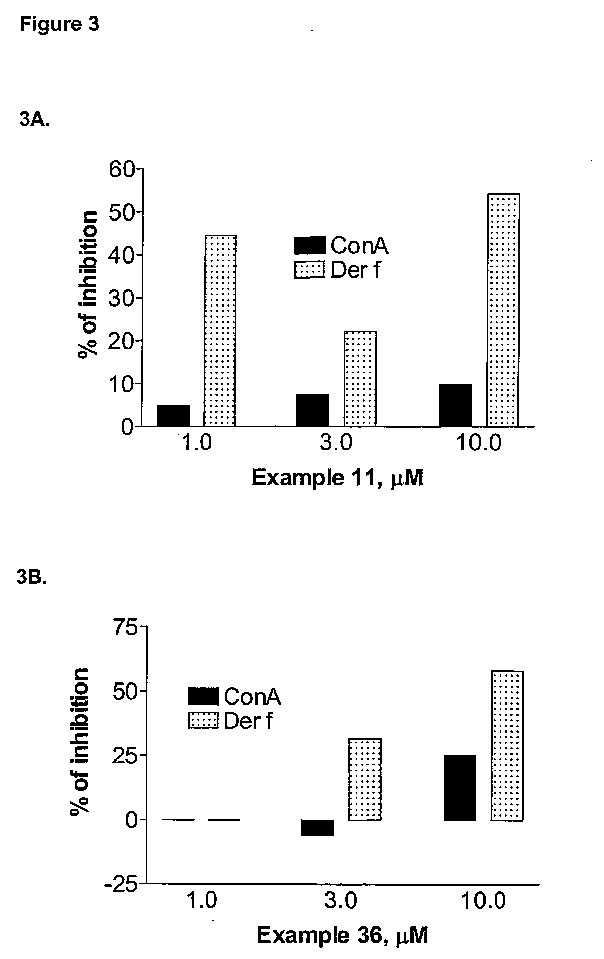

FIG. 3 shows the inhibition of human T cell proliferative responses to *Der f* but not ConA by two Cathepsin S inhibitors, Example 11 (FIG. 3A) and Example 36 (FIG. 3B). Purified PBMC from an allergy donor were cultured with allergen extracts prepared from *Der f* in the presence of titrated doses of indicated example compounds for seven days. Proliferation of T cells was scored by measuring ³H-thymidine incorporation for 18 h at the end of culture.

### Detailed Description of the Invention

A target of the present invention was to determine whether the presentation of particular antigens in a human system is affected by the inhibition of cathepsin S. According to the invention, it now has been found that inhibitors of cathepsin S block the presentation of several crude allergen extracts in a human ex *vivo* assay, thereby supporting the use of cathepsin S inhibitors for the treatment of such allergic conditions.

Blocking li degradation should decrease antigen presentation to CD4 T cells and disrupt the normal immune response. A cathepsin S inhibitor should specfically affect the activation of CD4 T cells, thus limiting the extent of concomitant immunosuppression, an undesirable side effect of corticosteroid therapy.

By using cathepsin S inhibitors according to the methods of the present invention, the immunological component of the allergic reaction can be blocked to varying degrees, with the advantage over current therapies of being more selective, having fewer or reduced side effects, or both. The present invention is based, in part, on the finding that cathepsin S inhibitors can block the presentation of crude allergen extracts in a human *ex vivo* assay. This *ex vivo* system closely mimics the process that occurs in the whole body wherein antigens enter the blood stream,and are presented by antigen presenting cells, which in turn activate CD4 T cells. In the case of treating a subject, the inhibitor or a metabolite thereof would also be present in the blood as in the ex *vivo* assay.

The invention features the treatment of an allergic condition using pyrazole compounds of formula (I).

### A. Terms

The following terms are defined below and by their usage throughout this disclosure.

"Alkyl" includes optionally substituted straight chain and branched hydrocarbons with at least one hydrogen removed to form a radical group. Alkyl groups include methyl, ethyl, propyl, isopropyl, butyl, isobutyl, t-butyl, 1-methylpropyl, pentyl, isopentyl, sec-pentyl, hexyl, heptyl, octyl, and so on. Alkyl includes cycloalkyl, such as cyclopropyl, cyclobutyl, cyclopentyl, and cyclohexyl.

"Alkenyl" includes optionally substituted straight chain and branched hydrocarbon radicals as above with at least one carbon-carbon double bond (sp²). Alkenyls include ethenyl (or vinyl), prop-1-enyl, prop-2-enyl (or allyl), isopropenyl (or 1-methylvinyl), but-1-enyl, but-2-enyl, butadienyls, pentenyls, hexa-2,4-dienyl, and so on. Hydrocarbon radicals having a mixture of double bonds and triple bonds, such as 2-penten-4-ynyl, are grouped as alkynyls herein. Alkenyl includes cycloalkenyl. *Cis* and *trans* or (E) and (Z) forms are included within the invention.

"Alkynyl" includes optionally substituted straight chain and branched hydrocarbon radicals as above with at least one carbon-carbon triple bond (sp). Alkynyls include ethynyl, propynyls, butynyls, and pentynyls. Hydrocarbon radicals having a mixture of double bonds and triple bonds, such as 2-penten-4-ynyl, are grouped as alkynyls herein. Alkynyl does not include cycloalkynyl.

"Alkoxy" includes an optionally substituted straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and SO₂. Heteroalkyl includes alkoxy, aminoalkyl, thioalkyl, and so on.

"Aryl" includes phenyl, naphthyl, biphenylyl, tetrahydronaphthyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on.

"Heterocyclyl" includes optionally substituted aromatic and nonaromatic rings having carbon atoms and at least one heteroatom (O, S, N) or heteroatom moiety (SO₂, CO, CONH, COO) in the ring. Unless otherwise indicated, a heterocyclic radical may have a valence connecting it to the rest of the molecule through a carbon atom, such as 3-furyl or 2-imidazolyl, or through a heteroatom, such as N-piperidyl or 1-pyrazolyl. Preferably a monocyclic heterocyclyl has between 4 and 7 ring atoms, or between 5 and 6 ring atoms; there may be between 1 and 5 heteroatoms or heteroatom moieties in the ring, and preferably between 1 and 3. A heterocyclyl may be saturated, unsaturated, aromatic (e.g., heteroaryl), nonaromatic, or fused.

Heterocyclyl also includes fused, e.g., bicyclic, rings, such as those optionally condensed with an optionally substituted carbocyclic or heterocyclic five- or six-membered aromatic ring. For example, "heteroaryl" includes an optionally substituted six-membered heteroaromatic ring containing 1, 2 or 3 nitrogen atoms condensed with an optionally substituted five- or six-memebered carbocyclic or heterocyclic aromatic ring. Said heterocyclic five- or six-membered aromatic ring condensed with the said five- or six-membered aromatic ring may contain 1, 2 or 3 nitrogen atoms where it is a six-membered ring, or 1, 2 or 3 heteroatoms selected from oxygen, nitrogen and sulfur where it is a five-membered ring.

Examples of heterocyclyls include thiazoylyl, furyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imdazolidinyl, imidazolinyl, pyrazolidinyl, pyrazolinyl, piperidyl, piperazinyl, indolinyl, and morpholinyl. For example, preferred heterocyclyls or heterocyclic radicals include morpholinyl, piperazinyl, pyrrolidinyl, pyridyl, cyclohexylimino, cycloheptylimino,and more preferably, piperidyl.

Examples illustrating heteroaryl are thienyl, furanyl, pyrrolyl, imidazolyl, oxazolyl, thiazolyl, benzothienyl, benzofuranyl, benzimidazolyl, benzoxazolyl, benzothiazolyl.

"Acyl" refers to a carbonyl moiety attached to either a hydrogen atom (i.e., a formyl group) or to an optionally substituted alkyl or alkenyl chain, or heterocyclyl.

"Halo" or "halogen" includes fluoro, chloro, bromo, and iodo, and preferably chloro or bromo as a substituent.

"Alkanediyl" or "alkylene" represents straight or branched chain optionally substituted bivalent alkane radicals such as, for example, methylene, ethylene, propylene, butylene, pentylene or hexylene.

"Alkenediyl" represents, analogous to the above, straight or branched chain optionally substituted bivalent alkene radicals such as, for example, propenylene, butenylene, pentenylene or hexenylene. In such radicals, the carbon atom linking a nitrogen preferably should not be unsaturated.

"Aroyl" refers to a carbonyl moiety attached to an optionally substituted aryl or heteroaryl group, wherein aryl and heteroaryl have the definitions provided above. In particular, benzoyl is phenylcarbonyl.

As defined herein, two radicals, together with the atom(s) to which they are attached may form an optionally substituted 4- to 7-, 5 - to 7-, or a 5- to 6-membered ring carbocyclic or heterocyclic ring, which ring may be saturated, unsaturated or aromatic. Said rings may be as defined above in the Summary of the Invention section. Particular examples of such rings are as follows in the next section.

"Pharmaceutically acceptable salts, esters, and amides" include carboxylate salts (e.g., C ₁₋₈ alkyl, cycloalkyl, aryl, heteroaryl, or non-aromatic heterocyclic) amino acid addition salts, esters, and amides which are within a reasonable benefit/risk ratio, pharmacologically effective and suitable for contact with the tissues of patients without undue toxicity, irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19. Representative pharmaceutically acceptable amides of the invention include those derived from ammonia, primary C₁₋₆alkyl amines and secondary di (C₁₋₆alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃alkyl primary amines, and di (C ₁₋₂ alkyl)amines. Representative pharmaceutically acceptable esters of the invention include C ₁₋₇ alkyl, C ₅₋₇ cycloalkyl, phenyl, and phenyl(C ₁₋₆)alkyl esters. Preferred esters include methyl esters.

"Patient" or "subject" includes mammals such as humans and animals (dogs, cats, horses, rats, rabbits, mice, non-human primates) in need of observation, experiment, treatment or prevention in connection with the relevant disease or condition. Preferably, the patient or subject is a human.

"Composition" includes a product comprising the specified ingredients in the specified amounts as well as any product which results directly or indirectly from combinations of the specified ingredients in the specified amounts.

"Therapeutically effective amount" or "effective amount" means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Concerning the various radicals in this disclosure and in the claims, three general remarks are made. The first remark concerns valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent). An example of a bivalent radical linking two parts of the molecule is G in formula (I) which links two rings.

Second, radicals or structure fragments as defined herein are understood to include substituted radicals or structure fragments. Hydrocarbyls include monovalent radicals containing carbon and hydrogen such as alkyl, alkenyl, alkynyl, cycloalkyl, and cycloalkenyl (whether aromatic or unsaturated), as well as corresponding divalent radicals such as alkylene, alkenylene, phenylene, and so on. Heterocarbyls include monovalent and divalent radicals containing carbon, hydrogen, and at least one heteroatom. Examples of monovalent heterocarbyls include acyl, acyloxy, alkoxyacyl, heterocyclyl, heteroaryl, aroyl, benzoyl, dialkylamino, hydroxyalkyl, and so on. Using "alkyl" as an example, "alkyl" should be understood to include substituted alkyl having one or more substitutions, such as between 1 and 5, 1 and 3, or 2 and 4 substituents. The substituents may be the same (dihydroxy, dimethyl), similar (chlorofluoro), or different (chlorobenzyl- or aminomethyl-substituted). Examples of substituted alkyl include haloalkyl (such as fluoromethyl, chloromethyl, difluoromethyl, perchloromethyl, 2-bromoethyl, perfluoromethyl, and 3-iodocyclopentyl), hydroxyalkyl (such as hydroxymethyl, hydroxyethyl, 2-hydroxypropyl, aminoalkyl (such as aminomethyl, 2-aminoethyl, 3-aminopropyl, and 2-aminopropyl), nitroalkyl, alkylalkyl, and so on. A di(C ₁₋₆ alkyl)amino group includes independently selected alkyl groups, to form, for example, methylpropylamino and isopropylmethylamino, in addition dialkylamino groups having two of the same alkyl group such as dimethyl amino or diethylamino.

Third, only stable compounds are intended. For example, where there is an NR'R" group, and R can be an alkenyl group, the double bond is at least one carbon removed from the nitrogen to avoid enamine formation. Similarly, where a dashed line is an optional sp² bond, if it is absent, the appropriate hydrogen atom(s) is(are) included.

Preferred substitutions for Ar include methyl, methoxy, fluoromethyl, difluoromethyl, perfluoromethyl (trifluoromethyl), 1-fluoroethyl, 2-fluoroethyl, ethoxy, fluoro, chloro, and bromo, and particularly methyl, bromo, chloro, perfluoromethyl, perfluoromethoxy, methoxy, and fluoro. Preferred substitution patterns for Ar or Ar₁ are 4-substituted or 3,4-disubstituted phenyl.
Compounds of the invention are further described in the next section.

### B. Compounds

The invention features the use of compounds of formula (I) as described in the Summary section for the treatment of an allergic condition.

Preferred compounds include those wherein:
(a) R¹ is hydrogen, halogen, C₁₋₅ alkoxy, hydroxy, C₁₋₅alkyl, cyano, nitro, R⁷R⁸N, C₂₋₈ acyl, or R¹⁰R¹¹NSO₂;
(b) R¹ is halogen, cyano, nitro, R⁷R⁸N, or R¹⁰R¹¹NSO₂;
(c) R² is hydrogen;
(d) each of R³ and R⁴ is independently hydrogen or C₁₋₃alkyl;
(e) one of R³ and R⁴ is hydrogen;
(f) each of R³ and R⁴ is hydrogen;
(g) one of R⁵ and R⁶ is hydrogen and the other is a 5-7 membered carbocyclyl or heterocyclyl, optionally substituted;
(h) R⁵ and R⁶ taken together form a six-membered heterocyclyl;
(i) R⁵ and R⁶ taken together form pyridinyl, pyrimidinyl, or piperazinyl, optionally N-substituted with R⁴⁰O(C=O)(C=O)-, R⁴⁰SO₂, R⁴⁰NHCO₂, R⁴⁰(C=O)- or R⁴⁰N(C=O)-;
(j) each of R⁷, R⁸, R²¹, R²², R²⁴, R²⁵ is independently hydrogen or C₁₋₅ alkyl; or, independently, each of R⁷ and R⁸, R²¹ and R²², and R²⁴ and R²⁵ can be taken together to form an optionally substituted 4- to 7- membered heterocyclic ring, which ring may be saturated, unsaturated or aromatic;
(k) at least one of R⁷ and R⁸, R²¹ and R²², and R²⁴ and R²⁵ , taken together, is morpholinyl, piperidinyl, or pyrrolidinyl;
(I) R⁹, R²³, R²⁶, and R²⁷ is each independently hydrogen or C₁₋₅ alkyl;
(m) G is C₃₋₄ alkanediyl, optionally substituted with hydroxy, (L)-C₁₋₅ alkyloxy-, or [(L)-C₁₋₅ alkylene]amino-;
(n) G is C₃alkanediyl, optionally substituted with hydroxy, (L)-C₁₋₅ alkyloxy-, or [(L)-C₁₋₅ alkylene]amino-;
(o) X is nitrogen;
(p) Y is CR¹³;
(q) Z is CR¹⁴;
(r) X is CH;
(s) R¹² is hydrogen, R²²O(C=O)NH-, R²²NH(C=O)NH-, R²²SO₂NH, R²³SO, or R²³SO₂. and R¹³ is hydrogen, R⁴³O(C=O)NH-, R⁴³NH(C=O)NH-, R⁴³SO₂NH, R⁴⁴SO, or R⁴⁴SO₂;
(t) R¹⁴ is hydrogen, halogen, C₁₋₅ alkoxy, C₁₋₅alkyl, cyano, nitro, R²⁵O(C=O)NH-, R²⁵NH(C=O)NH-, R²⁵SO₂NH or R²⁴R²⁵N;
(u) R¹⁴ is halogen, R²⁵O(C=O)NH-, R²⁵NH(C=O)NH-, R²⁵SO₂NH or R²⁴R²⁵N; Ar represents a monocyclic ring, optionally substituted with between 1 and 2 substituents selected independently from halogen, C₁₋₅alkyl, cyano, nitro, R¹⁵R¹⁶N, CF₃ and OCF₃;
(v) Ar is a six membered ring substituted with between 1 and 2 substituents selected from halo, CF₃, OCF₃, said substitutent or substitutents being at the 4-position or at the 3- and 4-positions, respectively;
(w) W is SO₂, C=O, or CHR²⁰;
(x) W is a covalent bond;
(y) W and R¹ taken together are formula (I)(a);
(z) W and R¹ taken together are formula (I)(b);
(aa) one of R³ and R⁴ is hydrogen; Ar represents a monocyclic ring, optionally substituted with between 1 and 2 substituents selected from halogen, C₁₋₅ alkyl, cyano, nitro, R¹⁵R¹⁶N, CF₃ and OCF₃; R¹² is hydrogen, R²³SO, or R²³SO₂; R¹³ is hydrogen, R⁴⁴SO, or R⁴⁴SO₂; R¹⁴ is hydrogen, halogen, C₁₋₅ alkoxy, C₁₋₅ alkyl, cyano, nitro, or R²⁴R²⁵N; and G is C₃₋₄ alkanediyl, optionally substituted with hydroxy, C ₁₋₃ alkyl, (L)-C₁₋₅ alkyloxy, or [(L)-C₁₋₅ alkylene]amino-;
(bb) each of R³ and R⁴ is hydrogen; Ar represents a six membered ring, optionally substituted with between 1 and 2 substituents selected from halogen, C₁₋₅alkyl, cyano, nitro, R¹⁵R¹⁶N, CF₃ and OCF₃; R¹² is hydrogen, R²³SO, or R²³SO₂; R¹³ is hydrogen, R⁴⁴SO, or R⁴⁴SO₂; R¹⁴ is hydrogen, halogen, C₁₋₅alkoxy, C₁₋₅alkyl, cyano, nitro, or R²⁴R²⁵N; and G is C₃ alkanediyl, optionally substituted with hydroxy, (L)-C₁₋₅ alkyloxy-, or (L)-C₁₋₅ alkylamino;
(cc) Ar is phenyl; and
(dd) combinations of the above.

Specific preferred compounds include the examples herein, such as: 1-[4-(2-Amino-6-chloro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol ; 1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-3-methyl-urea ; 1-[3-Chloro-2-(4-{2-hydroxy-3-[5-methanesulfonyl-3-(4-trifluoromethylphenyl)-4,5,6,7-tetrahydro-pyrazolo[4,3-c]pyridin-1-yl]-propyl}-piperazin-1-yl)-phenyl]-3-methyl-urea ; 3-Amino-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-benzoic acid methyl ester ; 3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenylamine ; 1-[2-(4-{3-[3-(4-Bromo-phenyl)-5-methanesulfonyl-4,5,6,7-tetrahydro-pyrazolo[4,3-c]pyridin-1-yl]-2-hydroxypropyl}-piperazin-1-yl)-3-chloro-phenyl]-3-methyl-urea; 1-{3-[4-(2-Chloro-6-methanesulfonylamino-phenyl)-piperazin-1-yl]-propyl}-3-(4-trifluoromethylphenyl)-1,4,6,7-tetrahydro-pyrazolo[4,3-c]pyridine-5-carboxylic acid amide ; [3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-carbamic acid methyl ester; 1-[3-(4-Benzo[d]isothiazol-3-yl-piperazin-1-yl)-propyl]-3-(4-bromo-phenyl)-1 ,4,6,7-tetrahydro-pyrazolo[4,3-c]pyridine-5-carboxylic acid amide ; 2-(4-{3-[5-Acetyl-3-(4-trifluoromethyl-phenyl)-4,5,6,7-tetrahydro-pyrazolo[4,3-c]pyridin-1-yl]-2-hydroxy-propyl}-piperazin-1-yl)-3-nitro-benzoic acid methyl ester ; 1-[4-(2-Chloro-6-nitro-phenyl)-piperazin-1-yl]-3-[5-methanesulfonyl-3-(4-trifluoromethyl-phenyl)-4,5,6,7-tetrahydro-pyrazofo[4,3-c]pyridin-1-yl]-propan-2-ol ; 2-(4-{2-Hydroxy-3-[3-(4-iodo-phenyl)-5-methanesulfonyl-4,5,6,7-tetrahydro-pyrazolo[4,3-c]pyridin-1-yl]-propyl)-piperazin-1-yl)-benzonitrile ; 3-(4-Bromo-phenyl)-1-{3-[4-(2-nitro-phenyl)-piperazin-1-yl]-propyl}-1,4,6,7-tetrahydro-pyrazolo[4,3-c]pyridine-5-carboxylic acid amide ; 2-(4-{3-[5-Acetyl-3-(4-iodo-phenyl)-4,5,6,7-tetrahydro-pyrazolo[4,3-c]pyridin-1-yl]-2-hydroxy-propyl}-piperazin-1-yl)-benzonitrile ; 2-(4-{3-[3-(4-Chloro-3-methyl-phenyl)-5-methanesulfonyl-4,5,6,7-tetrahydro-pyrazolo[4,3-c]pyridin-1-yl]-2-hydroxy-propyl}-piperazin-1-yl)-benzonitrile ; 1-(3-(4-Chloro-3-methyl-phenyl)-1-{3-[4-(2,4-dimethyl-phenyl)-piperazin-1-yl]-2-hydroxy-propyl}-1,4,6,7-tetrahydro-pyrazolo[4,3-c]pyridin-5-yl)-ethanone ; 1-{3-[4-(3,5-Dichloro-pyridin-4-yl)-piperazin-1-yl]-propyl)-5-methanesulfonyl-3-(4-trifluoromethyl-phenyl)-4,5,6,7-tetrahydro-1 H-pyrazolo[4,3-c]pyridine ; 2-(4-{3-[5-Methanesulfonyl-3-(4-trifluoromethyl-phenyl)-4,5,6,7-tetrahydro-pyrazolo[4,3-c]pyridin-1-yl]-propyl}-piperazin-1-yl)-benzonitrile; N-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide ; 3-(3,4-Dichloro-phenyl)-1-{3-[4-(2-nitro-phenyl)-piperazin-1-yl]-propyl}-1,4,6,7-tetrahydro-pyrazolo[4,3-c]pyridine-5-carboxylic acid amide ; and 3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridine-5-carboxylic acid amide.

Furthermore, preferred compounds include those wherein Ar is selected from 4-trifluoromethylphenyl, 4-bromophenyl, 4-chlorophenyl, 4-chloro-3-methylphenyl and 3,4-dichlorophenyl.

More preferred compounds include the compounds in Examples 19, 27, and 33.

### Related Compounds

The invention provides the disclosed compounds and closely related, pharmaceutically acceptable forms of the disclosed compounds, such as salts, esters, amides, acids, hydrates or solvated forms thereof; masked or protected forms; and racemic mixtures, or enantiomerically or optically pure forms. Related compounds also include compounds of the invention that have been modified to be detectable, e.g., isotopically labelled with ¹⁸F for use as a probe in positron emission tomography (PET) or single-photon emission computed tomography (SPECT).

The invention also includes disclosed compounds having one or more functional groups (e.g., hydroxyl, amino, or carboxyl) masked by a protecting group. See, e.g., Greene and Wuts, Protective Groups in Organic Synthesis, 3^{rd} ed., (1999) John Wiley & Sons, NY. Some of these masked or protected compounds are pharmaceutically acceptable; others will be useful as intermediates. Synthetic intermediates and processes disclosed herein, and minor modifications thereof, are also within the scope of the invention.

### HYDROXYL PROTECTING GROUPS

Protection for the hydroxyl group includes methyl ethers, substituted methyl ethers, substituted ethyl ethers, substitute benzyl ethers, and silyl ethers.

### Substituted Methyl Ethers

Examples of substituted methyl ethers include methyoxymethyl, methylthiomethyl, *t-*butylthiomethyl, (phenyldimethylsilyl)methoxymethyl, benzyloxymethyl, *p*-methoxybenzyloxymethyl, (4-methoxyphenoxy)methyl, guaiacolmethyl, *t*-butoxymethyl, 4-pentenyloxymethyl, siloxymethyl, 2-methoxyethoxymethyl, 2,2,2-trichloroethoxymethyl, bis(2-chloroethoxy)methyl, 2-(trimethylsilyl)ethoxymethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl S,S-dioxido, 1-[(2-chloro-4-methyl)phenyl]-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

### Substituted Ethyl Ethers

Examples of substituted ethyl ethers include 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, and benzyl.

### Substituted Benzyl Ethers

Examples of substituted benzyl ethers include p-methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, 2- and 4-picolyl, 3-methyl-2-picolyl N-oxido, diphenylmethyl, *p*, *p*'-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(Imidazol-1-ylmethyl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, and benzisothiazolyl S,S-dioxido.

### Silyl Ethers

Examples of silyl ethers include trimethylsilyl, triethylsilyl, triisopropylsilyl, dimethylisopropylsilyl, diethylisopropylsilyl, dimethylthexylsilyl, *t-*butyldimethylsilyl, *t*-butyldiphenylsilyl, tribenzylsilyl, tri-*p*-xylylsilyl, triphenylsilyl, diphenylmethylsilyl, and *t*-butylmethoxyphenylsilyl..

### Esters

In addition to ethers, a hydroxyl group may be protected as an ester. Examples of esters include formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, *p*-P-phenylacetate, 3-phenylpropionate, 4-oxopentanoate(levulinate), 4,4-(ethylenedithio)pentanoate, pivaloate, adamantoate, crotonate, 4-methoxycrotonate, benzoate, *p*-phenylbenzoate, 2,4,6-trimethylbenzoate(mesitoate)

### Carbonates

Examples of carbonate protecting groups include methyl, 9-fluorenylmethyl, ethyl, 2,2,2-trichloroethyl, 2-(trimethylsilyl)ethyl, 2-(phenylsulfonyl)ethyl, 2-(triphenylphosphonio)ethyl, isobutyl, vinyl, allyl, p-nitrophenyl, benzyl, p-methoxybenzyl, 3,4-dimethoxybenzyl, o-nitrobenzyl, p-nitrobenzyl, S-benzyl thiocarbonate, 4-ethoxy-1-naphthyl, and methyl dithiocarbonate.

### Assisted Cleavage

Examples of assisted cleavage include 2-iodobenzoate, 4-azidobutyrate, 4-nitro-4-methylpentanoate, *o*-(dibromomethyl)benzoate, 2-formylbenzenesulfonate, 2-(methylthiomethoxy)ethyl carbonate, 4-(methylthiomethoxy)butyrate, and 2-(methylthiomethoxymethyl)benzoate.

### Miscellaneous Esters

Examples of miscellaneous esters include 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate(tigloate), *o*-(methoxycarbonyl)benzoate, *p*-P-benzoate, α-naphthoate, nitrate, alkyl N,N,N',N'-tetramethylphosphorodiamidate, N-phenylcarbamate, borate, dimethylphosphinothioyl, and 2,4-dinitrophenylsulfenate.

### Sulfonates

Examples of sulfonates include sulfate, methanesulfonate(mesylate), benzylsulfonate, and tosylate.

### AMINO PROTECTING GROUPS

Protection for the amino group includes carbamates, amides, and special -NH protective groups.

Examples of carbamates include methyl and ethyl carbamates, substituted ethyl carbamates, assisted cleavage carbamates, photolytic cleavage carbamates, urea-type derivatives, and miscellaneous carbamates.

### Carbamates

Examples of methyl and ethyl carbamates include methyl and ethyl, 9-fluorenylmethyl, 9-(2-sulfo)fluorenylmethyl, 9-(2,7-dibromo)fluorenylmethyl, 2,7-di-*t*-butyl-[9-(10,10-dioxo-10,10,10,10-tetrahydrothioxanthyl)]methyl, and 4-methoxyphenacyl.

### Substituted Ethyl

Examples of substituted ethyl carbamates include 2,2,2-trichloroethyl, 2-trimethylsilylethyl, 2-phenylethyl, 1-(1-adamantyl)-1-methylethyl, 1,1-dimethyl-2-haloethyl, 1,1-dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'- and 4'-pyridyl)ethyl, 2-(N,N-dicyclohexylcarboxamido)ethyl, *t*-butyl, 1-adamantyl, vinyl, allyl, 1-isopropylallyl, cinnamyl, 4-nitrocinnamyl, 8-quinolyl, N-hydroxypiperidinyl, alkyldithio, benzyl, *p*-methoxybenzyl, p-nitrobenzyl, *p-*bromobenzyl, *p*-chlorobenzyl, 2,4-dichlorobenzyl, 4-methylsulfinylbenzyl, 9-anthrylmethyl and diphenylmethyl.

### Assisted Cleavage

Examples of assisted cleavage include 2-methylthioethyl, 2-methylsulfonylethyl, 2-(*p*-toluenesulfonyl)ethyl, [2-(1,3-dithianyl)]methyl, 4-methylthiophenyl, 2,4-dimethylthiophenyl, 2-phosphonioethyl, 2-triphenylphosphonioisopropyl, 1,1-dimethyl-2-cyanoethyl, m-chloro-p-acyloxybenzyl, p-(dihydroxyboryl)benzyl, 5-benzisoxazolylmethyl, and 2-(trifluoromethyl)-6-chromonylmethyl.

### Photolytic Cleavage

Examples of photolytic cleavage include m-nitrophenyl, 3,5-dimethoxybenzyl, o-nitrobenzyl, 3,4-dimethoxy-6-nitrobenzyl, and phenyl(o-nitrophenyl)methyl.

### Urea-Type Derivatives

Examples of urea-type derivatives include phenothiazinyl-(10)-carbonyl derivative, N'-*p*-toluenesulfonylaminocarbonyl, and N'-phenylaminothiocarbonyl.

### Miscellaneous Carbamates

Examples of miscellaneous carbamates include *t*-amyl, S-benzyl thiocarbamate, p-cyanobenzyl, cyclobutyl, cyclohexyl, cyclopentyl, cyclopropylmethyl, p-decyloxybenzyl, diisopropylmethyl, 2,2-dimethoxycarbonylvinyl, o-(N,N-dimethylcarboxamido)benzyl, 1,1-dimethyl-3-(N,N-dimethylcarboxamido)propyl, 1,1-dimethylpropynyl, di(2-pyridyl)methyl, 2-furanylmethyl, 2-iodoethyl, isobornyl, isobutyl, isonicotinyl, *p-*(*p'-*methoxyphenylazo)benzyl, 1-methylcyclobutyl, 1-methylcyclohexyl, 1-methyl-1-cyclopropylmethyl, 1-methyl-1-(3,5-dimethoxyphenyl)ethyl, 1-methyl-1-(*p-*phenylazophenyl)ethyl, 1-methyl-1-phenylethyl, 1-methyl-1-(4-pyridyl)ethyl, phenyl, p-(phenylazo)benzyl, 2,4,6-tri-*t*-butylphenyl, 4-(trimethylammonium)benzyl, and 2,4,6-trimethylbenzyl.

Examples of amides include:

### Amides

N-formyl, N-acetyl, N-chloroacetyl, N-trichloroacetyl, N-trifluoroacetyl, N-phenylacetyl, N-3-phenylpropionyl, N-picolinoyl, N-3-pyridylcarboxamide, N-benzoylphenylalanyl derivative, N-benzoyl, N-*p*-phenylbenzoyl.

### Assisted Cleavage

N-o-nitrophenylacetyl, N-*o*-nitrophenoxyacetyl, N-acetoacetyl, (N'-dithiobenzyloxycarbonylamino)acetyl, N-3-(*p*-hydroxyphenyl)propionyl, N-3-(*o-*nitrophenyl)propionyl, N-2-methyl-2-(*o*-nitrophenoxy)propionyl, N-2-methyl-2-(*o*-phenylazophenoxy)propionyl, N-4-chlorobutyryl, N-3-methyl-3-nitrobutyryl, N-*o*-nitrocinnamoyl, N-acetylmethionine derivative, N-*o*-nitrobenzoyl, N-*o-*(benzoyloxymethyl)benzoyl, and 4,5-diphenyl-3-oxazolin-2-one.

### Cyclic Imide Derivatives

N-phthalimide, N-dithiasuccinoyl, N-2,3-diphenylmaleoyl, N-2,5-dimethylpyrrolyl, N-1,1,4,4-tetramethyldisilylazacyclopentane adduct, 5-substituted 1,3-dimethyl-1,3,5-triazacyclohexan-2-one, 5-substituted 1,3-dibenzyl-1,3,5-triazacyclohexan-2-one, and 1-substituted 3,5-dinitro-4-pyridonyl.

### SPECIAL - NH PROTECTIVE GROUPS

Examples of special NH protective groups include

### N-Alkyl and N-Aryl Amines

N-methyl, N-allyl, N-[2-(trimethylsilyl)ethoxy]methyl, N-3-acetoxypropyl, N-(1-isopropyl-4-nitro-2-oxo-3-pyrrolin-3-yl), quaternary ammonium salts, N-benzyl, N-di(4-methoxyphenyl)methyl, N-5-dibenzosuberyl, N-triphenylmethyl, N-(4-methoxyphenyl)diphenylmethyl, N-9-phenylfluorenyl, N-2,7-dichloro-9-fluorenylmethylene, N-ferrocenylmethyl, and N-2-picolylamine N'-oxide.

### Imine Derivatives

N-1,1-dimethylthiomethylene, N-benzylidene, N-p-methoxybenzylidene, N-diphenylmethylene, N-[(2-pyridyl)mesityl]methylene, and N-(N' ,N'-dimethylaminomethylene).

### PROTECTION FOR THE CARBONYL GROUP

### Acyclic Acetals and Ketals

Examples of acyclic acetals and ketals include dimethyl, bis(2,2,2-trichloroethyl), dibenzyl, bis(2-nitrobenzyl) and diacetyl.

### Cyclic Acetals and Ketals

Examples of cyclic acetals and ketals include 1,3-dioxanes, 5-methylene-1,3-dioxane, 5,5-dibromo-1,3-dioxane, 5-(2-pyridyl)-1,3-dioxane, 1,3-dioxolanes, 4-bromomethyl-1,3-dioxolane, 4-(3-butenyl)-1,3-dioxolane, 4-phenyl-1,3-dioxolane, 4-(2-nitrophenyl)-1,3-dioxolane, 4,5-dimethoxymethyl-1,3-dioxolane, *O*,*O*'-phenylenedioxy and 1,5-dihydro-3H-2,4-benzodioxepin.

### Acyclic Dithio Acetals and Ketals

Examples of acyclic dithio acetals and ketals include S,S'-dimethyl, S,S'-diethyl, S,S'-dipropyl, S,S'-dibutyl, S,S'-dipentyl, S,S'-diphenyl, S,S'-dibenzyl and S,S'-diacetyl.

### Cyclic Dithio Acetals and Ketals

Examples of cyclic dithio acetals and ketals include 1,3-dithiane, 1,3-dithiolane and 1,5-dihydro-3H-2,4-benzodithiepin.

### Acyclic Monothio Acetals and Ketals

Examples of acyclic monothio acetals and ketals include *O*-trimethylsilyl-S-alkyl, *O*-methyl-S-alkyl or -S-phenyl and *O*-methyl-S-2-(methylthio)ethyl.

### Cyclic Monothio Acetals and Ketals

Examples of cyclic monothio acetals and ketals include 1,3-oxathiolanes.

### MISCELLANEOUS DERIVATIVES

### O-Substituted Cyanohydrins

Examples of *O*-substituted cyanohydrins include *O*-acetyl, *O-*trimethylsilyl, *O*-1-ethoxyethyl and *O*-tetrahydropyranyl.

### Substituted Hydrazones

Examples of substituted hydrazones include N,N-dimethyl and 2,4-dinitrophenyl.

### Oxime Derivatives

Examples of oxime derivatives include *O*-methyl, *O*-benzyl and *O-*phenylthiomethyl.

### Imines

### Substituted Methylene Derivatives, Cyclic Derivatives

Examples of substituted methylene and cyclic derivatives include oxazolidines, 1-methyl-2-(1'-hydroxyalkyl)imidazoles, N,N'-dimethylimidazolidines, 2,3-dihydro-1,3-benzothiazoles, diethylamine adducts, and methylaluminum bis(2,6-di-*t*-butyl-4-methylphenoxide)(MAD)complex.

### PROTECTION FOR THE CARBOXYL GROUP

### Esters

### Substituted Methyl Esters

Examples of substituted methyl esters include 9-fluorenylmethyl, methoxymethyl, methylthiomethyl, tetrahydropyranyl, tetrahydrofuranyl, methoxyethoxymethyl, 2-(trimethylsilyl)ethoxymethyl, benzyloxymethyl, phenacyl, p-bromophenacyl, α-methylphenacyl, p-methoxyphenacyl, carboxamidomethyl, and N-phthalimidomethyl.

### 2-Substituted Ethyl Esters

Examples of 2-substituted ethyl esters include 2,2,2-trichloroethyl, 2-haloethyl, ω-chloroalkyl, 2-(trimethylsilyl)ethyl, 2-methylthioethyl, 1,3-dithianyl-2-methyl, 2-(p-nitrophenylsulfenyl)ethyl, 2-(p-toluenesulfonyl)ethyl, 2-(2'-pyridyl)ethyl, 2-(diphenylphosphino)ethyl, 1-methyl-1-phenylethyl, *t*-butyl, cyclopentyl, cyclohexyl, allyl, 3-buten-1-yl, 4-(trimethylsilyl)-2-buten-1-yl, cinnamyl, α-methylcinnamyl, phenyl, p-(methylmercapto)phenyl and benzyl.

### Substituted Benzyl Esters

Examples of substituted benzyl esters include triphenylmethyl, diphenylmethyl, bis(*o*-nitrophenyl)methyl, 9-anthrylmethyl, 2-(9,10-dioxo)anthrylmethyl, 5-dibenzosuberyl, 1-pyrenylmethyl, 2-(trifluoromethyl)-6-chromylmethyl, 2,4,6-trimethylbenzyl, p-bromobenzyl, *o*-nitrobenzyl, p-nitrobenzyl, p-methoxybenzyl, 2,6-dimethoxybenzyl, 4-(methylsulfinyl)benzyl, 4-sulfobenzyl, piperonyl, 4-picolyl and p-P-benzyl.

### Silyl Esters

Examples of silyl esters include trimethylsilyl, triethylsilyl, *t*-butyldimethylsilyl, *i*-propyldimethylsilyl, phenyldimethylsilyl and di-*t* butylmethylsilyl.

### Activated Esters

Examples of activated esters include thiols.

### Miscellaneous Derivatives

Examples of miscellaneous derivatives include oxazoles, 2-alkyl-1,3-oxazolines, 4-alkyl-5-oxo-1,3-oxazolidines, 5-alkyl-4-oxo-1,3-dioxolanes, ortho esters, phenyl group and pentaaminocobalt(III) complex.

### Stannyl Esters

Examples of stannyl esters include triethylstannyl and tri-n-butylstannyl.

### AMIDES AND HYDRAZIDES

### Amides

Examples of amides include N,N-dimethyl, pyrrolidinyl, piperidinyl, 5,6-dihydrophenanthridinyl, o-nitroanilides, N-7-nitroindolyl, N-8-Nitro-1,2,3,4-tetrahydroquinolyl, and p-P-benzenesulfonamides.

### Hydrazides

Examples of hydrazides include N-phenyl and N,N'-diisopropyl hydrazides.

### C. Synthesis

The compounds of the present invention may be prepared by conventional synthetic organic chemistry and by matrix or combinatorial methods according to Schemes 1 to 11 below, and representative detailed Examples 1 to 24. Those of ordinary skill in the art will be able to modify and adapt the guidance provided herein to make the disclosed compounds.

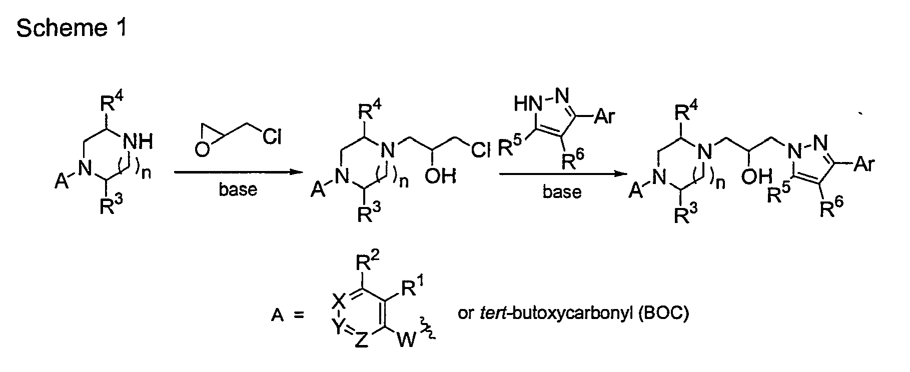

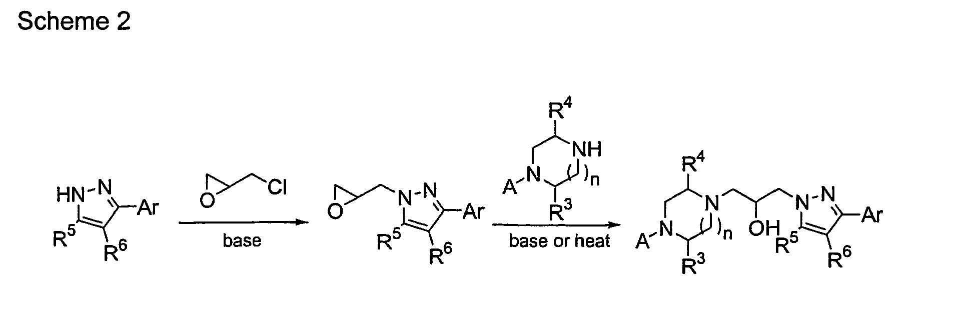

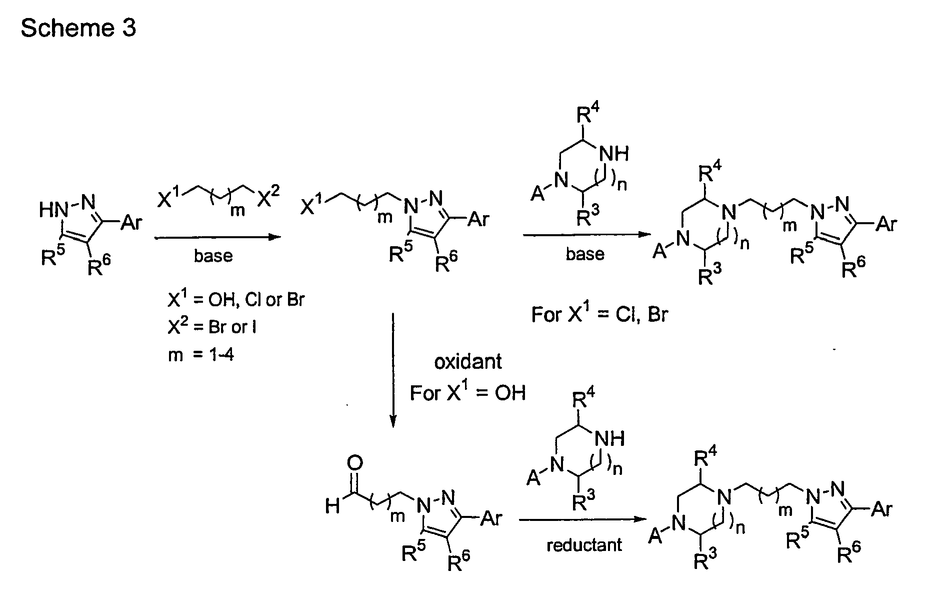

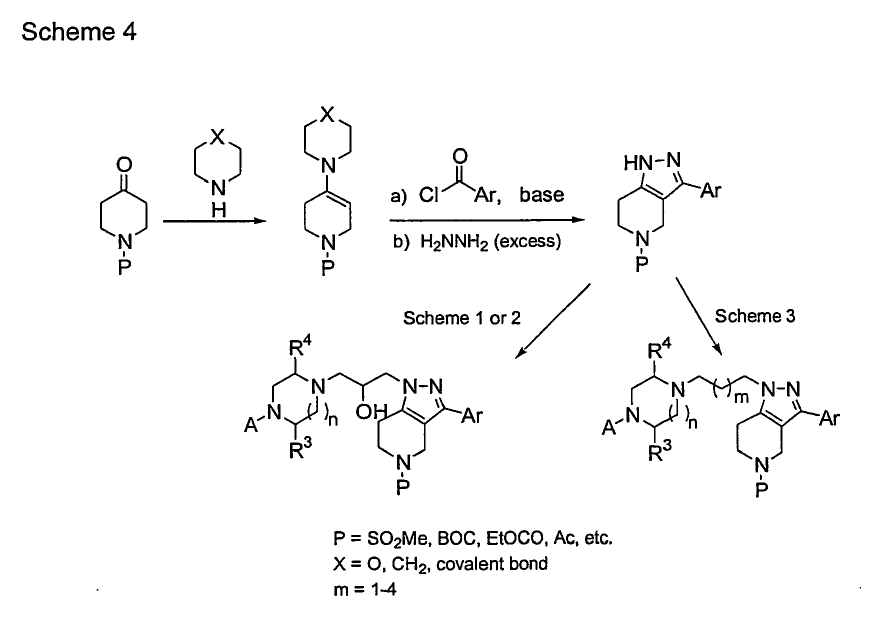

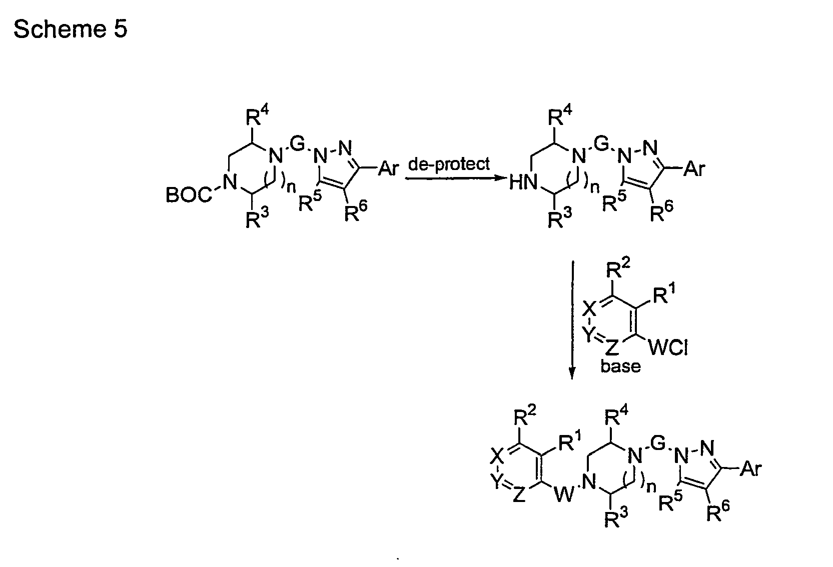

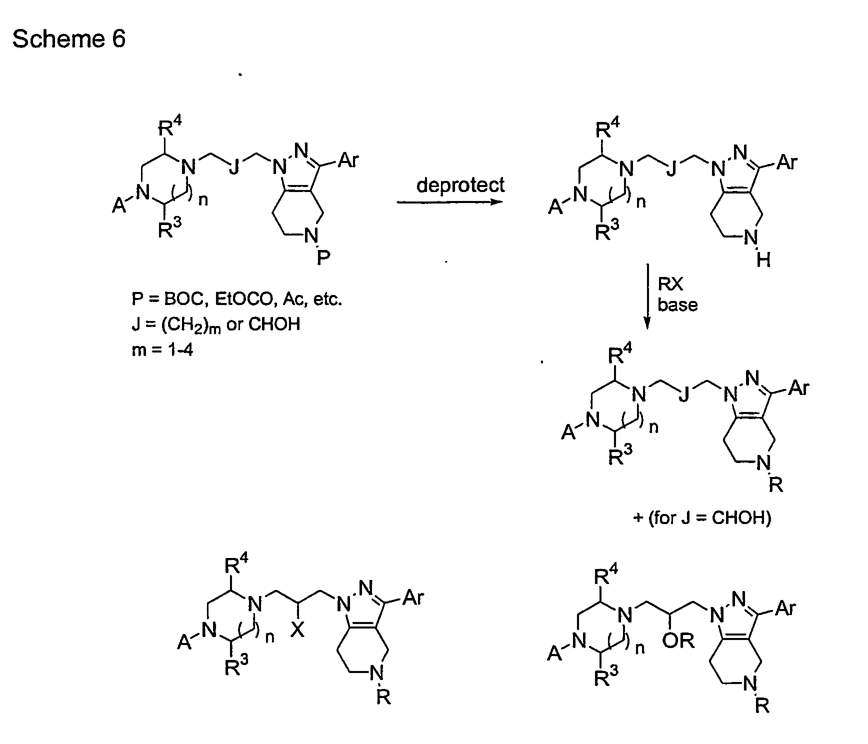

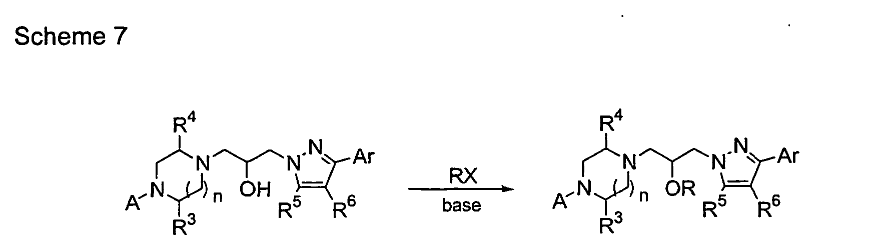

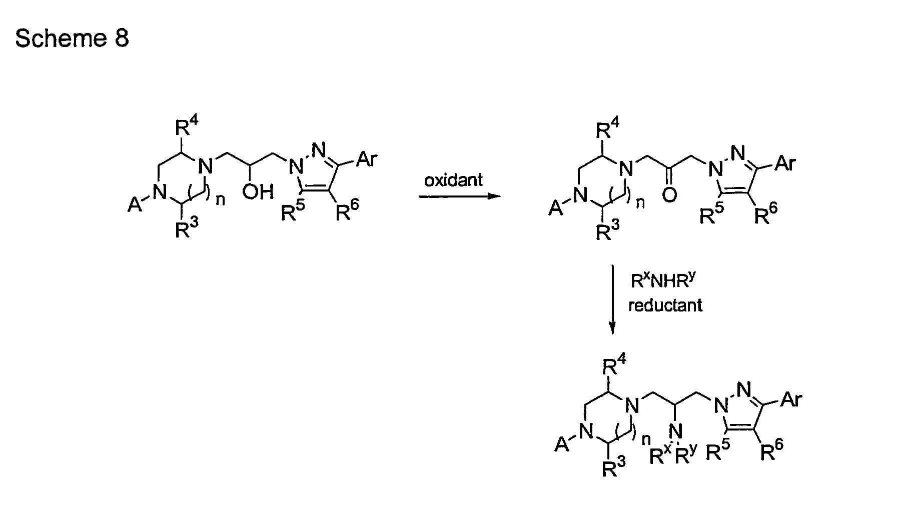

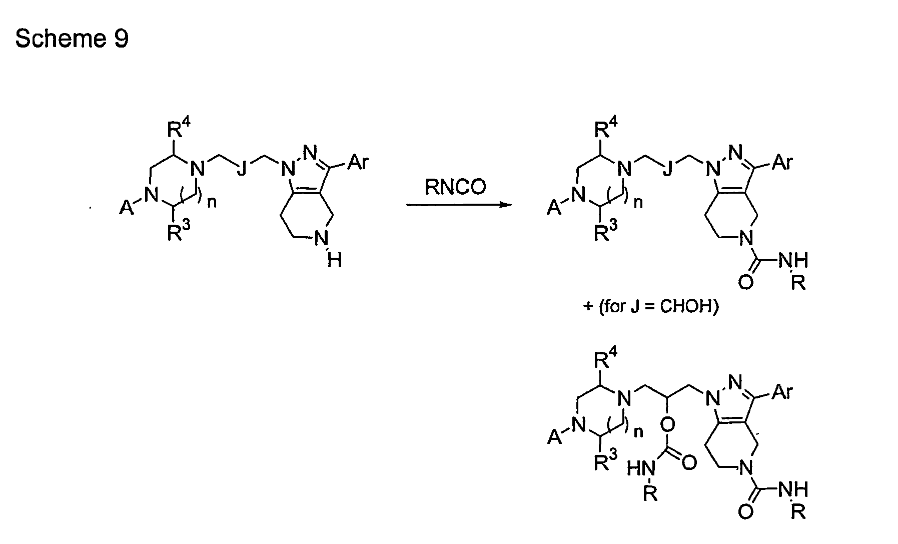

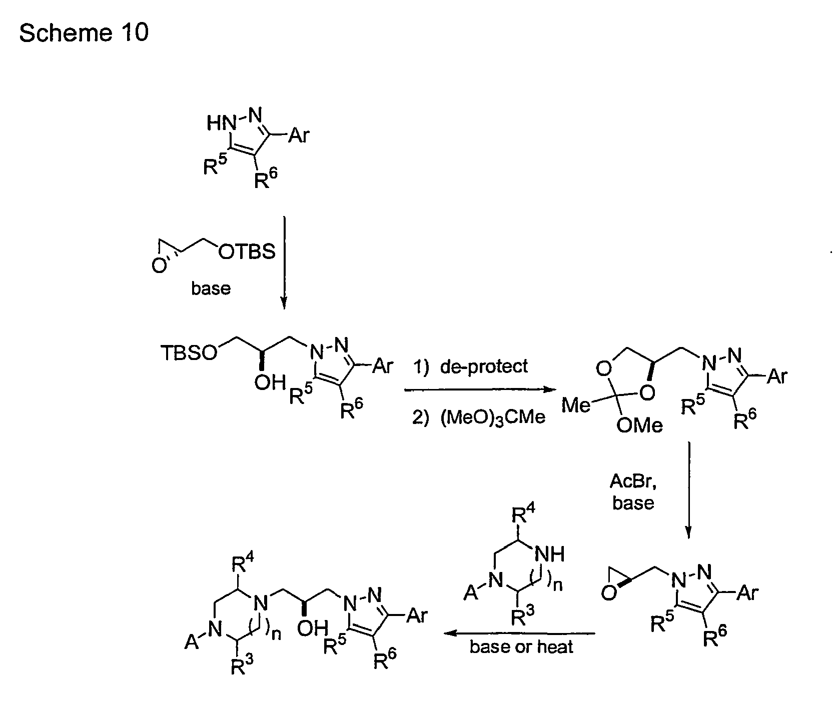

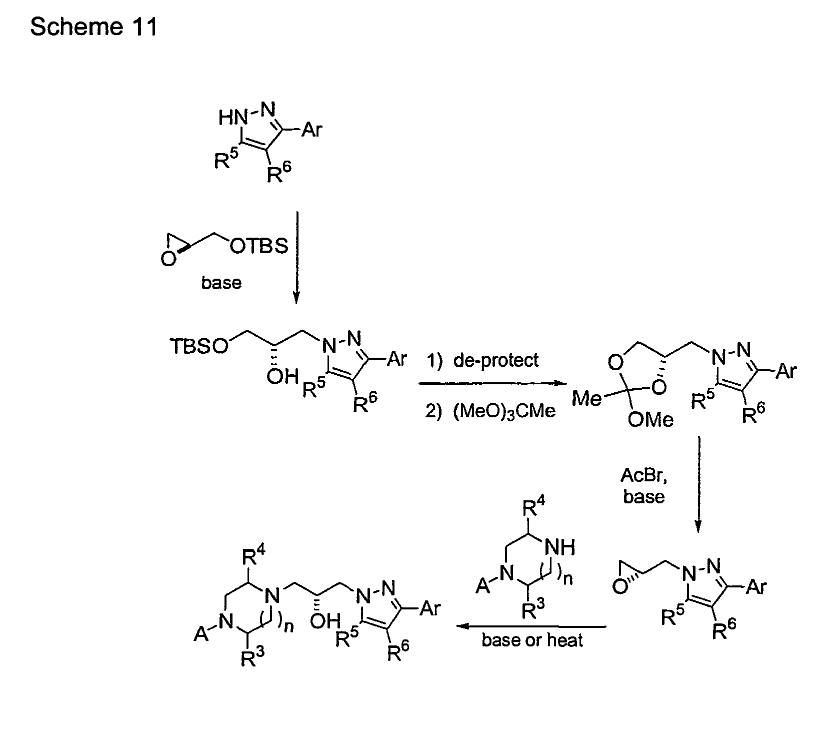

### D. Formulation and Administration

The present compounds inhibit the proteolytic activity of human cathepsin S and therefore are useful as a medicine especially in methods for treating patients suffering from allergic disorders or conditions which are modulated or regulated by the inhibition of cathepsin S activity.

The invention features the use of a therapeutically effective amount of a pharmaceutical composition comprising a compound of the invention for the manufacture of a medicament for treating a subject with an allergic condition mediated by cathepsin S. The invention also provides the use of a therapeutically effective amount of a pharmaceutical composition comprising a compound of the invention for the manufacture of a medicament for inhibiting cathepsin S activity in a subject.

In view of their inhibitory effect on the proteolytic activity of human cathepsin S the compounds of the present invention may be formulated into various pharmaceutical forms for administration purposes. To prepare these pharmaceutical compositions, an effective amount of a particular compound, in base or acid addition salt form, as the active ingredient is intimately mixed with a pharmaceutically acceptable carrier.

A carrier may take a wide variety of forms depending on the form of preparation desired for administration. These pharmaceutical compositions are desirably in unitary dosage form suitable, preferably, for oral administration or parenteral injection. For example, in preparing the compositions in oral dosage form, any of the usual pharmaceutical media may be employed. These include water, glycols, oils, and alcohols in the case of oral liquid preparations such as suspensions, syrups, elixirs and solutions; or solid carriers such as starches, sugars, kaolin, lubricants, binders and disintegrating agents in the case of powders, pills, capsules and tablets. In view of their ease in administration, tablets and capsules represent the most advantageous oral dosage unit form, in which case solid pharmaceutical carriers are generally employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions ma also be prepared in which case appropriate liquid carriers and suspending agents may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Such additives may facilitate the administration to the skin and/or may be helpful for preparing the desired compositions. These compositions may be administered in various ways, e.g., as a transdermal patch, as a spot-on, as an ointment. Acid addition salts of the compounds of formula (I), due to their increased water solubility over the corresponding base form, are more suitable in the preparation of aqueous compositions.

It is especially advantageous to formulate the aforementioned pharmaceutical compositions in dosage unit form for ease of administration and uniformity of dosage. Dosage unit form as used in the specification herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls and tablespoonfuls, and segregated multiples thereof.

Pharmaceutically acceptable acid addition salts include the therapeutically active non-toxic acid addition salt forms which the disclosed compounds are able to form. The latter can conveniently be obtained by treating the base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, *p*-aminosalicylic and palmoic acids. The term addition salt also comprises the solvates which the disclosed componds, as well as the salts thereof, are able to form. Such solvates are for example hydrates and alcoholates. Conversely the salt form can be converted by treatment with alkali into the free base form.

Stereoisomeric form defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the (R)- or (S)-configuration; substituents on bivalent cyclic saturated radicals may have either the cis- or trans-configuration. The invention encompasses stereochemically isomeric forms including diastereoisomers, as well as mixtures thereof in any proportion of the disclosed compounds. The disclosed compounds may also exist in their tautomeric forms. Such forms although not explicitly indicated in the above and following formulae are intended to be included within the scope of the present invention.

Those of skill in the treatment of disorders or conditions mediated by the cathepsin S enzyme could easily determine the effective daily amount from the test results presented hereinafter and other information. In general it is contemplated that a therapeutically effective dose would be from 0.001 mg/kg to 5 mg/kg body weight, more preferably from 0.01 mg/kg to 0.5 mg/kg body weight. It may be appropriate to administer the therapeutically effective dose as two, three, four or more sub-doses at appropriate intervals throughout the day. Said sub-doses may be formulated as unit dosage forms, for example, containing 0.05 mg to 250 mg, and in particular 0.5 to 50 mg of active ingredient per unit dosage form. Examples include 2 mg, 4 mg, 7 mg, 10 mg, 15 mg, 25 mg, and 35 mg dosage forms. Compounds of the invention may also be prepared in time-release or subcutaneous or transdermal patch formulations. Disclosed compound may also be formulated as a spray or other topical or inhalable formulations.

The exact dosage and frequency of administration depends on the particular compound of formula (I) used, the particular condition being treated, the severity of the condition being treated, the age, weight and general physical condition of the particular patient as well as other medication the patient may be taking, as is well known to those skilled in the art. Furthermore, it is evident that said effective daily amount may be.lowered or increased depending on the response of the treated patient and/or depending on the evaluation of the physician prescribing the compounds of the instant invention. The effective daily amount ranges mentioned herein are therefore only guidelines.

The next section includes detailed information relating to the preparation, characterization, and use of the disclosed compounds.

### E. Examples

### EXAMPLE 1

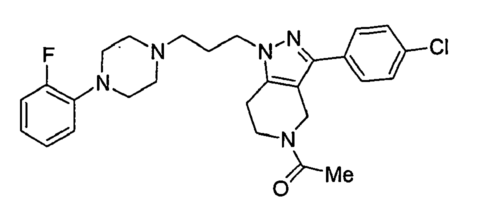

### 1-(3-(4-Chloro-phenyl)-1-{3-[4-(2-fluoro-phenyl)-piperazin-1-yl]-propyl}-1,4,6,7-tetrahydro-pyrazolo[4,3-c]pyridin-5-yl)-ethanone.

### A. 1-[3-(4-Chloro-phenyl)-1,4,6,7-tetrahydro-pyrazolo[4,3-c]pyridin-5-yl]-ethanone.

To a stirred solution of 50 g (0.35 mol) of N-acetyl-4-piperidone and 31 g (0.35 mol) of morpholine in benzene (350 mL) was added a catalytic amount (~ 0.25 g) of p-toluenesulfonic acid. The mixture was heated to reflux for 10 h with a Dean-Stark trap. The solvent was removed under reduced pressure to give a brown oil. The crude product was diluted with CH₂Cl₂ (175 mL) and 50.0 mL (0.35 mol) of Et₃N was added. The mixture was cooled to 0 °C and a solution of 45.0 mL (0.35 mol) of 4-chlorobenzoyl chloride in CH₂Cl₂ (50 mL) was added slowly by dropping funnel over 1 h. The mixture was allowed to warm to room temperature and stirred overnight. The reaction was then diluted with 1 N HCl (150 mL) and stirred vigorously for 3 h. The aqueous layer was extracted with CH₂Cl₂ (3 x 250 mL) and the combined extracts were dried over Na₂SO₄ and the solvent was removed under reduced pressure. The crude oil was diluted with EtOH (350 mL) and cooled to 0 °C. To this stirred solution was slowly added 33.0 mL (1.06 mol) of hydrazine and the mixture was allowed to warm to room temperature and stir overnight during which time a white precipitate formed. The volume of the reaction was reduced to -150 mL and EtOAc (750 mL) was added to the mixture. The suspension was stirred vigorously for 2 h and was filtered then washed with EtOAc (2 x 200 mL) and dried under vacuum to afford 41.4 g (42% over 3 steps) of a pale yellow solid. TLC (silica, 5% MeOH/CH₂Cl₂): R_{*f*}= 0.3. MS (electrospray), *m*/*z* calculated for C₁₄H₁₄ClN₃O [M+H]⁺ 276.08, observed 276.0. ¹H NMR (400 MHz, CDCl₃, a mixture of amide rotamers): 7.65 (d, *J* = 8.4 Hz, 2H), 7.64 (d, *J* = 9.3 Hz, 2H), 7.58 (d; *J* = 10.5 Hz, 2H), 7.55 (d, *J* = 8.5 Hz, 2H), 4.94 (s, 2H), 4.78 (s, 2H), 4.08 (t, *J* = 5.9 Hz, 2H), 3.90 (t, *J* = 5.8 Hz, 2H), 3.02 (t, *J* = 5.8 Hz, 2H), 2.96 (t, *J* = 5.9 Hz, 2H), 2.36 (s, 3H), 2.31 (s, 3H).

### B. 1-[3-(4-Chloro-phenyl)-1-(3-chloro-propyl)-1,4,6,7-tetrahydro-pyrazolo[4,3-c]pyridin-5-yl]-ethanone.

CS₂CO₃ (2.66 g, 8.2 mmol) was added to a solution of 1-[3-(4-chloro-phenyl)-1,4,6,7-tetrahydro-pyrazolo[4,3-c]pyridin-5-yl]-ethanone (1.0 g, 5.4 mmol) in DMF (10 mL) and stirred for 15 min. 1-Bromo-3-chloropropane (1.28 g, 8.2 mmol) was added and stirred under N₂ at room temperature for 36 h. Water (50 mL) was added to the reaction and stirred for 5 min. The product precipitated out. The aqueous portion was decanted and water was added to the residue and decanted again. The semisolid was taken up in CH₂Cl₂ and passed through a short plug of SiO₂ (5% MeOH/EtOAc) to obtain 1.06 g (83%) of a pale yellow semisolid. MS (electrospray): exact mass calculated for C₁₇H₁₉Cl₂N₃O, 351.09; m/z found, 352.0 [M+H]⁺. ¹H NMR (500 MHz, CDCl₃, a mixture of 1:1 rotamers): 7.60 (d, J = 8.3 Hz, 1 H), 7.53 (d, J = 8.3 Hz, 1 H), 7.40 (d, J = 8.3 Hz, 1 H), 7.36 (d, J = 8.3 Hz, 1 H), 4.77 (s, 1 H), 4.61 (s, 1 H), 4.20 (t, J = 6.2 Hz, 2H), 3.94 (t, J = 5.8 Hz, 1 H), 3.76 (t, J = 5.8 Hz, 1 H), 3.52 (q, J = 6.1 Hz, 2H), 2.84 (t, J = 5.5 Hz, 1H), 2.77 (t, J = 5.6 Hz, 1H), 2.37 (sextet, J = 6.1 Hz, 2 H), 2.21 (s, 1.5 H), 2.16 (s, 1.5 H).

### C. 1-(3-(4-Chloro-phenyl)-1-{3-[4-2-fluoro-phenyl)-piperazin-1-yl]-propyl}-1,4,6,7-tetrahydro-pyrazolo[4,3-c]pyridin-5-yl)-ethanone.

1-[3-(4-Chloro-phenyl)-1-(3-chloro-propyl)-1,4,6,7-tetrahydro-pyrazolo[4,3-c]pyridin-5-yl]-ethanone (0.053 g, 0.15 mmol) was dissolved in CH₃CN (0.5 mL) and a solution of 1-(2-fluorophenyl)piperazine (0.053 g, 0.30 mmol) in CH₃CN (0.5 mL) was added, followed by K₂CO₃ (0.031 g, 0.22 mmol) and Bu₄NI (0.018 g, 0.05 mmol). The mixture was stirred at room temperature for 7 d. Preparative TLC (silica, 5% MeOH/EtOAc) afforded 30 mg (41%) of the title compound. MS (electrospray): exact mass calculated for C₂₇H₃₁ClFN₅O, 495.22; *m*/*z* found, 496.3 [M+H]⁺. ¹H NMR (500 MHz, CDCl₃, a mixture of 1:1 rotamers): 7.60 (d, *J* = 8.3 Hz, 1H), 7.54 (d, *J* = 8.3 Hz, 1H), 7.39 (d, *J* = 8.3 Hz, 1H), 7.35 (d, *J* = 8.3 Hz, 1H), 7.06-6.90 (m, 4H), 4.77 (s, 1H), 4.60 (s, 1H), 4.10 (t, *J* = 6.8 Hz, 2H), 3.92 (t, *J* = 5.7 Hz, 1H), 3.74 (t, *J* = 5.7 Hz, 1H), 3.08 (br s, 4H), 2.83 (t, *J* = 5.6 Hz, 1H), 2.77 (t, *J* = 5.7 Hz, 1H), 2.58 (br s, 4H), 2.41-2.38 (m, 2H), 2.19 (s, 1.5H), 2.13 (s, 1.5H), 2.10-2.07 (m, 2H).

### EXAMPLE 2

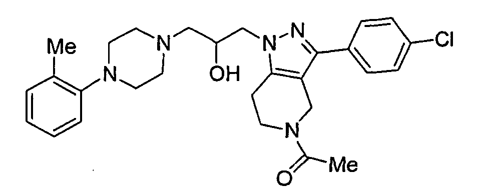

### 1-{3-(4-Chloro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone.

### A.1-[3-(4-Chlorophenyl)-1-oxiranylmethyl-1,4,6,7-tetrahydro-pyrrazolo[4,3-c]pyridin-5-yl]-ethanone.

To a stirred solution of 1.00 g (3.63 mmol) of 1-[3-(4-chloro-phenyl)-1,4,6,7-tetrahydro-pyrazolo[4,3-c]pyridin-5-yl]-ethanone and 2.85 mL (36.3 mmol) of epichlorohydrin was added 1.30 g (3.99 mmol) of solid CS₂CO₃. The reaction was stirred for 48 h and the solvent was removed under reduced pressure. The residue was then diluted with H₂O (50 mL) and EtOAc (50 mL). The layers were separated, and the organic layer was washed with H₂O (25 mL) and brine (25 mL), dried over Na₂SO₄ and the solvent was removed under reduced pressure. Purification by flash chromatography (silica, 0-15% acetone/CH₂Cl₂) afforded 0.72 g (60%) of a white solid. TLC (silica, 5% MeOH/CH₂Cl₂): R_{*f*} = 0.5. MS (electrospray): *m*/*z* calculated for C₁₇H₁₈ClN₃O₂ [M+H]⁺, 332.11, observed 332.0. ¹H NMR (400 MHz, CDCl₃, a mixture of amide rotamers): 7.60 (d, *J* = 8.6 Hz, 2H), 7.54 (d, *J* = 8.4 Hz, 2H), 7.40 (d, *J* = 8.6 Hz, 2H), 7.36 (d, *J* = 8.4 Hz, 2H), 4.80 and 4.73 (A and B of AB quartet, *J*_{ab} = 15.8 Hz, 2H), 4.60 (s, 2H), 4.47 (dd, *J* = 15.3, 2.5 Hz, 1H), 4.42 (dd, *J* = 15.0, 2.7 Hz, 1H), 4.11 (dd, *J* = 5.3, 2.5 Hz, 1 H), 4.08 (dd, *J* = 5.1, 3.3 Hz, 1 H), 3.99-3.85 (m, 2H), 3.73 (dt*, J* = 5.9, 1.8 Hz, 2H), 3.37 (m, 2H), 2.87-2.80 (m, 3H), 2.80-2.69 (m, 3H), 2.53 (dd, *J* = 4.7, 2.5 Hz, 1 H), 2.48 (dd, *J* = 4.6, 2.6, 1 H), 2.19 (s, 3H), 2.15 (s, 3H).

### B. 1-{3-(4-Chloro-phenyl)-1-[2-hydroxy-3-(4-o-tolyl-piperazin-1-yl)-propyl]-1,4,6,7-tetrahydro-pyrazolo[4,3-c]pyridin-5-yl)-ethanone.

A solution of of 1-[3-(4-chloro-phenyl)-1-oxiranylmethyl-1,4,6,7-tetrahydro-pyrazolo[4,3-c]pyridin-5-yl]-ethanone (0.8 g, 2.42 mmol) in CH₂Cl₂ (12 mL) was treated with ytterbium(III) triflate (0.15 g, 0.24 mmol) and 1-(O-tolyl)-piperazine (0.51 g, 2.90 mmol) at 25 °C. The reaction mixture was stirred for 24 h and diluted with EtOAc (100 mL) and H₂O (50 mL). The organic layer was separated, washed with H₂O (2 x 50 mL), dried over Na₂SO₄, and concentrated. Column chromatography (silica, 5% MeOH/CH₂Cl₂) afforded 1.08 g (88%) of the target compound, a white powder. TLC (silica, 10% MeOH/CH₂Cl₂): R_{*f*}= 0.38. MS (electrospray): *m*/*z* 508.3 ([M+H]⁺, C₂₈H₃₄CIN₅O₂ requires 507.2). ¹H NMR (CDCl₃, 400 MHz, a mixture of two rotamers): 7.60 and 7.37 (AB pattern, *J*_{ab} = 8.8 Hz, 2H), 7.54 and 7.40 (AB pattern, *J*_{ab} = 8.8 Hz, 2H), 7.18-7.14 (m, 2H), 7.00-6.97 (m, 2H), 4.85 and 4.73 (AB pattern, *J*_{ab} = 15.5 Hz, 1 H), 4.62 (s, 1 H), 4.20-4.11 (m, 2H), 4.06-4.01 (m, 1 H), 3.88-3.70 (m, 2H), 2.97-2.87 (m, 6H), 2.85-2.75 (m, 2H), 2.65-2.55 (m, 2H), 2.51-2.48 (m, 2H), 2.29 (s, 3H), 2.21 (s, 1.5H), 2.17 (s, 1.5H).

### EXAMPLE 3

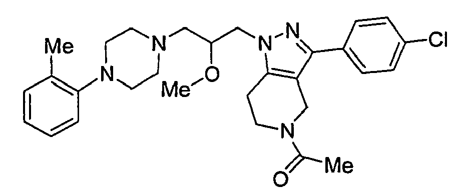

### 1-{3-(4-Chloro-phenyl)-1-[2-methoxy-3-(4-o-tolyl-piperazin-1-yl)-propyl]-1,4,6,7-tetrahydro-pyrazolo[4,3-c]pyridin-5-yl}-ethanone.

A stirred solution of 1-(3-(4-chloro-phenyl)-1-[2-hydroxy-3-(4-o-tolyl-piperazin-1-yl)-propyl]-1,4,6,7-tetrahydro-pyrazolo[4,3-c]pyridin-5-yl)-ethanone (25 mg, 0.05 mmol) in THF (0.2 mL) was treated with NaH (1.42 mg, 0.06 mmol) at 25 °C. After 20 min, methyl iodide (3.7 µL, 0.06 mmol) was added and the reaction mixture was stirred for an additional 2 h. Preparative TLC (silica, 5% MeOH/CH₂Cl₂) afforded 14.6 mg (56%) of a colorless film. TLC (silica, 10% MeOH/CH₂Cl₂): R_{*f*} = 0.38. MS (electrospray): m/z 522.2 ([M+H]⁺, C₂₉H₃₆CIN₅O₂ requires 521.3). ¹H NMR (CDCl₃, 400 MHz, a mixture of two rotamers): 7.62 and 7.37 (AB pattern, *J*_{ab} = 8.8 Hz, 2H), 7.55 and 7.40 (AB pattern, *J*_{ab} = 8.8 Hz, 2H), 7.18-7.14 (m, 2H), 7.02-6.95 (m, 2H), 4.82 and 4.75 (AB pattern, *J*_{ab} = 15.5 Hz, 1H), 4.62 (s, 1H), 4.30-4.25 (m, 1H), 4.09-3.73 (m, 4H), 3.29 (s, 1.5H), 3.27 (s, 1.5H), 2.93-2.55 (m, 12H), 2.30 (s, 3H), 2.21 (s, 1.5H), 2.16 (s, 1.5H).

### EXAMPLE 4

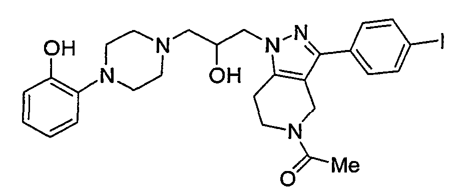

### 1-[1-{2-Hydroxy-3-[4-(2-hydroxy-phenyl)-piperazin-1-yl]-propyl}-3-(4-iodophenyl)-1,4,6,7-tetrahydro-pyrazolo[4,3-c]pyridin-5-yl]-ethanone.

### A. 1-[3-(4-Iodo-phenyl)-1,4,6,7-tetrahydro-pyrazolo[4,3-c]pyridin-5-yl]-ethanone.

A flask equipped with a Dean-Stark trap was charged with *N*-acetyl-4-piperidone (27.29g, 137 mmol), piperidine (16.5 mL, 129 mmol), p-toluenesulfonic acid (0.5 g) and benzene (150 mL). The mixture was heated to 125 °C. After 8 h the mixture was allowed to cool, and concentrated in vacuo to give the corresponding enamine (35 g). A solution of p-iodobenzoyl chloride (9.28 g, 34.8 mmol) in CH₂Cl₂ (40 mL) was added dropwise to a 0 °C solution of the enamine (11.0 g, ca. 41 mmol) in CH₂Cl₂ (80 mL) over 2 h. The mixture was then allowed to warm to room temperature and stirred for an additional 17 h. The solution was treated with 1 N HCl (200 mL) and stirred vigorously for 5 h. The layers were separated and the aqueous layer was extracted with CH₂Cl₂ (3 x 150 mL). The combined extracts were dried over Na₂SO₄ and concentrated. The residue was dissolved in EtOH (200 mL) and treated with NH₂NH₂ (16.0 mL, 51 mmol). The mixture was stirred for 17 h and H₂O (300 mL) was added. The precipitate formed was collected by filtration and air dried to give 8.82 g (59%) of 1-[3-(4-iodo-phenyl)-1,4,6,7-tetrahydro-pyrazolo[4,3-c]pyridin-5-yl]-ethanone which was suitable for use without further purification. TLC (silica, 5% MeOH/CH₂Cl₂): R_{*f*} = 0.3. MS (electrospray): *mlz* calculated for C₁₄H₁₅IN₃O [M+H]⁺ 368.03, found 368.0. ¹H NMR (CD₃OD/CDCl₃, 500 MHz, a mixture of amide rotamers): 7.72 (d, *J* = 8.2 Hz, 2H), 7.69 (d, *J* = 8.3 Hz, 2H), 7.24 (d, *J* = 8.2 Hz, 2H), 7.20 (d, *J* = 8.3 Hz, 2H), 4.69 (s, 2H), 4.56 (s, 2H), 3.83 (t, *J* = 6.0 Hz, 2H), 3.69 (t, *J* = 5.8 Hz, 2H), 2.79, (t, *J* = 5.7 Hz, 2H), 2.72, (t, *J* = 5.8 Hz, 2H), 2.13 (s, 3H), 2.08 (s, 3H).

### B. 1-[3-(4-Iodo-phenyl)-1-oxiranylmethyl-1,4,6,7-tetrahydro-pyrazolo[4,3-c]pyridin-5-yl]-ethanone.

Cs₂CO₃ (1.30 g, 4.01 mmol) was added to a solution of 1-[3-(4-iodo-phenyl)-1,4,6,7-tetrahydro-pyrazolo[4,3-c]pyridin-5-yl]-ethanone (1.34 g, 3.65 mmol) and epichlorohydrin (2.85 mL, 36.4 mmol) in DMF (10.0 mL). The mixture was stirred for 17 h then partitioned between EtOAc (400 mL) and saturated NaHCO₃ (150 mL). The NaHCO₃ layer was extracted with EtOAc (2 x 150 mL). The combined extracts were washed with H₂O (2 x 150 mL), brine (150 mL), dried over Na₂SO₄ and concentrated. The residue was purified by column chromatography (silica, 10-25% acetone/CH₂Cl₂) to give 890 mg (58%) of the title compound. HPLC , *t*_{*R*} = 5.53 min. (Reverse phase conditions: HP 1100 LCMS, Phenomenex luna 2.1 x 150 mm column, 60% MeOH/ H₂O (0.5% AcOH) to 90%MeOH/ H₂O (0.5% AcOH), held at initial conditions for 2 min then ramped to final conditions over 5 min.) MS (electrospray), m/z calculated for C₁₇H₁₈lN₃O₂Na [M+Na]⁺ 445.04, found 445.95. ¹H NMR (CDCl₃, 500 MHz, a mixture of amide rotamers): 7.76 (d, *J* = 8.3 Hz, 2H), 7.75 (d, *J* = 8.3 Hz, 2H), 7.42 (d, *J* = 8.2 Hz, 2H), 7.35 (d, *J* = 8.2 Hz, 2H), 4.80 and 4.73 (A and B of AB quartet, *J*_{ab} = 15.6 Hz, 2H), 4.60 (s, 2H), 4.84 (dd, *J* = 15.1, 2.1 Hz, 1H), 4.42 (dd, *J* = 15.0, 2.1 Hz, 1H), 4.11 (t, *J* = 5.0, Hz, 1H), 4.08 (t, *J* = 5.0 Hz, 1H), 3.98-3.87 (m, 2H), 3.74 (m, 2H), 3.34 (m, 2H), 2.87-2.72 (m, 6H), 2.52 (dd, *J* = 4.6, 2.6 Hz, 1H), 2.48 (dd, *J* = 4.5, 2.6, 1H), 2.20 (s, 3H), 2.14 (s, 3H).

### C. 1-[1-{2-Hydroxy-3-[4-(2-hydroxy-phenyl)-piperazin-1-yl]-propyl}-3-(4-iodophenyl)-1,4,6,7-tetrahydro-pyrazolo[4,3-c]pyridin-5-yl]-ethanone.

1-[3-(4-Iodo-phenyl)-1-oxiranylmethyl-1,4,6,7-tetrahydro-pyrazolo[4,3-c]pyridin-5-yl]-ethanone (62 mg, 0.15 mmol) and 4-(2-hydroxyphenyl)-piperazine (34 mg, 0.19 mmol) were combined in CH₂Cl₂ (0.5 mL) and the solution treated with Yb(OTf)_{3•}H₂O (44 mg, 0.071 mmol). The mixture was shaken for 72 h then diluted with CH₂Cl₂ (1 mL). Purification by preparative TLC (silica, 10% MeOH/CH₂Cl₂) gave 45 mg (51%) of an off-white powder. TLC (silica, 8% MeOH/CH₂Cl₂): R_{*f*} = 0.2. MS (electrospray): *m*/*z* calculated for C₂₇H₃₃IN₅O₃ [M+H]⁺ 602.15, found 602.2. ¹H NMR (CDCl₃, 500 MHz, a mixture of amide rotamers): 7.76 (d, *J* = 8.6 Hz, 1H), 7.72 (d, *J* = 8.6 Hz, 1H), 7.42 (d, *J* = 8.5 Hz, 1H), 7.34 (d, *J* = 8.5 Hz, 1H), 7.14 (m, 1H), 7.80 (t, *J* = 7.7 Hz, 1H), 6.94 (d, *J* = 8.1 Hz, 1H), 6.86 (t, *J* = 7.7 Hz, 1H), 4.83 and 4.72 (A and B of AB quartet, *J*_{ab} = 15.6 Hz, 1H), 4.61 (s, 1H), 4.22-4.15 (m, 2H), 4.02 (m, 2H), 3.88 (m, 1H), 3.76 (m, 3H), 3.00-2.49 (m, 11H), 2.20 (s, 1.5H), 2.15 (s, 1.5H).

### EXAMPLE 5

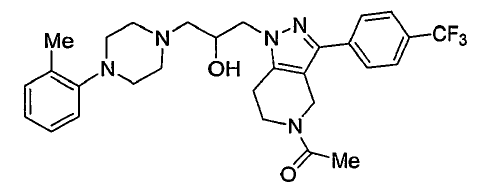

### 1-[1-[2-Hydroxy-3-(4-o-tolyl-piperazin-1-yl)-propyl]-3-(4-trifluoromethyl-phenyl)-1,4,6,7-tetrahydro-pyrazolo[4,3-c]pyridin-5-yl]-ethanone.

### A. 1-[3-(4-Trifluoromethyl-phenyl)-1,4,6,7-tetrahydro-pyrazolo[4,3-c]pyridin-5-yl]-ethanone.

A solution of *N*-acetyl-4-piperidone (2.82 g, 20 mmol), morpholine (1.93 mL, 22 mmol) and p-toluenesulfonic acid (5 mg) in benzene (8.5 mL) was refluxed for 8 h in a Dean-Stark apparatus. The solvent was removed and the residue dissolved in CH₂Cl₂ (20 mL). Triethylamine (3.1 mL) was added and p-trifluoromethylbenzoyl chloride (3.27 mL, 22 mmol) in CH₂Cl₂ (4 mL) was added dropwise into the solution at 0 °C. The reaction mixture was stirred at 25 °C for 24 h and diluted with aqueous HCl (5%, 25 mL). After stirring for another 30 min, the organic layer was separated, washed with H₂O (20 mL), dried (Na₂SO₄), and concentrated. The residue was dissolved in EtOH (95%, 18 mL) and treated at 0 °C with hydrazine (2.9 mL, 60 mmol). The mixture was stirred at 25°C for 3 h and H₂O (4 mL) was added. Most of the volatiles were removed and the residue extracted with CH₂Cl₂ (50 mL). The organic layer was separated, washed with H₂O(20 mL), dried over Na₂SO₄, and concentrated. Column chromatography (silica, 5% MeOH/CH₂Cl₂) provided 5.1 g (83%) of a white powder. TLC (silica, 10% MeOH/CH₂Cl₂): R_{f} = 0.30. MS (electrospray): m/z 332.0 ([M+Na]⁺, C₁₅H₁₄F₃N₃O requires 309.1). ¹H NMR (CDCl₃, 400 MHz, a mixture of two rotamers): 7.73-7.67 (m, 4H), 4.85 (s, 1.2H), 4.68 (s, 0.8H), 3.96 (t, *J* = 4.5 Hz, 0.8H), 3.78 (t, *J* = 4.5 Hz, 1.2H), 2.89 (t, *J* = 4.5 Hz, 1.2H), 2.83 (t, *J* = 4.5 Hz, 0.8H), 2.23 (s, 1.8H), 2.18 (s, 1.2H).

### B.1-[1-Oxiranylmethyl-3-(4-trifluoromethyl-phenyl)-1,4,6,7-tetrahydropyrazolo[4,3-c]pyridin-5-yl]-ethanone

A solution of 1-[3-(4-trifluoromethyl-phenyl)-1,4,6,7-tetrahydro-pyrazolo[4,3-c]pyridin-5-yl]-ethanone (2.4 g, 7.77 mmol) in DMF (15 mL) was treated with cesium carbonate (5.05 g, 15.5 mmol) and epichlorohydrin (6.1 mL, 77.7 mmol) at 25 °C and stirred for 24 h before it was diluted with EtOAc (100 mL) and H₂O (50 mL). The organic layer was separated, washed with H₂O (2 x 50 mL), brine (50 mL), dried over Na₂SO₄, and concentrated. Column chromatography (silica, 10% acetone/CH₂Cl₂) provided 2.30 g (81%) of a white powder. TLC (silica, 10% MeOH/CH₂Cl₂): R_{*f*} = 0.35. MS (electrospray): m/z 388.0 ([M+Na]⁺, Cl₁₈H₁₈F₃N₃O₂ requires 365.1). ¹H NMR (CDCl₃, 400 MHz, a mixture of two rotamers): 7.77 and 7.63 (AB pattern, *J*_{ab} = 8.2 Hz, 2H), 7.71 and 7.67 (AB pattern, *J*_{ab} = 8.4 Hz, 2H), 4.82 and 4.76 (AB pattern, *J*_{ab} = 15.5 Hz, 1.2H), 4.58 (s, 0.8H), 4.45-4.35 (m, 1H), 4.08-4.02 (m, 1H), 3.92-3.80 (m, 1H), 3.70-3.63 (m, 1H), 3.30 (m, 1H), 2.80-2.67 (m, 3H), 2.48-2.42 (m, 1H), 2.13 (s, 1.3H), 2.08 (s, 1.7H).

### C. 1-[1-[2-Hydroxy-3-(4-O-tolyl-piperazin-1-yl)-propyl]-3-(4-trifluoromethylphenyl)-1,4,6,7-tetrahydro-pyrazolo[4,3-c]pyridin-5-yl]-ethanone.

A solution of 1-[1-oxiranylmethyl-3-(4-trifluoromethyl-phenyl)-1,4,6,7-tetrahydro-pyrazolo[4,3-c]pyridin-5-yl]-ethanone (1.16 g, 3.20 mmol) in CH₂Cl₂ (15 mL) was treated with ytterbium(III) triflate (0.40 g, 0.64 mmol) and 1-(O-tolyl)-piperazine (0.84 g, 4.77 mmol) at 25 °C and stirred for 48 h before it was diluted with CH₂Cl₂ (100 mL) and H₂O (50 mL). The organic layer was separated, washed with H₂O(2 x 50 mL), dried over Na₂SO₄, and concentrated. Column chromatography (silica, 5% MeOH/CH₂Cl₂) afforded 1.54 g (89%) of a white powder. TLC (silica, 10% MeOH/CH₂Cl₂): R_{*f*} = 0.35. MS (electrospray): m/z 542.3 ([M+H]⁺, C₂₉H₃₄F₃N₅O₂ requires 541.3). ¹H NMR (CDCl₃, 400 MHz, a mixture of two rotamers): 7.82 and 7.65 (AB pattern, *J*_{ab} = 8.2 Hz, 2H), 7.72 and 7.68 (AB pattern, *J*_{ab} = 8.4 Hz, 2H), 7.18-6.97 (m, 4H), 4.88 and 4.76 (AB pattern, *J*_{ab} = 16 Hz, 0.9H), 4.65 (s, 1.1H), 4.23-4.12 (m, 2H), 4.08-4.00 (m, 2H), 3.88-3.70 (m, 2H), 3.02-2.85 (m, 6H), 2.85-2.75 (m, 2H), 2.65-2.55 (m, 2H), 2.53-2.45 (m, 2H), 2.29 (s, 3H), 2.21 (s, 1.8H), 2.17 (s, 1.2H).

### EXAMPLE 6

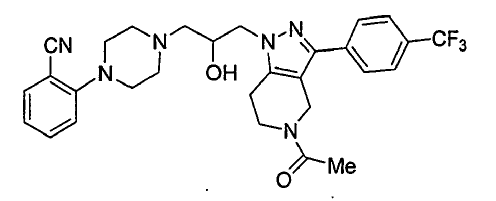

### 2-(4-{3-[5-Acetyl-3-(4-trifluoromethyl-phenyl)-4,5,6,7-tetrahydro-pyrazolo[4,3-c]pyridin-1-yl]-2-hydroxy-propyl}-piperazin-1-yl)-benzonitrile.

A solution of 1-[1-oxiranylmethyl-3-(4-trifluoromethyl-phenyl)-1,4,6,7-tetrahydro-pyrazolo[4,3-c]pyridin-5-yl]-ethanone (0.84 g, 2.30 mmol) in CH₂Cl₂ (10 mL) was treated with ytterbium(III) triflate (0.29 g, 0.46 mmol) and 1-(2-cyanophenyl)-piperazine (0.75 g, 3.5 mmol) at 25 °C and stirred for 48 h before it was diluted with CH₂Cl₂ (100 mL) and H₂O (50 mL). The organic layer was separated, washed with H₂O (2 x 50 mL), dried over Na₂SO₄, and concentrated. Column chromatography (silica, 5% MeOH/CH₂Cl₂) afforded 1.15 g (90%) of light yellow crystals. TLC (silica, 10% MeOH/CH₂Cl₂): R_{*f*} = 0.30. MS (electrospray): m/z 553.3 ([M+H]⁺, C₂₉H₃₁F₃N₆O₂ requires 552.3). ¹H NMR (CDCl₃, 400 MHz, a mixture of two rotamers): 7.82 and 7.68 (AB pattern, *J*_{ab} = 8.2 Hz, 2H), 7.76 and 7.72 (AB pattern, *J*_{ab} = 8.4 Hz, 2H), 7.60-7.48 (m, 2H), 7.05-7.00 (m, 2H), 4.90 and 4.78 (AB pattern, *J*_{ab} = 16 Hz, 1H), 4.69 (s, 1H), 4.30-3.71 (m, 6H), 3.25 (m, 4H), 3.02-2.75 (m, 4H), 2.70-2.65 (m, 2H), 2.60-2.53 (m, 2H), 2.23 (s, 1.5 H), 2.18 (s, 1.5H).

### EXAMPLE 7

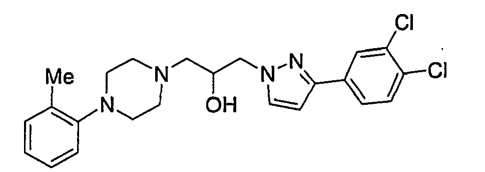

### 1-[3-(3,4-Dichloro-phenyl)-pyrazol-1-yl]-3-(4-o-tolyl-piperazin-1-yl)-propan-2-ol.

### A. 3-(3,4-Dichloro-phenyl)-1-oxiranylmethyl-1H-pyrazole.

A stirred solution of 3-(3,4-dichlorophenyl)pyrazole (300 mg, 1.4 mmol) in DMF (5 mL) was treated with cesium carbonate (550 mg, 1.69 mmol) and epichlorohydrin (1.1 mL, 14.0 mmol) at room temperature for 18 h. The crude reaction mixture was then partitioned between EtOAc (50 mL) and water (35 mL). The aqueous phase was further extracted (2 x 50 mL) and the combined organic layers were washed with brine, dried (Na₂SO₄), filtered and concentrated under reduced pressure to yield crude product. Purification by column chromatography (silica, 25% EtOAc/hexanes) afforded 308 mg (82%) of the title compound. ¹NMR (400 MHz, CDCl₃): 7.83 (d, *J* = 2 Hz, 1H), 7.54 (dd, *J* = 2, 8 Hz, 1 H), 7.44 (d, *J*=2 Hz, 1 H), 7.38 (d, J = 8 Hz, 1 H), 6.48 (d, *J* =2 Hz, 1H), 4.45 (dd, *J* = 3, 9.7 Hz, 1H), 4.12 (dd, *J* = 6, 15 Hz, 1H), 3.31 (m, 1H), 2.81 (dd, *J* = 4.0, 4.6 Hz, 1H), 2.47 (dd, *J* = 2.6, 4.7 Hz, 1H).

### B. 1-[3-(3,4-Dichloro-phenyl)-pyrazol-1-yl]-3-(4-o-tolyl-piperazin-1-yl)-propan-2-ol.

A solution of 3-(3,4-dichloro-phenyl)-1-oxiranylmethyl-1 H-pyrazole (30 mg, 0.11 mmol) and 1-(2-methylphenyl)-piperazine (22 mg, 0.12 mmol) in EtOH (1 mL) was heated to 80 °C overnight. Removal of solvent and purification by column chromatography (silica, 0-5% acetone/CH₂Cl₂) afforded 35 mg (70%) of the title compound. ¹H NMR (400 MHz, CDCl₃): 7.89 (d, J = 2 Hz, 1 H), 7.61 (dd, J = 2, 8.7 Hz, 1 H), 7.57 (d, J = 2 Hz, 1 H), 7.45 (d, J = 8.4 Hz, 1 H), 7.16 (m, 2H), 6.99 (m, 2H), 6.54 (d, J = 2.3 Hz, 1 H), 4.31 (m, 1 H), 4.18 (m, 2H), 2.93 (m, 4H), 2.60 (m, 2H), 2.47 (m, 3H), 2.88 (s, 3H).

### EXAMPLE 8

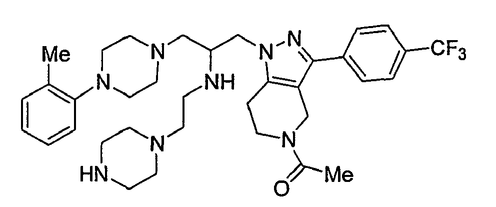

### 1-[1-[2-(2-Piperazin-1-yl-ethylamino)-3-(4-o-tolyl-piperazin-1-yl)-propyl]-3-(4-trifluoromethyl-phenyl)-1,4,6,7-tetrahydro-pyrazolo[4,3-c]pyridin-5-yl]-ethanone.

### A. 1-[5-Acetyl-3-(4-trifluoromethyl-phenyl)-4,5,6,7-tetrahydro-pyrazolo[4,3-c]pyridin-1-yl]-3-(4-o-tolyl-piperazin-1-yl)-propan-2-one.

A solution of DMSO (3.55 mL, 50 mmol) in CH₂Cl₂ (7 mL) was treated with oxalyl chloride (2.90 mL, 33 mmol) at -78 °C and stirred for 30 min. A solution of 1-[1-[2-hydroxy-3-(4-o-tolyl-piperazin-1-yl)propyl]-3-(4-trifluoromethylphenyl)-1,4,6,7-tetrahydro-pyrazolo[4,3-c]pyridin-5-yl]-ethanone (1.8 g, 3.3 mmol) in CH₂Cl₂ (7 mL) was then slowly added and the reaction mixture was stirred for an additional 30 min before it was quenched with addition of triethylamine (18.4 mL, 132 mmol). The reaction mixture was slowly warmed to 25 °C and diluted with EtOAc (50 mL) and sat. NaHCO₃ (30 mL). The organic layer was separated, washed with H₂O(2 x 50 mL), dried over Na₂SO₄, and concentrated. Column chromatography (silica, 2-5% MeOH/CH₂Cl₂) afforded 1.50 g (83%) of a light yellow powder. TLC (silica, 10% MeOH/CH₂Cl₂): R_{*f*} = 0.35. MS (electrospray): m/z 540.3 ([M+H]⁺, C₂₉H₃₂F₃N₅O₂ requires 539.3). ¹H NMR (CDCl₃, 400 MHz, a mixture of two rotamers): 7.78 and 7.62 (AB pattern, *J*_{ab} = 8.2 Hz, 2H), 7.70 and 7.64 (AB pattern, *J*_{ab} = 8.4 Hz, 2H), 7.18-6.95 (m, 4H), 5.10 (s, 1H), 5.07 (s, 1H), 4.84 (s, 1H), 4.68 (s, 1H), 3.96 (t, *J* = 4.4 Hz, 1 H), 3.78 (t, *J* = 4.4 Hz, 1H), 3.47 (3.47 (s, 4H), 3.34 (s, 2H), 2.74-2.65 (m, 6H), 2.29 (s, 3H), 2.20 (s, 1.5H), 2.17 (s, 1.5H).

### B. 1-[1-[2-(2-Piperazin-1-yl-ethylamino)-3-(4-o-tolyl-piperazin-1-yl)-propyl]-3-(4-trifluoromethyl-phenyl)-1,4,6,7-tetrahydro-pyrazolo[4,3-c]pyridin-5-yl]-ethanone.

A solution of 1-[5-acetyl-3-(4-trifluoromethyl-phenyl)-4,5,6,7-tetrahydro-pyrazolo[4,3-c]pyridin-1-yl]-3-(4-o-tolyl-piperazin-1-yl)-propan-2-one (54 mg, 0.1 mmol) in 1,2-dichloroethane (0.5 mL) was treated with 1-(2-aminoethyl)piperazine (26 µL, 0.2 mmol) and glacial acetic acid (34 µL, 0.6 mmol) at 25 °C and stirred for 30 min. Sodium triacetoxyborohydride (63.6 mg, 0.3 mmol) was added and the reaction mixture was stirred for an additional 4 h before it was quenched with CH₂Cl₂ (5 mL) and sat. NaHCO₃ (5 mL). The organic layer was separated, washed with H₂O (2 x 5 mL), dried over Na₂SO₄, and concentrated. Preparative TLC (silica, 10% MeOH/CH₂Cl₂) afforded 22 mg (35%) of a light yellow film. TLC (10% MeOH/CH₂Cl₂): R_{f} = 0.2. MS (electrospray): m/z 653.3 ([M+H]⁺, C₃₅H₄₇F₃N₈O requires 652.4). ¹H NMR (CDCl₃, 400 MHz, a mixture of two rotamers): 7.78-7.60 (m, 4H), 7.18-6.82 (m, 4H), 4.88-4.30 (m, 2H), 4.23-3.90 (m, 2H), 3.85-3.70 (m, 2H), 3.22-2.85 (m, 10H), 2.85-2.30 (m, 15H), 2.30 (s, 3H), 2.21 (s, 1.5H), 2.17 (s, 1.5H).

### EXAMPLE 9

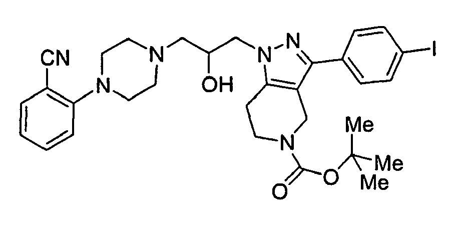

### 1-{3-[4-(2-Cyano-phenyl)-piperazin-1-yl]-2-hydroxy-propyl}-3-(4-iodo-phenyl)-1,4,6,7-tetrahydro-pyrazolo[4,3-c]pyridine-5-carboxylic acid tert-butyl ester.

### A. 3-(4-Iodo-phenyl)-1,4,6,7-tetrahydro-pyrazolo[4,3-c]pyridine-5-carboxylic acid tert-butyl ester.

p-Toluenesulfonic acid (0.055 g. 0.29 mmol) and morpholine (4.76 mL, 54 mmol) were added to a solution of *tert*-butyl 4-oxo-1-piperidinecarboxylate (10.3 g. 52 mmol) in benzene (22 mL). The reaction mixture was heated in a flask equipped with a condenser and a Dean-Stark trap at reflux for 20 h. The reaction mixture was cooled and concentrated in vacuo to give the enamine which was used without further purification. The enamine was dissolved in CH₂Cl₂ (60 mL) and cooled to 0°C. Triethylamine (8.67 mL, 62 mmol) was added, followed by dropwise addition of 4-iodobenzoyl chloride (13.8 g, 52 mmol) dissolved in CH₂Cl₂ (10 mL). The reaction mixture was allowed to warm to room temperature and stirred for 72 h. The reaction mixture was poured over water (200 mL) and the CH₂Cl₂ layer was separated, dried (Na₂SO₄), and concentrated. The resulting oil was taken up in EtOH (200 mL) and treated with hydrazine (4.88 mL, 155 mmol) at 0 °C. The reaction mixture was allowed to warm to room temperature and stirred for 17 h. The mixture was concentrated and the resulting material was triturated with EtOAc to afford 9.52 g (43%) of a white solid. TLC (silica, 10% acetone/CH₂Cl₂): *R*_{*f*}= 0.18. MS (electrospray): *m*/*z* 426.0 (426.1 calculated for C₁₇H₂₀IN₃O₂, [M+H]⁺). ¹H NMR (400 MHz, CDCl₃): 7.74 (br s, 2H), 7.31 (br d, *J* = 8.0 Hz, 2H), 4.63 (br s, 2H), 3.73 (br s, 2H), 2.77 (br s, 2H), 1.49 (s, 9H).

### B. 3-(4-lodo-phenyl)-1-oxiranylmethyl-1,4,6,7-tetrahydro-pyrazolo[4.3-c]pyridine-5-carboxylic acid tert-butyl ester.

Cesium carbonate (1.84 g, 5.65 mmol) was added to a solution of epichlorohydrin (3.68 mL, 47.05 mmol) and 3-(4-iodo-phenyl)-1,4,6,7-tetrahydro-pyrazolo[4,3-c]pyridine-5-carboxylic acid tert-butyl ester (2.0 g, 4.71 mmol) in DMF (10 mL). The reaction mixture was allowed to stir for 24 h, then partitioned between aqueous NaHCO₃ and EtOAc. The aqueous layer was extracted with EtOAc and the combined organic layers were washed with water and brine, dried (Na₂SO₄), and concentrated. Purification by column chromatography (silica, 0-10% acetone/CH₂Cl₂) afforded 2.26 g (69%) of a white foam. TLC (silica, 10% acetone/CH₂Cl₂): *R*_{*f*} = 0.44. MS (electrospray): m/z 482.0 (482.1 calculated for C₂₀H₂₄IN₃O₃, [M+H]⁺). ¹H NMR (400 MHz, CDCl₃): 7.60 (br s, 2H), 7.28 (br d, J = 8.2 Hz, 2H), 4.48 (br s, 2H), 4.32 (br d, J = 14.8 Hz, 1H), 3.99 (dd, J = 15.0, 5.4 Hz, 1H), 3.61 (br s, 1H), 3.26-3.20 (m, 1 H), 2.72 (t, J = 4.4 Hz, 1 H), 2.65-2.58 (m, 2H), 2.40 (br s, 1 H), 1.36 (s, 9H).

### C. 1-{3-[4-(2-Cyano-phenyl)-piperazin-1-yl]-2-hydroxy-propyl}-3-(4-iodophenyl)-1,4,6,7-tetrahydro-pyrazolo[4,3-c]pyridine-5-carboxylic acid tert-butyl ester.

Ytterbium (III) trifluoromethanesulfonate hydrate (0.193 g, 0.311 mmol) and 1-(2-cyanophenyl)-piperazine (0.292 g, 1.56 mmol) were dissolved in CH₂Cl₂ (2 mL) and added to a solution of 3-(4-iodo-phenyl)-1-oxiranylmethyl-1,4,6,7-tetrahydro-pyrazolo[4,3-c]pyridine-5-carboxylic acid tert-butyl ester in CH₂Cl₂ (5 mL). The reaction mixture was allowed to stir for 48 h at 25 °C. Purification by flash chromatography (silica, 0-15% acetone/CH₂Cl₂) afforded 392 mg (56%) of a white foam. TLC (silica, 10% acetone/CH₂Cl₂): *R*_{*f*}= 0.41. MS (electrospray): *m*/*z* 669.2 (669.2 calculated for C₃₁H₃₇IN₆O₃, [M+H]⁺). ¹H NMR (400 MHz, CDCl₃): 7.73 (br s, 2H), 7.58-7.56 (m, 1 H), 7.52-7.48 (m, 1 H), 7.39 (br d, *J* = 7.1 Hz, 2H), 7.04-7.00 (m, 2H), 4.60 (br s, 2H), 4.06-4.04 (m, 2H), 4.06-4.04 (m, 1 H), 3.76-3.70 (m, 2H), 3.26 (br s, 4H), 2.84-2.38 (m, 7H), 1.56-1.53 (m, 2H), 1.48 (s, 9H).

### EXAMPLE 10

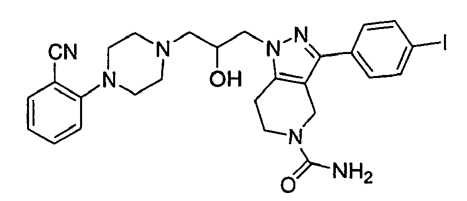

### 1-{3-[4-(2-Cyano-phenyl)-piperazin-1-yl]-2-hydroxy-propyl}-3-(4-iodo-phenyl)-1,4,6,7-tetrahydro-pyrazolo[4,3-c]pyridine-5-carboxylic acid amide.

### A. 2-4-{2-Hydroxy-3-[3-(4-iodo-phenyl)-4,5,6,7-tetrahydro-pyrazolo[4,3-c]pyridin-1-yl]-propyl}-piperazin-1-yl)-benzonitrile.

Trifluoroacetic acid (3 mL) was added to a solution of 1-{3-[4-(2-cyano-phenyl)-piperazin-1-yl]-2-hydroxy-propyl}-3-(4-iodo-phenyl)-1,4,6,7-tetrahydro-pyrazolo[4,3-c]pyridine-5-carboxylic acid tert-butyl ester (0.402 g, 0.601 mmol) in CH₂Cl₂ (3 mL) and the reaction mixture was stirred for 2 h. The mixture was concentrated, then diluted with EtOAc. The organic layer was washed with aqueous NaHCO₃ and brine, dried (Na₂SO₄), and concentrated to afford the amine (0.342 g, 100%) as a yellowish foam. TLC (silica, 10% acetone/CH₂Cl₂): *R*_{*f*}*=* 0.14. MS (electrospray): m/z 569.2 (569.1, calculated for C₂₆H₂₉IN₆O, [M+H]⁺). ¹H NMR (400 MHz, CDCl₃:CD₃OD(6:1)): 7.73 (d, *J* = 8.6 Hz, 2H), 7.56 (dd, J = 7.6, 1.8 Hz, 1 H), 7.52 (t, J = 8.0 Hz, 1 H), 7.25 (d, *J* = 8.6 Hz, 2H), 7.09 (t, *J* = 7.6 Hz, 1 H), 7.02 (dd, *J* = 8.4 Hz, 1H), 4.43-4.36 (m, 1H), 4.31 (s, 2H), 4.21 (dd, *J* = 14.1, 4.5 Hz, 1H), 4.11 (dd, *J* = 14.5, 6.3 Hz, 1H), 3.54-3.49 (m, 2H), 3.40-3.24 (m, 8H), 3.18-3.06 (m, 3H), 3.03-2.95 (m, 3H).

### B. 1-{3-[4-(2-Cyano-phenyl)-piperazin-1-yl]-2-hydroxy-propyl}-3-(4-iodophenyl)-1,4,6,7-tetrahydro-pyrazolo[4,3-c]piperidine-5-carboxylic acid amide.

Diisopropylethylamine (0.531 mL, 3.05 mmol), DMAP (5 mg), and trimethylsilyl isocyanate (0.413 mL, 3.05 mmol) were added to a solution of 2-(4-{2-hydroxy-3-[3-(4-iodo-phenyl)-4,5,6,7-tetrahydro-pyrazolo[4,3-c]pyridin-1-yl]-propyl}-piperazin-1-yl)-benzonitrile in pyridine (3 mL) and CH₂Cl₂(6 mL). The reaction mixture was stirred for 20 h, then partitioned between aqueous NaHCO₃ and CH₂Cl₂. The CH₂Cl₂ layer was washed with brine, dried (Na₂SO₄), and concentrated. The resulting product was dissolved in CH₂Cl₂ (5 mL) and treated with 21 wt% sodium ethoxide in EtOH (0.5 mL) for 3 h. The reaction mixture was washed with brine, dried (Na₂SO₄), and concentrated. Purification by column chromatography (silica, 0-10% MeOH/CH₂Cl₂) afforded 290 mg (78%) of the title compound. HPLC (reverse phase conditions), *t*_{R} = 4.21 min. MS (electrospray): m/z 612.2 (612.5, calculated for C₂₇H₃₀IN₇O₂, M⁺+H). ¹H NMR (400 MHz, CDCl₃): 7.73 (d, *J* = 8.6 Hz, 2H), 7.57 (dd, *J* = 7.6, 1.6 Hz, 1H), 7.50 (t, *J* = 7.8 Hz, 1H), 7.36 (d, *J* = 8.6 Hz, 2H), 7.05 (t, *J* = 7.6 Hz, 1H), 7.00 (d, *J* = 8.4 Hz, 1H), 4.64 (br s, 2H), 4.57 (br s, 2H), 4.30 (br s, 1H), 4.20 (dd, *J* = 14.1, 3.3 Hz, 1H), 4.06 (dd, *J* = 14.1, 6.3 Hz, 1H), 3.82-3.65 (m, 2H), 3.29-3.20 (m, 4H), 3.04-2.80 (m, 6H), 2.68 (br s, 2H).

### EXAMPLE 11

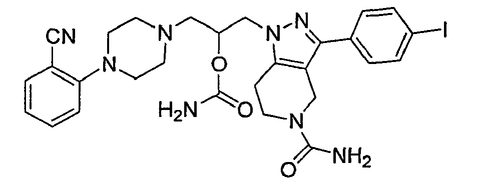

### Carbamic acid 1-[5-carbamoyl-3-(4-iodo-phenyl)-4,5,6,7-tetrahydro-pyrazolo[4,3-c]pyridin-1-ylmethyl]-2-[4-(2-cyano-phenyl)-piperazin-1-yl]-ethyl ester.

The title compound (13 mg, 3%) was obtained along with 1-{3-[4-(2-cyanophenyl)-piperazin-1-yl]-2-hydroxy-propyl}-3-(4-iodo-phenyl)-1,4,6,7-tetrahydro-pyrazolo[4,3-c]pyridine-5-carboxylic acid as described in example 10. MS (electrospray): m/z 655.2 (655.2, calculated for C₂₈H₃₁IN₈O₃, [M+H]⁺). HPLC (reverse phase conditions): *t*_{R} = 6.29 min. ¹H NMR (400 MHz, CDCl₃): 7.69 (d, J = 8.1 Hz, 2H), 7.50 (d, *J* = 7.52, 1H), 7.43 (t, *J* = 8.1 Hz, 1H), 7.31 (d, *J* = 8.1 Hz, 2H), 6.96 (t, J = 9.0 Hz, 2H), 4.64 (br s, 2H), 4.08 (d, *J* = 16.8 Hz, 2H), 3.96 (dd, *J* = 14.0, 6.6 Hz, 1H), 3.80-3.69 (m, 2H), 3.10-2.80 (m, 4H), 2.66 (br s, 2H), 2.50 (br s, 2H).

### EXAMPLE 12

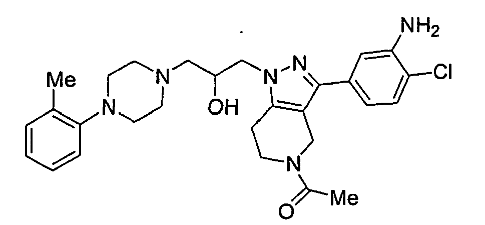

### 1-{3-(3-Amino-4-chloro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone.

### A. 1-[3-(4-Chloro-3-nitro-phenyl)-1,4,6,7-tetrahydro-pyrazolo[4,3-c]pyridin-5-yl]-ethanone.

A flask equipped with a Dean-Stark trap was charged with N-acetyl-4-piperidone (27.29g, 137 mmol), piperidine (16.5 mL, 129 mmol), p-toluenesulfonic acid (0.5 g) and benzene (150 mL). The mixture was heated to 125 °C. After 8 h the mixture was allowed to cool, and concentrated in vacuo to give the corresponding enamine (35 g). A solution of the enamine (3.87 g, 20.0 mmol) in dichloromethane (24 mL) was treated with triethylamine (3.07 mL, 22.0 mmol) and 4-chloro-3-nitrobenzoyl chloride (4.84 g, 22.0 mmol). The reaction mixture was stirred at 0 °C for 1 h and then at room temperature for 16 h. Hydrazine (1.88 mL, 60 mmol) was added to the reaction mixture. This solution was stirred at room temperature for an additional 16 h. The solvents were removed under reduced pressure. Ethyl acetate (100 mL) was added to the residue to form a suspension. This suspension was filtered and dried to afford 6.4 g (100%) of a yellow solid. MS (electrospray): m/z 321.0 (321.0, calculated for C₁₄H₁₃CIN₄O₃, [M+H]⁺). ¹H NMR (CDCl₃, 400 MHz, a mixture of two rotamers): 8.10-8.00 (m, 3H), 4.90 (s, 0.8H), 4.85 (s, 1.2H), 3.96 (m, 2H), 2.95 (m, 2H), 2.20 (s, 3H).

### B. 1-[3-(4-Chloro-3-nitro-phenyl)-1-oxiranylmethyl-1,4,6,7-tetrahydro-pyrazolo[4,3-c]pyridin-5-yl]-ethanone.

A solution of 1-[3-(4-chloro-3-nitro-phenyl)-1,4,6,7-tetrahydro-pyrazolo[4,3-c]pyridin-5-yl]-ethanone (6.4 g, 20.0 mmol) in DMF (60 mL) was treated with cesium carbonate (13.0 g, 40 mmol) and epichlorohydrin (15.6 mL, 200.0 mmol) at room temperature. The reaction mixture was stirred at room temperature for an additional 24 h before it was diluted with ethyl acetate (350 mL) and water (50 mL). The organic layer was separated, washed with water (2 x 50 mL), brine (50 mL), dried over sodium sulfate, and concentrated under reduced pressure. The residue was purified by column chromatography (silica, 10% acetone/CH₂Cl₂) to provide 7.5 g (83%) of a light yellow powder. MS (electrospray): m/z 377.0 (377.0, calculated for C₁₇H₁₇CIN₄O₄, [M+H]⁺). ¹H NMR (CDCl₃, 400 MHz, a mixture of two rotamers): 8.15-8.05 (m, 1H), 7.75-7.65 (m, 1 H), 7.55-7.45 (m, 1 H), 4.80-4.65 (m, 1.2H), 4.60 (s, 0.8H), 4.45-4.35 (m, 1 H), 4.08-4.02 (m, 1 H), 3.92-3.80 (m, 1 H), 3.70-3.63 (m, 1 H), 3.30-3.20 (m, 1 H), 2.90-2.67 (m, 3H), 2.55-2.48 (m, 1 H), 2.15 (s, 1.7H), 2.10 (s, 1.3H).

### C. 1-{3-(4-Chloro-3-nitro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone.

A solution of 1-[3-(4-chloro-3-nitro-phenyl)-1-oxiranylmethyl-1,4,6,7-tetrahydro-pyrazolo[4,3-c]pyridin-5-yl]-ethanone (0.754 g, 2.0 mmol) in dichloromethane (10 mL) was treated with ytterbium(III) triflate (0.25 g, 0.40 mmol) and 1-(2-methylphenyl)-piperazine (0.705 g, 4.0 mmol) at room temperature. The reaction mixture was stirred at room temperature for 16 h and diluted with dichloromethane (100 mL) and water (50 mL). The organic layer was separated, washed with water (2 x 50 mL), dried over sodium sulfate and concentrated under reduced pressure. The residue was purified by column chromatography (silica, 5% MeOH/CH₂Cl₂) to afford 0.98 g (90%) of the desired product as a light yellow solid. MS (electrospray): m/z 553.2 (553.2, calculated for C₂₈H₃₃CIN₆O₄, [M+H]⁺). ¹H NMR (CDCl₃, 400 MHz, a mixture of two rotamers): 8.25-8.15 (m, 1 H), 7.75-7.70 (m, 1H), 7.63-7.55 (m, 1 H) 7.20-7.10(m, 2H), 7.05-6.95 (m, 2H), 4.90-4.70 (m, 1 H), 4.65 (s, 1 H), 4.30-4.15 (m, 2H), 4.10-3.70 (m, 4H), 3.00-2.40 (m, 12H), 2.20(s, 3H), 2.15 (s, 1.5H), 2.10 (s, 1.5H).

### D. 1-{3-(3-amino-4-chloro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone.

To a solution of sodium hydrosulfite (1.28 g, 7.3 mmol) in 30 mL water was added 1-{3-(4-chloro-3-nitro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone (810 mg, 1.5 mmol) in 15 mL THF. The reaction mixture was stirred at room temperature for 5 min. The color of the solution changed from light yellow to colorless. Hydrochloride solution (1 *N*, 10 mL) was added to the reaction mixture. This solution was stirred at room temperature for 30 min, and treated with saturated sodium bicarbonate until the pH of the solution between 7 to 8. The product was extracted with dichloromethane (3 x 80 mL). The organic phases were combined, dried over sodium sulfate, and concentrated under reduced pressure to a residue. This residue was purified by column chromatography (silica, 5-20% MeOH/CH₂Cl₂) to afford 644 mg (84.1 %) of the title compound. MS (electrospray): m/z 523.3 (523.3, calculated for C₂₈H₃₅CIN₆O₂, [M+H]⁺). ¹H NMR (CDCl₃, 400 MHz, a mixture of two rotamers): 7.30-6.70 (m, 7H), 4.80-4.60 (m, 1 H), 4.55 (s, 1 H), 4.20-4.05 (m, 4H), 3.95-3.90 (m, 2H), 3.80-3.60 (m, 2H), 2.90-2.30 (m, 9H), 2.20 (s, 3H), 2.15 (s, 1.5H), 2.10 (s, 1.5H).

### EXAMPLE 13

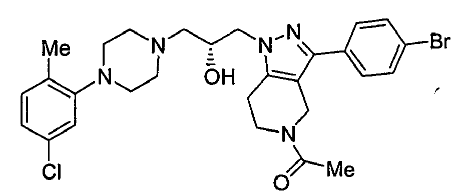

### (R)-1-(3-(4-Bromo-phenyl)-1-{3-[4-(5-chloro-2-methyl-phenyl)-piperazin-1-yl]-2-hydroxy-propyl}-1,4,6,7-tetrahydro-pyrazolo[4,3-c]pyridin-5-yl)-ethanone.

### A. (2S)-1-tert-Butyldimethylsilylglycidol.

*tert*-Butylchlorodimethylsilane (9.41 g, 62.4 mmol) followed by Et₃N (13.5 mL, 96.8 mmol) was added to a 0°C solution of *R*-(+)-glycidol (3.88 g, 52.4 mmol) in CH₂Cl₂ (100 mL). The solution was allowed to warm to 23 °C with stirring over 17h. The resulting pink solution was diluted with Et₂O (250 mL) and stirred an additional 30 min. The solution was partitioned between Et₂O (800 mL) and sat. aqueous NaHCO₃ (200 mL). The Et₂O layer was washed with sat. aqueous NaHCO₃ (250 mL), H₂O (3 x 200 mL), brine (100 mL), dried over Na₂SO₄ and concentrated. Purification of the residue by column chromatography (silica, 5-10% Et₂O/hexanes) provided 8.21 g (84%) of the title compound. TLC (silica, 10% Et₂O/hexanes): R_{*f*}= 0.5.¹H NMR (CDCl₃, 400 MHz): 3.85 (dd, J = 11.9, 3.2 Hz, 1 H), 3.66 (dd, J = 11.9, 4.8 Hz, 1 H), 3.09 (m, 1 H), 2.77 (dd, J = 5.0, 4.2 Hz, 1 H), 2.64 (dd, J = 5.2, 2.7 Hz, 1 H), 0.90 (s, 9H), 0.08 (s, 3H), 0.07 (s, 3H).

### B. 1-[3-(4-Bromo-phenyl)-1,4,6,7-tetrahydro-pyrazolo[4,3-c]pyridin-5-yl]-ethanone.

A flask equipped with a Dean-Stark trap was charged with N-acetyl-4-piperidone (100.1 g, 709 mmol), piperidine (68 mL, 779 mmol), pTsOH (3.7 g) and benzene (500 mL). The mixture was heated to 125 °C. After 17 h the mixture was allowed to cool and divided into two portions. A solution of p-bromobenzoyl chloride (70.0 g, 319 mmol) in CH₂Cl₂ (400 mL) was added dropwise to a 0 °C solution of the enamine (ca. 355 mmol) in CH₂Cl₂ (320 mL) over 15 h. The mixture was then allowed to warm to 23 °C and stirred for an additional 5 h. The solution was treated with 1 N HCl (500 mL) and stirred vigorously for 1.5 h. The layers were separated and the aqueous layer was extracted with CH₂Cl₂ (2 x 300 mL). The combined extracts were washed with sat. aqueous NaHCO₃ (300 mL), H₂O (300 mL), brine (300 mL), dried over Na₂S0₄ and concentrated. The residue was dissolved in MeOH (300 mL) and treated with NH₂NH₂ (50.0 mL, 1.59 mol). The mixture was stirred for 17 h before the precipitate formed was collected by filtration and air dried to give 52 g (50%) of the title compound which was suitable for use without further purification. TLC (silica, 5% MeOH/CH₂Cl₂): R_{*f*}= 0.3. MS (electrospray): m/z calculated for C₁₄H₁₅⁷⁹BrN₃O [M+H]⁺, 320.04, found 320. ¹H NMR (CD₃OD/CDCl₃, 400 MHz, a mixture of amide rotamers): 7.53 and 7.35 (A and B of AA'BB', J = 8.5 Hz, 2H), 7.51 and 7.39 (A and B of AA'BB', J = 8.6 Hz, 2H), 4.72 (s, 2H), 4.58 (s, 2H), 3.85 (t, J = 5.9 Hz, 2H), 3.71 (t, J = 5.8 Hz, 2H), 2.81, (t, J = 5.8 Hz, 2H), 2.74, (t, J = 5.8 Hz, 2H), 2.16 (s, 3H), 2.11 (s, 3H).

### C. (S)-1-[3-(4-Bromo-phenyl)-1-oxiranylmethyl-1,4,6,7-tetrahydro-pyrazolo[4,3-c]pyridin-5-yl]-ethanone.

A solution of KHMDS in toluene (0.5 M, 3.7 mL, 1.85 mmol) was added to a 0 °C solution of 1-[3-(4-bromo-phenyl)-1,4,6,7-tetrahydro-pyrazolo[4,3-c]pyridin-5-yl]-ethanone (492 mg, 1.54 mmol) in DMF (4.0 mL). The mixture was stirred for 1 h before (2*S*)-1-*tert*-butyldimethylsilylglycidol (870 mg, 4.62 mmol) was added neat via syringe. The mixture was stirred an additional 48 h and partitioned between EtOAc (300 mL) and sat. aqueous NaHCO₃ (100 mL). The aqueous layer was extracted with EtOAc (2 x 100 mL). The combined extracts were washed with H₂O (2 x 100 mL), brine (100 mL), dried over Na₂SO₄ and concentrated. The residue was dissolved in MeOH (50 mL) and treated with CSA (97 mg). The mixture was stirred for 17 h and concentrated to dryness. The residue was suspended in MeC(OMe)₃ (50 mL) and stirred for an additional 17 h. The mixture was diluted with EtOAc (400 mL) and washed with saturated aqueous NaHCO₃ (50 mL), H₂O (2 x 50 mL), brine (50 mL), dried over Na₂SO₄ and concentrated. The crude orthoester was dissolved in CH₂Cl₂ (5 mL), cooled to 0 °C, and treated with AcBr (0.18 mL, 2.4 mmol). The mixture was allowed to warm with stirring over 4 h before being worked up as described above. The crude acetyl-bromide obtained was dissolved in MeOH (50 mL), treated with K₂CO₃ (207 mg, 1.50 mmol) and stirred for 4 h. The reaction mixture was diluted with EtOAc (400 mL) and washed with saturated aqueous NH₄Cl (100 mL). The EtOAc layer was washed with H₂O(2 x 100 mL), brine (100 mL), dried over Na₂S0₄ and concentrated. The crude product was purified by column chromatography (silica, 10-40% acetone/CH₂Cl₂) to afford 158 mg (27%) of the title compound. Chiral HPLC (Daicel OD, 0.5% Et₂NH/MeOH) analysis indicated >95% optical purity. HPLC (reverse phase conditions): *t*_{*R*} = 4.90 min. MS (electrospray): *mlz* calculated for C₁₇H₁₉⁷⁹BrN₃O₂ [M⁺+H], 376.07, found 376.0. ¹H NMR (CDCl₃, 400 MHz, a mixture of amide rotamers): 7.47 (d with fine splittings (partially obscured), J = 8.5, Hz, 2H), 7.44 (m, 4H), 7.38 (d with fine splittings, J = 8.5, Hz, 2H), 4.71 and 4.64 (A and B of AB quartet, *J*_{ab} = 15.7 Hz, 2H), 4.51 (s, 2H), 4.39 (dd, J = 15.1, 2.5 Hz, 1 H), 4.34 (dd, J = 15.0, 2.9 Hz, 1 H), 4.02 (dd, J = 5.2, 3.9 Hz, 1 H), 3.98 (dd, J = 5.3, 3.7 Hz, 1 H), 3.83 (m, 2H), 3.64 (m, 2H), 3.25 (br m, 2H), 2.80-2.60 (m, 6H), 2.46 (dd, J = 4.6, 2.6 Hz, 1 H), 2.38 (dd, J = 4.6, 2.6 Hz, 1 H), 2.10 (s, 3H), 2.06 (s, 3H).

### D. (R)-1-(3-(4-Bromo-phenyl)-1-{3-[4-(5-chloro-2-methyl-phenyl)-piperazin-1-yl]-2-hydroxy-propyl}-1,4,6,7-tetrahydro-pyrazolo[4,3-c]pyridin-5-yl)-ethanone.

(*S*)-1-[3-(4-Bromo-phenyl)-1-oxiranylmethyl-1,4,6,7-tetrahydro-pyrazolo[4,3-c]pyridin-5-yl]-ethanone (37 mg, 0.98 mmol) and 4-(2-methyl-5-chlorophenyl)piperazine (36 mg, 0.17 mmol) were combined in EtOH (0.4 mL) and heated to 70 °C. After 18 h the mixture was allowed to cool, diluted with CH₂Cl₂ and purified by preparative TLC (silica, 8% MeOH/CH₂Cl₂) to give 35 mg (61 %) the title compound. HPLC (reverse phase conditions): *t*_{*R*} = 4.41 min. MS (electrospray): m/z calculated for C₂₈H₃₄³⁵Cl⁷⁹BrN₅O₂ [M⁺+H], 586.16, found 586.2. ¹H NMR (CDCl₃, 400 MHz, a mixture of amide rotamers): 7.56 (d (partially obscured), *J* = 8.5, Hz, 2H), 7.53 (s, 4H), 7.48 (d, *J* = 8.5 Hz, 2H), 7.08 (br d, *J* = 8.5 Hz, 1H), 6.95 (m, 2H), 4.85 and 4.73 (A and B of AB quartet, *J*_{ab} = 15.6 Hz, 1H), 4.62 (s, 1H), 4.20 (m, 2H), 4.04 (m, 2H), 3.90-3.71 (m, 2H), 2.92-2.53 (m, 11H), 2.21 (s, 1.5H), 2.16 (s, 1.5H).

### EXAMPLE 14

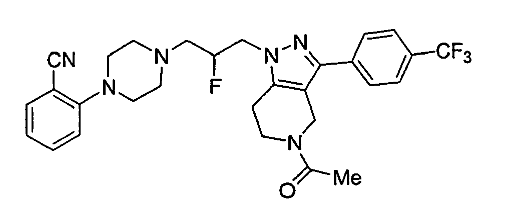

### 2-(4-{3-[5-Acetyl-3-(4-trifluoromethyl-phenyl)-4,5,6,7-tetrahydro-pyrazolo[4,3-c]pyridin-1-yl]-2-fluoro-propyl}piperazin-1-yl)-benzonitrile.

A solution of 2-(4-{3-[5-acetyl-3-(4-trifluoromethyl-phenyl)-4,5,6,7-tetrahydro-pyrazolo[4,3-c]pyridin-1-yl]-2-hydroxy-propyl}-piperazin-1-yl)-benzonitrile (150 mg, 0.27 mmol) in CH₂Cl₂ (1 mL) was treated with DAST (Et₂NSF₃, 7 µL, 0.60 mmol) at -78 °C. The reaction mixture was slowly warmed to 25 °C for 1 h and then to 60 °C for an additional 2 h. Preparative TLC (silica, 5% MeOH/CH₂Cl₂) provided 75 mg (50%) of the title compound as a light yellow powder. TLC (5% MeOH/CH₂Cl₂): R_{*f*} = 0.28. MS (electrospray): m/z 555.2 ([M⁺H]⁺, C₂₉H₃₀F₄N₆O requires 554.2). ¹H NMR (CDCl₃, 400 MHz, a mixture of two rotamers): 7.71 and 7.59 (AB pattern, *J*_{ab} = 8.2 Hz, 2H), 7.66 and 7.62 (AB pattern, *J*_{ab} = 8.4 Hz, 2H), 7.50-7.38 (m, 2H), 6.96-6.92 (m, 2H), 5.01 (dp, *J* = 49.0, 3.0 Hz, 1 H), 4.77 and 4.73 (AB pattern, *J*_{ab} = 15.7 Hz, 1.1H), 4.59 (s, 0.9H), 4.41-4.18 (m, 2H), 3.95-3.80 (m, 1H), 3.69 (dd, *J* = 5.5, 5.5 Hz, 1H), 3.18 (m, 4H), 2.83-2.65 (m, 8H), 2.14 (s, 1.6H), 2.10 (s, 1.4H).

### EXAMPLE 15

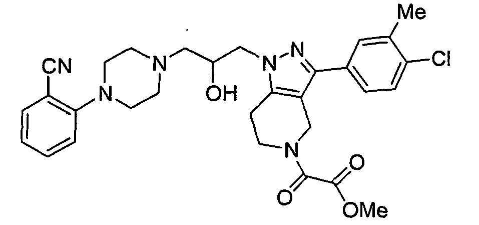

### (3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridin-5-yl)-oxo-acetic acid methyl ester.

### A. 4-Chloro-3-methyl-benzoyl chloride.

To a suspension of 52.55 g (0.31 mol) of 4-chloro-3-methyl-benzoic acid in CH₂Cl₂ (1.2 L) with DMF (1 mL) at 0 °C under N₂ with an outlet sparging through 2.5 N sodium hydroxide was added 29.56 mL (0.339 mol) of oxalyl chloride. The mixture was allowed to warm to room temperature over a 3 h period. The reaction mixture was concentrated and taken forward crude.

### B. 3-(4-Chloro-3-methyl-phenyl)-1,4,6,7-tetrahydro-pyrazolo[4,3-c]pyridine-5-carboxylic acid tert-butyl ester.

To a stirred solution of 55.8 g (0.28 mol) of 4-oxo-piperidine-1-carboxylic acid tert-butyl ester and 25.7 g (0.29 mol) of morpholine in benzene (125 mL) was added a catalytic amount (-0.25 g) of p-toluenesulfonic acid. The mixture was heated to reflux for 10 h under a Dean-Stark trap. The solvent was removed under reduced pressure to give a brown oil. The crude product was diluted with CH₂Cl₂ (400 mL), and 46.83 mL (0.34 mol) of Et₃N was added. The mixture was cooled to 0 °C, and a solution of 4-chloro-3-methyl-benzoyl chloride (0.35 mol) in CH₂Cl₂ (200 mL) was added slowly by dropping funnel over 2 h. The reaction mixture was poured over water (400 mL) and the CH₂Cl₂layer was separated, dried (Na₂SO₄), and concentrated. The resulting oil was taken up in EtOH (400 mL) and treated with 35 mL of hydrazine at 0 °C. The reaction mixture was allowed to warm to room temperature and stirred for 17 h, during which time a white precipitate formed. The volume of the reaction mixture was reduced to ~150 mL, and Et₂O (750 mL) was added. The suspension was stirred vigorously for 2 h and was filtered then washed with Et₂O (2 x 200 mL) and dried under vacuum to afford 50.74 g (52% over 3 steps) of 3-(4-chloro-3-methyl-phenyl)-1,4,6,7-tetrahydro-pyrazolo[4,3-c]pyridine-5-carboxylic acid tert-butyl ester as a pale orange solid. MS (electrospray): exact mass calculated for C₁₈H₂₂CIN₃O₂, 347.1; *m*/*z* found, 348.1 [M+H]⁺. ¹H NMR (400 MHz, CDCl₃): 7.26-7.43 (m, 4H), 4.65 (br s, 2H), 3.73 (br s, 2H), 2.77 (br s, 2H), 2.34 (s, 3H), 1.49 (s, 9H).

### C. 3-4-Chloro-3-methyl-phenyl)-1-oxiranylmethyl-1,4,6,7-tetrahydro-pyrazolo[4,3-c]pyridine-5-carboxylic acid tert-butyl ester.

To a solution of 3-(4-chloro-3-methyl-phenyl)-1,4,6,7-tetrahydro-pyrazolo[4,3-c]pyridine-5-carboxylic acid tert-butyl ester (18.26 g, 53 mmol) and -epichlorohydrin (41.12 mL, 526 mmol) in DMF (100 mL) was added cesium carbonate (20.56 g, 63 mmol). The reaction mixture was allowed to stir for 72 h, diluted with EtOAc (200 mL) and washed with saturated NaHCO₃ and brine. The organic layer was dried over Na₂SO₄, concentrated and purified by column chromatography (silica, 20% acetone/CH₂Cl₂) to afford 3-(4-chloro-3-methylphenyl)-1-oxiranylmethyl-1,4,6,7-tetrahydro-pyrazolo[4,3-c]pyridine-5-carboxylic acid tert-butyl ester (12.0 g, 57%). TLC (silica, 20% acetone/CH₂Cl₂): R_{f} = 0.68. MS (electrospray) m/z 491.2 (491.2, calculated for C₂₇H₃₁CIN₆O, [M+H]⁺). ¹H NMR (400 MHz, CDCl₃) 7.55 (s, 1 H), 7.36 (m, 2H), 4.61 (m, 2H), 4.38-4.47 (m, 1H), 4.11 (dd, J = 14.3, 5.7 Hz, 1 H), 3.67-3.79 (m, 2H), 3.34 (m, 1 H), 2.83 (t, J = 4.5 Hz, 1 H), 2.75 (m, 2H), 2.51 (m, 1H), 2.41 (s, 3H), 1.48 (s, 9H).

### D. 3-(4-Chloro-3-methyl-phenyl)-1-{3-[4-(2-cyano-phenyl)-piperazin-1-yl]-2-hydroxy-propyl}-1, 4, 6, 7-tetrahydro-pyrazolo[4 3-c]pyridine-5-carboxylic acid tert-butyl ester.

3-(4-Chloro-3-methyl-phenyl)-1-oxiranylmethyl-1,4,6,7-tetrahydro-pyrazolo[4,3-c]pyridine-5-carboxylic acid tert-butyl ester (5.33 g, 13.2 mmol) and 1-(2-cyanophenyl)-piperazine (2.97 g, 15.86 mmol) were partially dissolved in EtOH (50 mL) and triethylamine (2 mL). The reaction mixture was heated to 80 °C for 18 h. The mixture was concentrated and purified by column chromatography (silica, 20% acetone/CH₂Cl₂) to give 3-(4-chloro-3-methylphenyl)-1-{3-[4-(2-cyano-phenyl)-piperazin-1-yl]-2-hydroxy-propyl}-1,4,6,7-tetrahydro-pyrazolo[4,3-c]pyridine-5-carboxylic acid tert-butyl ester (6.51 g, 83%) as a yellow solid. TLC (silica, 20% acetone/CH₂Cl₂): *R*_{*f*}*=* 0.35. MS (electrospray): m/z 591.3 (591.3, calculated for C₃₂H₃₉ClN₆O₃, [M+H]⁺).

### E. 2-(4-{3-[3-(4-Chloro-3-methyl-phenyl)-4,5,6,7-tetrahydro-pyrazolo[4,3-c]pyridin-1-yl]-2-hydroxy-propyl}-piperazin-1-yl)-benzonitrile.

3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridine-5-carboxylic acid tert-butyl ester (1.26 g, 2.13 mmol) was dissolved in trifluoroacetic acid (3 mL) and CH₂Cl₂ (3 mL) and allowed to stir for 2 h. The reaction mixture was concentrated, taken up in EtOAc (50 mL) and washed with aqueous NaHCO₃ (2 X 25 mL). The EtOAc layer was dried over Na₂SO₄ and concentrated to give 2-(4-{3-[3-(4-chloro-3-methyl-phenyl)-4,5,6,7-tetrahydro-pyrazolo[4,3-c]pyridin-1-yl]-2-hydroxy-propyl}-piperazin-1-yl)-benzonitrile (1.05 g, 99%) as a yellow foam. TLC (silica, 10% MeOH/CH₂Cl₂): R_{*f*} = 0.27. MS (electrospray): m/z 491.2 (491.2, calculated for C₂₇H₃₁ClN₆O, [M+H]⁺). ¹H NMR (400 MHz, CDCl₃): 9.8 (br s, 1 H), 7.55 (d, J = 7.6 Hz, 1 H), 7.50 (t, J = 8.2 Hz, 1 H), 7.38 (s, 1 H), 7.31 (d, J = 8.2 Hz, 1 H), 7.20 (d, J = 8.2 Hz, 1 H), 7.11 (t, J = 8.2 Hz, 1 H), 6.98 (d, J = 8.2 Hz, 1H), 4.56 (br s, 1 H), 4.12-4.32 (m, 4H), 2.98-3.51 (m, 13 H), 2.35 (s, 3H).

### F. (3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridin-5-yl)-oxo-acetic acid methyl ester.

2-(4-{3-[3-(4-Chloro-3-methyl-phenyl)-4,5,6,7-tetrahydro-pyrazolo[4,3-c]pyridin-1-yl]-2-hydroxy-propyl}-piperazin-1-yl)-benzonitrile (58 mg, 0.118 mmol) was dissolved in CH₂Cl₂ (0.59 mL) and treated with methyl chlorooxoacetate (16 mg, 0.129 mmol). The reaction mixture was allowed to stir for 18 h at room temperature. Column chromatography (silica, 2-10% MeOH/CH₂Cl₂) gave (3-(4-chloro-3-methyl-phenyl)-1-{3-[4-(2-cyano-phenyl)-piperazin-1-yl]-2-hydroxypropyl}-1,4,6,7-tetrahydro-pyrazolo[4,3-c]pyridin-5-yl)-oxo-acetic acid methyl ester (54 mg, 79%) as a white solid. MS (electrospray): m/z 577.3 (577.2, calculated for C₃₀H₃₃ClN₆O₄, [M+H]⁺). ¹H NMR (400 MHz, CDCl₃): 7.32-7.62 (m, 5H), 7.14 (t, J = 7.6 Hz, 1 H), 7.05 (d, J = 8.2 Hz, 1 H), 4.59-4.80 (m, 3H), 4.12-4.28 (m, 2H), 3.92 (s, 3H), 3.78-3.86 (m, 2H), 3.44-3.60 (m, 5H), 3.15-3.40 (m, 4H), 2.83-3.05 (m, 2H), 2.41 (s, 3H).

### EXAMPLE 16

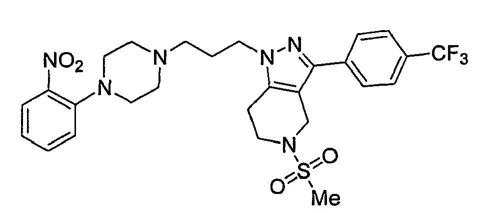

### 5-Methanesulfonyl-1-{3-[4-(2-nitro-phenyl)-piperazin-1-yl]-propyl}-3-(4-trifluoromethyl-phenyl)-4,5,6,7-tetrahydro-1H-pyrazolo[4,3-c]pyridine.

### A. 1-Methanesulfonyl-piperidin-4-one.

Potassium carbonate (324 g, 2340 mmol) was added to a solution of 4-piperidone monohydrate hydrochloride (90 g, 586 mmol) in chloroform (300 mL) and water (300 mL). The slurry was cooled to 0 °C and treated with methylsulfonyl chloride (136 mL, 1760 mmol) by dropwise addition over a 1 h period (gas evolution was observed). The reaction mixture was allowed to shake for 72 h and was partitioned between CH₂Cl₂ (500 mL) and saturated aqueous NaHCO₃ (500 mL). The aqueous layer was extracted with CH₂Cl₂ (3 X 200 mL). The organic layer was washed with 1 % KHSO₄ (250 mL), dried (Na₂SO₄), and concentrated to afford 90.5 g (87%) of a white solid. MS (electrospray): exact mass calculated for C₆H₁₁NO₃S, 177.1; m/z found, 178.1 [M+H]⁺. HPLC (reverse phase conditions):*t*_{R}= 2.19 min. ¹H NMR (400 MHz, CDCl₃): 3.60 (t, J = 6.5 Hz, 4H), 2.89 (s, 3H), 2.59 (t, J = 6.3 Hz, 4H).

### B. 5-Methanesulfonyl-3-(4-trifluoromethyl-phenyl)-4,5,6,7-tetrahydro-1H-pyrazolo[4,3-c]pyridine.

p-Toluenesulfonic acid (1.34 g. 7.0 mmol) and morpholine (25.83 mL, 296 mmol) were added to a solution of 1-methanesulfonyl-piperidin-4-one (50.0 g. 282 mmol) in benzene (282 mL). The reaction mixture was heated in a flask equipped with a condenser and a Dean-Stark trap at reflux for 15 h. The reaction mixture was cooled and concentrated in vacuo to give the enamine which was used without further purification. The enamine was dissolved in CH₂Cl₂ (200 mL) and cooled to 0°C. To this was added triethylamine (47.2 mL, 339 mmol) followed by dropwise addition of 4-trifluoromethylbenzoyl chloride (42.3 mL, 285 mmol) dissolved in CH₂Cl₂ (82 mL). The reaction mixture was allowed to warm to room temperature and stirred for 20 h. The reaction mixture was washed with 1 *N* aqueous HCl (250 mL) and the CH₂Cl₂layer was separated, dried (Na₂SO₄), and concentrated. The resulting oil was taken up in EtOH (300 mL) and treated with hydrazine (44.3 mL, 1.41 mol) at 0 °C. The reaction mixture was allowed to warm to room temperature and stirred for 24 h. The mixture was concentrated and the resulting solid was filtered with EtOH wash and dried in vacuo to afford 70 g (72%) of 5-methanesulfonyl-3-(4-trifluoromethyl-phenyl)-4,5,6,7-tetrahydro-1H-pyrazolo[4,3-c]pyridine as a white solid. MS (electrospray): exact mass calculated for C₁₄H₁₄F₃N₃O₂S, 345.0; *m*/*z* found, 346.0 [M+H]⁺. HPLC (reverse phase conditions): *t*_{R} = 6.33 min. ¹H NMR (400 MHz, CDCl₃): 7.72 (s, 4H), 4.58 (s, 2H), 3.69 (t, *J* = 5.7 Hz, 2H), 2.99 (t, *J* = 5.7 Hz, 2H), 2.92 (s, 3H).

### C. 3-[5-Methanesulfonyl-3-(4-trifluoromethyl-phenyl)-4,5,6,7-tetrahydro-pyrazolo[4,3-c]pyridin-1-yl]-propan-1-ol.

Cs₂CO₃ (33.74 g, 103.5 mmol) was added to a solution of 5-methanesulfonyl-3-(4-trifluoromethyl-phenyl)-4,5,6,7-tetrahydro-1H-pyrazolo[4,3-c]pyridine (29.8 g, 86.3 mmol) in anhydrous DMF (70 mL) and stirred for 25 min. 3-Bromo-1-propanol (8.6 mL, 13.2 g, 94.9 mmol) was added and stirred under N₂ at room temperature for 18 h. Water (500 mL) was added to the reaction and stirred for 5 min. The precipitated material was filtered out and washed with water (4 X 100 mL) and dried in a Freeze Drying System. The crude material (31.0 g) was taken up in anhydrous DMF (65 mL) and Cs₂CO₃ (33.74 g, 103.5 mmol) was added, and stirred for 10 min. 3-Bromo-1-propanof (8.6 mL, 13.2 g, 94.9 mmol) and MeOH (6.0 mL, 4.75 g, 148 mmol) were added and stirring continued under N₂ at room temperature for 15 h. Water (500 mL) was added to the reaction and stirred for 10 min. The precipitated material was filtered and washed with water (3 X 100 mL). The filter cake was dissolved in CH₂Cl₂ (200 mL) and washed with brine (50 mL), dried (Na₂SO₄), and concentrated. The solid was triturated with Et₂O (200 mL), filtered, washed with Et₂O, and dried to furnish 16.0 g of the desired compound. The mother liquor was chromatographed (silica, 0-10% acetone/EtOAc) to obtain an additional 3.0 g of the title compound. The combined yield was 54.6%. MS (electrospray): calculated for C₁₇H₂₀F₃N₃O₃S, 403.12; *m*/*z* found, 404.0 [M+H]⁺, 426.0 [M+Na]⁺. ¹H NMR (400 MHz, CDCl₃): 7.71 (d, *J* = 8.2 Hz, 2H), 7.66 (d, *J* = 8.5 Hz, 2H), 4.55 (s, 2H), 4.23 (t, J = 6.5 Hz, 2H), 3.70-3.63 (m, 4H), 2.90 (s, 3H), 2.90 (t, J = 5.1 Hz, 2H), 2.62 (t, J = 5.9 Hz, 1H), 2.06 (q, J = 6.1 Hz, 2H).

### D. 3-[5-Methanesulfonyl-3-(4-trifluoromethyl-phenyl)-4,5,6,7-tetrahydro-pyrazolo[4,3-c]pyridin-1-yl]-propionaldehyde.

Dess-Martin periodinane (3.45 g, 8.2 mmol) was added to a solution of 3-[5-methanesulfonyl-3-(4-trifluoromethyl-phenyl)-4,5,6,7-tetrahydro-pyrazolo[4,3-c]pyridin-1-yl]-propan-1-ol (3.0 g, 7.4 mmol) in CH₂Cl₂ (20 mL) at 0 °C under N₂. After 15 min, the reaction was allowed to warm to room temperature and stirred for another 1.5 h. The reaction was diluted with Et₂O (60 mL) and 20 % aq. NaHCO₃ (35 mL) was added slowly. Then Na₂S₂O₃ was added and stirred at room temperature for 30 min. The layers were separated and the aqueous portion was extracted with Et₂O (2 x 30 mL). The combined organic extracts were washed with brine, dried (Na₂SO₄) and concentrated. MPLC (1-10% MeOH/CH₂Cl₂) afforded 2.53 g of the desired aldehyde in 85% yield. MS (electrospray): calculated for C₁₇H₁₈F₃N₃O₃S, 401.11; m/z found, 402.1 [M+H], 434.1 [M+MeOH+H]. ¹H NMR (400 MHz, CDCl₃): 9.82 (s, 1 H), 7.63 (d, J = 8.4 Hz, 2H), 7.58 (d, J = 8.4 Hz, 2H), 4.68 (s, 2H), 4.25 (t, J = 6.1 Hz, 2H), 3.63 (t, J = 5.8 Hz, 4H), 3.14 (t, J = 6.1 Hz, 2H), 2.92 (t, J = 5.8 Hz, 2H), 2.81 (s, 3H).

### E. 5-Methanesulfonyl-1-{3-[4-(2-nitro-phenyl)-piperazin-1-yl]-propyl}-3-(4-trifluoromethyl-phenyl)-4,5,6,7-tetrahydro-1H-pyrazolo[4.3-c]pyridine.

To a stirred solution of 3-[5-methanesulfonyl-3-(4-trifluoromethyl-phenyl)-4,5,6,7-tetrahydro-pyrazolo[4,3-c]pyridin-1-yl]-propionaldehyde (0.060 g, 0.15 mmol) and 1-(2-nitro-phenyl)-piperazine (0.032 g, 0.157 mmol) in CH₂Cl₂ (0.5 mL), glacial AcOH (8.5 µL, 0.15 mmol) was added and stirred for 15 min at room temperature. NaBH(OAc)₃ (0.041 g, 0.19 mmol) was added and stirred under nitrogen overnight. Saturated NaHCO₃ (0.5 mL) was then added and stirred for 15 min. The layers separated and the aqueous layer was extracted with CH₂Cl₂ (0.5 mL). MPLC purification (silica, 2-15% MeOH/CH₂Cl₂) afforded the desired product as a white solid (0.063 g, 71%). TLC (silica, 12% MeOH/CH₂Cl₂): R_{*f*} = 0.67. MS (electrospray): exact mass calculated for C₂₇H₃₁F₃N₆O₄S, 592.21; m/z found, 593.2 [M+H]⁺. ¹H NMR (400 MHz, CDCl₃): 7.80 (dd, J = 1.6, 8.2 Hz, 1 H), 7.77 (d, J = 8.3 Hz, 2H), 7.70 (d, J = 8.3 Hz, 2H), 7.52 (ddd, J = 1.6, 7.3, 8.3 Hz, 1 H), 7.19 (dd, J = 1.2, 8.3 Hz, 1 H), 7.09 (m, 1H), 4.59 (s, 2H), 4.17 (t, J = 6.9 Hz, 2H), 3.71 (t, J = 5.8 Hz, 2H), 3.13 (br t, J = 4.8 Hz, 4H), 2.96 (t, J = 5.6 Hz, 2H), 2.95 (s, 3H), 2.66 (br t, J = 4.4 Hz, 4H), 2.51 (t, J = 7.0 Hz, 2H), 2.17 (q, J = 6.9 Hz, 2H).

### EXAMPLE 17

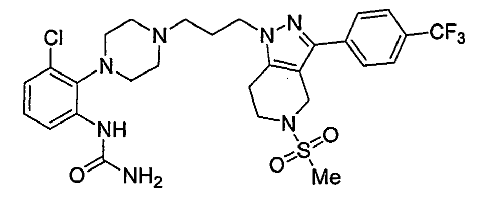

### 1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-urea.

### A. 4-(2-chloro-6-nitro-phenyl)-piperazine-1-carboxylic acid tert-butyl ester.

To a stirred solution of 1,2-dichloro-3-nitrobenzene (0.96 g, 5.0 mmol) and piperazine-1-carboxylic acid tert-butyl ester (0.93 g, 5.0 mmol) in acetonitrile (5 mL) was added of K₂CO₃ (1.38 g, 10 mmol). The mixture was heated at reflux for 48 h. The solvent was removed under reduced pressure. The crude material was partitoned between EtOAc (100 mL) and H₂O (20 mL). The organic layer was washed with H₂O (2 x 20 mL), dried over Na₂SO₄ and concentrated. Column chromatography (silica, 10-20% EtOAc/hexanes) provided 4-(2-chloro-6-nitro-phenyl)-piperazine-1-carboxylic acid tert-butyl ester (1.2 g, 70%). TLC (silica, 20% EtOAc/hexanes): R_{*f*} = 0.45. MS (electrospray): exact mass calculated for C₁₅H₂₀CIN₃O₄, 341.1; m/z found, 364.1 [M+Na]⁺. ¹H NMR (400 MHz, CDCl₃) 7.56 (dd, *J* = 8.2, 1.4 Hz, 1 H), 7.50 (dd, *J* = 8.2, 1.4 Hz, 1 H), 7.13 (t, J = 8.2 Hz, 1 H), 3.38-3.56 (m, 4H), 3.06 (m, 4H), 1.48 (s, 9H).

### B. 1-(2-chloro-6-nitro-phenyl)-piperazine.

4-(2-Chloro-6-nitro-phenyl)-piperazine-1-carboxylic acid tert-butyl ester (1.87 g, 5.47 mmol) was dissolved in trifluoroacetic acid (5.0 mL) and CH₂Cl₂ (5.0 mL) and allowed to stir for 2 h. The reaction mixture was concentrated, diluted with EtOAc, and washed with saturated aq. NaHCO₃. The organic layer was dried over Na₂SO₄, concentrated and purified by column chromatography (silica, 100% CH₂Cl₂) to afford 1-(2-chloro-6-nitro-phenyl)-piperazine (1.26 g, 95%). MS (electrospray): exact mass calculated for C₁₀H₁₂ClN₃O₂, 241.1; *m*/*z* found, 242.1 [M+H]⁺. ¹H NMR (400 MHz, CDCl₃): 7.54 (dd, *J* = 8.2, 1.6 Hz, 1 H), 7.49 (dd, *J* = 8.2, 1.6 Hz, 1H), 7.10 (t, *J* = 8.2 Hz, 1 H), 3.08 (br s, 4H), 2.99 (br s, 4H), 2.07-2.12 (m, 1H).

### C. 1-{3-[4-(2-Chloro-6-nitro-phenyl)-piperazin-1-yl]-propyl}-5-methanesulfonyl-3-(4-trifluoromethyl-phenyl)-4,5,6,7-tetrahydro-1H-pyrazolo[4.3-c]pyridine.

To a stirred solution of 3-[5-methanesulfonyl-3-(4-trifluoromethyl-phenyl)-4,5,6,7-tetrahydro-pyrazolo[4,3-c]pyridin-1-yl]-propionaldehyde (0.5 g, 1.25 mmol) and 1-(2-chloro-6-nitrophenyl)-piperazine (0.301 g, 1.25 mmol) in CH₂Cl₂ (6 mL) was added sodium sulfate (0.354 g, 2.50 mmol) and sodium triacetoxyborohydride (0.396 g, 1.87 mmol). The mixture was allowed to stir at room temperature overnight. The mixture was diluted with CH₂Cl₂ and washed with water. The CH₂Cl₂ layer was dried over Na₂SO₄, and the solvent was removed under reduced pressure. The residue was purified by column chromatography (silica, 10% acetone/CH₂Cl₂) to afford of 1-{3-[4-(2-chloro-6-nitro-phenyl)-piperazin-1-yl]-propyl}-5-methanesulfonyl-3-(4-trifluoromethylphenyl)-4,5,6,7-tetrahydro-1H-pyrazolo[4,3-c]pyridine (0.380 g, 49%). MS (electrospray): exact mass calculated for C₂₇H₃₀ClF₃N₆O₄S, 626.2; m/z found, 627.2 [M+H]⁺. ¹H NMR (400 MHz, CDCl₃): 7.73 (d, J = 8.2 Hz, 2H), 7.66 (d, J = 8.2 Hz, 2H), 7.54 (dd, J = 8.2, 1.2 Hz, 1H), 7.49 (dd, *J =* 8.2,1.2 Hz, 1H), 7.10 (t, J = 8.2 Hz, 1 H), 4.58 (s, 2H), 4.13 (t, J = 6.5 Hz, 2H), 3.71 (t, J = 5.9 Hz, 2H), 3.01-3.11 (m, 4H), 2.95 (t, J = 5.9 Hz, 2H), 2.92 (s, 3H), 2.42-2.53 (m, 4H), 2.40 (t, J = 6.5 Hz, 2H), 2.12 (q, J = 6.5 Hz, 2H).

### D. 3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenylamine.

To a stirred solution of 1-{3-[4-(2-chloro-6-nitro-phenyl)-piperazin-1-yl]-propyl}-5-methanesulfonyl-3-(4-trifluoromethyl-phenyl)-4,5,6,7-tetrahydro-1H-pyrazolo[4,3-c]pyridine (0.153 g, 0.244 mmol) in EtOH (2.44 mL) was added zinc dust (0.80 mg, 1.22 mmol) and slow addition of acetic acid (0.70 mL). After 15 min the yellow solution became colorless and the access zinc dust was filtered through a plug of celite. The filtrate was concentrated and the residue was purified by column chromatography (silica, 0-10% MeOH/CH₂Cl₂) to afford 3-chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenylamine (0.146 g, 100%). MS (electrospray): exact mass calculated for C₂₇H₃₂CIF₃N₆O₂S, 596.2; *m*/*z* found, 597.2 [M+H]⁺. ¹H NMR (400 MHz, CDCl₃): 7.73 (d, *J* = 8.2 Hz, 2H), 7.66 (d, *J* = 8.2 Hz, 2H), 6.88 (t, *J* = 8.2 Hz, 1H), 6.63 (t, J = 7.6 Hz, 2H), 4.55 (s, 2H), 4.36 (s, 2H), 4.15 (t, J = 6.5 Hz, 2H), 3.60-3.70 (m, 4H), 2.97 (t, J = 5.3 Hz, 2H), 2.90 (s, 3H), 2.83 (d, J = 10.8 Hz, 2H), 2.74 (d, J = 11.5 Hz, 2H), 2.37 (t, J = 6.6 Hz, 2H), 2.11-2.20 (m, 4H).

### E. 1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-urea,

To a stirred solution of 3-chloro-2-(4-{3-[5-methanesulfonyl-3-(4-trifluoromethylphenyl)-4,5,6,7-tetrahydro-pyrazolo[4,3-c]pyridin-1-yl]-propyl}-piperazin-1-yl)-phenylamine (0.062 g, 0.104 mmol) in CH₂Cl₂ (0.52 mL) was added trimethylsilyl isocyanate (0.017 mL, 0.125 mmol). The reaction mixture was allowed to stir for 48 h at room temperature. The reaction had not gone to completion, so an additional 0.017 mL (0.125 mmol) of trimethylsilyl isocyanate was added and the reaction was heated to 45 °C for 10 h. Column chromatography (silica, 3-10% MeOH/CH₂Cl₂) afforded 1-[3-chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-urea (0.015 g, 22%). MS (electrospray): exact mass calculated for C₂₈H₃₃CIF₃N₇O₃S, 639.2; m/z found, 640.2 [M+H]⁺. ¹H NMR (400 MHz, CDCl₃): 8.26 (br s, 1 H), 8.05 (d, J = 8.2 Hz, 1 H), 7.73 (d, J = 8.2 Hz, 2H), 7.66 (d, J = 8.2 Hz, 2H), 7.09 (t, J = 8.2 Hz, 1 H), 6.92 (d, J = 8.2 Hz, 1 H), 4.65 (s, 2H), 4.55 (s, 2H), 4.15 (t, J = 6.7 Hz, 2H), 3.65-3.73 (m, 4H), 2.96 (t, J = 5.6 Hz, 2H), 2.87-2.92 (m, 2H), 2.91 (s, 3H), 2.70 (d, J = 11.4 Hz, 2H), 2.40 (t, J = 6.7 Hz, 2H), 2.09-2.22 (m, 4H).

### EXAMPLE 18

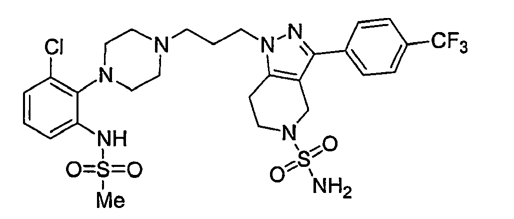

### 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-sulfonic acid amide.

### A. 3-(4-Trifluoromethyl-phenyl)-1,4,6,7-tetrahydro-pyrazolo[4,3-c]pyridine-5-carboxylic acid tert-butyl ester.

To a stirred solution of 500 g (2.51 mol) of 1-*tert*-butoxycarbonyl-4-piperidone and 87.1 g (2.76 mol) of morpholine in benzene (1.25 L) was added a catalytic amount (- 0.25 g) of p-TsOH. The mixture was heated to reflux for 36 h with a Dean-Stark trap. One half of the solvent was removed under reduced pressure and the resulting solution was cooled and filtered. The filtrate was then concentrated to yield 630 g (94%) of an orange red oil. The eneamine was divided and 320 g (1.19 mol) was diluted with CH₂Cl₂ (1.0 L) and 165.0 mL (1.19 mol) of Et₃N was added. The mixture was cooled to 0 °C and a solution of 225 g (1.08 mol) of 4-trifluoromethylbenzoyl chloride in CH₂Cl₂ (0.5 L) was added slowly by dropping funnel over 1 h. The mixture was allowed to warm to rt and stir overnight. The reaction was then diluted with 1 N HCl (450 mL) and stirred vigorously for 3 h. The aqueous layer was extracted with CH₂Cl₂ (3 x 500 mL) and the combined extracts were dried over Na₂SO₄ and the solvent was removed under reduced pressure. The crude oil was diluted with EtOH (1 L) and cooled to 0 °C. To this stirred solution was slowly added 115 g (3.57 mol) of hydrazine and the mixture was allowed to warm to rt and stir overnight during which time a white precipitate formed. The volume of the reaction was reduced to -500 mL and cooled. The precipitate was collected to afford 285 g (72% from eneamine) of a white solid. ¹H NMR (400 MHz, CDCl₃): 7.63-7.55 (m, 4H), 4.58 (br s, 2H), 3.69-3.62 (br m, 2H), 2.74-2.68 (br m, 2H), 1.47 (s, 9H).

### B. 1-(2-Methoxycarbonyl-ethyl)-3-(4-trifluoromethyl-phenyl)-1,4,6,7-tetrahydro-pyrazolo[4,3-c]pyridine-5-carboxylic acid tert-butyl ester,

3-(4-Trifluoromethyl-phenyl)-1,4,6,7-tetrahydro-pyrazolo[4,3-c]pyridine-5-carboxylic acid tert-butyl ester (1.85g, 5.04 mmol) and methyl acrylate (0.50 mL, 5.6 mmol) were combined in toluene (30 mL) and heated to 75 °C. The resulting mixture was treated with *t*-BuONa (100 mg), and heating continued for 48 h. The mixture was allowed to cool and partitioned between EtOAc (300 mL) and NaHCO₃ (75 mL). The aqueous layer was extracted with EtOAc (3 x 75 mL). The combined extracts were dried over Na₂SO₄ and concentrated. Column chromatography (silica, 30-60% EtOAc/hexanes) afforded 343 mg (15%) of the title compound. TLC (silica, 50% EtOAc/hexanes): R*f*= 0.4. MS (electrospray): m/z calculated for C₂₂H₂₇F₃N₃O₄ [M⁺+H] 454.20, found 454.1. ¹H NMR (CDCl₃, 400 MHz): 7.75 (br d, *J* = 8.1 Hz, 2H), 7.64 (br s, 2H), 4.63 (br s, 2H), 4.30 (t, J = 6.6 Hz, 2H), 3.75 (br s, 2H), 3.68 (s, 3H), 2.98 (t, J = 6.6 Hz, 2H), 2.79 (br t, J = 5.6 Hz, 2H), 1.48 (s, 9H).

### C. 1-(3-Hydroxy-propyl-3-(4-trifluoromethyl-phenyl)-1,4,6,7-tetrahydro-pyrazolo[4,3-c]pyridine-5-carboxylic acid tert-butyl ester.

A solution of LiBH₄ (26 mg, 1.2 mmol) in THF (0.5 mL) was added to a 0 °C solution of 1-(2-methoxycarbonyl-ethyl)-3-(4-trifluoromethyl-phenyl)-1,4,6,7-tetrahydro-pyrazolo[4,3-c]pyridine-5-carboxylic acid tert-butyl ester (317 mg, 0.70 mmol) in THF (4.0 mL). The mixture was stirred for 5 min then additional LiBH₄ (15 mg) was added and stirring continued for 17 h. The mixture was partitioned between EtOAc (80 mL) and saturated aqueous NaHCO₃ (20 mL). The aqueous layer was extracted with EtOAc (2 x 20 mL). The combined extracts were dried over Na₂SO₄ and concentrated. Column chromatography (silica, 0-8% MeOH/CH₂Cl₂) afforded 268 mg (95%) of the title compound. HPLC (reverse phase conditions), *t*_{R} = 6.82 min. MS (electrospray): *mlz* calculated for C₂₁H₂₆F₃N₃O₃ [M⁺+Na] 448.18, found 448.10. ¹H NMR (CDCl₃, 400 MHz): 7.73 (br d, *J* = 8.2 Hz, 2H), 7.65 (br s, 2H), 4.64 (br s, 2H), 4.21 (t, *J* = 6.4 Hz, 2H), 3.76 (br s, 2H), 3.66 (t, J = 5.7 Hz, 2H), 2.73 (br t, J = 5.4 Hz, 2H), 2.04 (q, J = 6.1, 2H), 1.48 (s, 9H).

### D. 1-(3-Oxo-propyl)-3-(4-trifluoromethyl-phenyl)-1,4,6,7-tetrahydro-pyrazolo[4,3-c]pyridine-5-carboxylic acid tert-butyl ester.

Dess-Martin periodinane (1.43 g, 3.36 mmol) was added portion wise to a stirred solution of 1-(3-hydroxy-propyl)-3-(4-trifluoromethyl-phenyl)-1,4,6,7-tetrahydropyrazolo[4,3-c]pyridine-5-carboxylic acid tert-butyl ester (1.30 g, 3.05 mmol) in CH₂Cl₂ (15 mL) at 0 °C under N₂.Then the reaction was stirred at 0 °C for 15 min and allowed to warm to room temperature. After stirring at room temperature for 1.5 h the reaction was diluted with Et₂O(50 mL) and saturated NaHCO₃ (15 mL) was added slowly (caution! gas evolution). Then Na₂S₂O₃.5H₂O (5.31 g, 21.4 mmol) was added and stirred for 30 min. The layers were separated and the aqueous layer was extracted with Et₂O (2 x 30 mL). The combined extracts were washed with brine, dried (Na₂SO₄) and concentrated. MPLC (1-10% MeOH/CH₂Cl₂) afforded the aldehyde in 79% yield (1.02 g). TLC (silica, 10% MeOH/CH₂Cl₂): R_{*f*} = 0.67. MS (electrospray) calculated for C₂₁H₂₄F₃N₃O₃, 424.2 ([M+H]⁺), m/z found, 424.2. ¹H NMR (400 MHz, CDCl₃): 9.82 (s, 1 H), 7.65 (br d, J = 8.0 Hz, 2H), 7.54 (br s, 2H), 4.53 (s, 2H), 4.21 (t, J = 6.2 Hz, 2H), 3.68 (br s, 2H), 3.04 (t, J = 6.2 Hz, 2H), 2.70 (t, J = 5.6 Hz, 2H), 1.39 (s, 9H).

### E. 4-(2-Chloro-6-nitro-phenyl)-piperazine-1-carboxylic acid tert-butyl ester.

To a stirred solution of 0.96 g (5.0 mmol) of 1,2-dichloro-3-nitrobenzene and 0.93 g (5.0 mmol, 1 eq) of 1-*tert*-butyloxycarbonylpiperazine in acetonitrile (5 mL) was added 1.38 g (10 mmol, 2 eq) of K₂CO₃. The mixture was heated to reflux for 48 h. The solvent was removed under reduced pressure. The crude product was partitioned between EtOAc (100 mL) and 20 mL of H₂O. The organic layer was washed with H₂O (2 x 20 mL), dried over Na₂SO₄ and concentrated. Column chromatography (silica, 10-20% EtOAc/hexanes) provided 1.2 g (70%) of 4-(2-chloro-6-nitro-phenyl)-piperazine-1-carboxylic acid tert-butyl ester. TLC (silica, 20% EtOAc/hexanes): R_{*f*}= 0.45. ¹H NMR (400 MHz, CDCl₃): 7.56 (dd, J = 8.2, 1.4 Hz, 1 H), 7.50 (dd, J = 8.2, 1.4 Hz, 1 H), 7.13 (t, J = 8.2 Hz, 1H), 3.56-3.38 (m, 4H), 3.10-3.00 (m, 4H), 1.48 (s, 9H).

### F. 1-{3-[4-(2-Chloro-6-nitro-phenyl)-piperazin-1-yl]-propyl}-3-(4-trifluoromethylphenyl)-1,4,6,7-tetrahydro-pyrazolo[4,3-c]pyridine-5-carboxylic acid tert-butyl ester.

4-(2-Chloro-6-nitro-phenyl)-piperazine-1-carboxylic acid tert-butyl ester (940 mg, 2.75 mmol) in 10 mL of CH₂Cl₂ was treated with 5 mL of trifluoroacetic acid and stirred at 25 °C for 1 h. The volatiles were then removed. The residue was taken up in CH₂Cl₂ (60 mL) and KOH (4 *N*, 20 mL). The organic layer was separated, dried over Na₂SO₄, and concentrated. The yellow oil was dissolved in CH₂Cl₂ and added into the 996 mg (2.35 mmol) of 1-(3-oxo-propyl)-3-(4-trifluoromethyl-phenyl)-1,4,6,7-tetrahydro-pyrazolo[4,3-c]pyridine-5-carboxy lic acid tert-butyl ester. The yellow solution was treated with glacial acetic acid (0.8 mL, 6 eq) and stirred at 25 °C for 1 h. NaBH(OAC)₃ (1.5 g, 7.05 mmol) was added and stirred under nitrogen for 2 h. Then saturated NaHCO₃ (20 mL) was added and stirred for 30 min, and the layers were separated. The organic extract was washed with brine, dried over Na₂SO₄, and concentrated under reduced pressure. Column chromatography (silica, 2-5% MeOH/CH₂Cl₂) afforded 1-{3-[4-(2-chloro-6-nitro-phenyl)-piperazin-1-yl]-propyl}-3-(4-trifluoromethyl-phenyl)-1,4,6,7-tetrahydro-pyrazolo[4,3-c]pyridine-5-carboxylic ' acid tert-butyl ester as a white solid (1.40 g, 92%). TLC (silica, 5% MeOH/CH₂Cl₂): R_{f} = 0.3. MS (electrospray): exact mass calculated for C₃₁H₃₆CIF₃N₆O₄, 648.24; m/z found 649.3 [M+H]⁺. ¹H NMR (400 MHz, CDCl₃): 7.69 (d, J = 8.2 Hz, 1 H), 7.60-7.50 (m, 1H), 7.45-7.37 (m, 4H), 7.02 (t, J = 8.2 Hz, 1 H), 4.58 (br s, 2H), 4.04 (t, J = 6.7 Hz, 2H), 3.73-3.65 (m, 2H), 3.05-2.95 (m, 4H), 2.71 (t, J = 5.6 Hz, 2H), 2.50-2.35 (m, 4H), 2.30 (t, J = 6.8 Hz, 2H), 2.05-1.95 (m, 2H), 1.41 (s, 9H).

### G. 1-{3-[4-(2-Amino-6-chloro-phenyl-piperazin-1-yl]-propyl)-3-(4-trifluoromethyl-phenyl)-1,4,6,7-tetrahydro-pyrazolo[4,3-c]pyridine-5-carboxylic acid tert-butyl ester.

A solution of 360 mg (0.56 mmol) of 1-{3-[4-(2-chloro-6-nitro-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester in 4 mL of MeOH was treated with 182 mg (5 eq) of zinc dust and glacial acetic acid (1.57 mL, 50 eq) at 25 °C. The reaction mixture was stirred at 25 °C for 1 h. The reaction mixture was then filtered through a pad of celite and concentrated to obtain a thick oil. The residue was taken up in CH₂Cl₂ (50 mL) and sat. NaHCO₃ (20 mL). The organic layer was separated, washed with H₂O (2x 10 mL), dried over Na₂SO₄, and concentrated to afford 1-{3-[4-(2-amino-6-chloro-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester. TLC (silica, 10% MeOH/CH₂Cl₂): R_{*f*} = 0.3. MS (electrospray): exact mass calculated for C₃₁H₃₈CIF₃N₆O₂, 618.27; m/z found, 619.3 [M+H]⁺.

### H. 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester.

A solution of 1-{3-[4-(2-amino-6-chloro-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester (257 mg, 0.42 mmol) in 4 mL of CH₂Cl₂ was treated with 32 L (0.42 mmol, 1.0 eq) of methanesulfonyl chloride and 116 L (0.83 mmol, 2 eq) of triethylamine and the reaction mixture stirred at 25 °C for 1 h. EtOAc (40 mL) and sat. NaHCO₃ (20 mL) were added. The organic layer was separated and washed with H₂O (20 mL), brine (20 mL), dried over Na₂SO₄, and concentrated to afford the crude 1-{3-[4-(2-chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester. TLC (silica, 10% MeOH/CH₂Cl₂): R_{f}= 0.3. MS (electrospray): exact mass calculated for C₃₂H₄₀ClF₃N₆O₄S, 696.25; *m*/*z* found, 697.2 [M+H]⁺.

### I. 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-tert-butoxycarbonyl-sulfonic acid amide.

A solution of 97 mg (0.14 mmol) of 1-{3-[4-(2-chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-carboxylic acid tert-butyl ester in 3 mL of CH₂Cl₂ was treated with 1.5 mL of trifluoroacetic acid. The reaction mixture was stirred at 25 °C for 1 h before all volatiles were removed. To this crude material in 0.5 mL of CH₂Cl₂ was added dropwise a premixed solution of chlorosulfonyl isocyanate (18 µL, 0.209 mmol) and 2-methyl-2-propanol (20 µL, 0.209 mmol) in CH₂Cl₂ (0.150 mL). The reaction mixture was allowed to stir at 25 °C overnight. Preparative TLC (silica, 2-10% MeOH/CH₂Cl₂) provided the title compound (84 mg, 78%). TLC (silica, 10% MeOH/CH₂Cl₂): R_{*f*} = 0.3. MS (electrospray): exact mass calculated for C₃₂H₄₁ClF₃N₇O₆S₂, 775.22; *m*/*z* found, 776.2 [M+H]⁺.

### J. 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-sulfonic acid amide.

1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-tert-butoxycarbonyl-sulfonic acid amide (84 mg, 0.11 mmol) was dissolved in trifluoroacetic acid (0.75 mL) and CH₂Cl₂ (0.75 mL). The reaction mixture was allowed to stir at 25 °C for 2 h. Removal of volatiles under a stream of nitrogen provided 1-{3-[4-(2-chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-sulfonic acid amide in quantitative yield as a trifluoroacetic acid salt. MS (electrospray): exact mass calculated for C₂₇H₃₃ClF₃N₇O₄S₂, 675.17; *m*/*z* found, 676.2 [M+H]⁺. ¹H NMR (400 MHz, CDCl₃): 7.73 and 7.63 (AB pattern, *J* = 8.2 Hz, 4H), 7.37 (d, J = 7.8 Hz, 1 H), 7.13 (t, J = 7.8 Hz, 1 H), 7.04 (d, J = 7.8 Hz, 1 H), 4.32 (s, 2H), 4.20 (t, J = 6.3 Hz, 2H), 3.87-3.80 (m, 2H), 3.80-3.75 (m, 4H), 3.70-3.25 (m, 7H), 3.00-2.75 (m, 4H), 2.25-2.15 (m, 2H).

### EXAMPLE 19

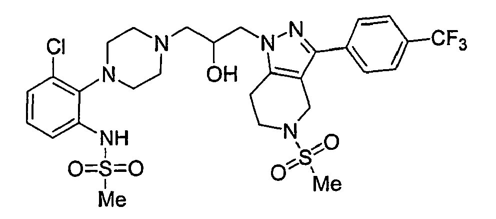

### N-[3-Chloro-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide.

### A. 5-Methanesulfonyl-1-oxiranylmethyl-3-(4-trifluoromethyl-phenyl)-4,5,6,7-tetrahydro-1 H-pyrazolo[4,3-c]pyridine.

5-Methanesulfonyl-3-(4-trifluoromethyl-phenyl)-4,5,6,7-tetrahydro-1H-pyrazolo[4,3-c]pyridine (10.0 g, 29.0 mmol) and epichlorohydrin (24 mL, 307 mmol) were set stirring in DMF (150 mL) containing Cs₂CO₃ (10.4 g, 31.9 mmol). After stirring at room temperature for 4 days the mixture was evaporated, brought up in EtOAc and washed with water. The organics were dried (MgSO₄) and evaporated to give a light yellow solid. Column chromatography (silica, 5% acetone/CH₂Cl₂) gave 4.1g (35%) of a white solid. TLC (silica, 5% acetone/CH₂Cl₂): R_{f} =0.28. MS (electrospray): exact mass calculated for C₁₇H₁₈F₃N₃O₃S, 401.10; *m*/*z* found, 402.1 [M+H]⁺. ¹H NMR (400 MHz, CDCl₃): 7.84 (d, *J* = 8.3 Hz, 2H), 7.79 (d, *J* = 8.3 Hz, 2H), 4.70-4.62 (m, 3H), 4.25 (d, J = 5.4 Hz, 1 H), 3.90-3.70 (m, 2H), 3.47 (m, 1 H), 3.10-2.9 (m, 6H), 2.65-2.60 (m, 1H).

### B. 4-(2-Chloro-6-nitro-phenyl)-piperazine-1-carboxylic acid tert-butyl ester.

To a stirred solution of 0.96 g (5.0 mmol) of 1,2-dichloro-3-nitrobenzene and 0.93 g (5.0 mmol, 1 eq) of 1-*tert*-butyloxycarbonylpiperazine in acetonitrile (5 mL) was added 1.38 g (10 mmol, 2 eq) of K₂CO₃. The mixture was heated to reflux for 48 h. The solvent was removed under reduced pressure. The crude product was partitioned between EtOAc (100 mL) and 20 mL of H₂O. The organic layer was washed with H₂O (2 x 20 mL), dried over Na₂SO₄ and concentrated. Column chromatography (silica, 10-20% EtOAc/hexanes) provided 1.2 g (70%) of 4-(2-chloro-6-nitro-phenyl)-piperazine-1-carboxylic acid tert-butyl ester. TLC (silica, 20% EtOAc/hexanes): R_{*f*}= 0.45. ¹H NMR (400 MHz, CDCl₃): 7.56 (dd, J = 8.2, 1.4 Hz, 1 H), 7.50 (dd, J = 8.2, 1.4 Hz, 1H), 7.13 (t, J = 8.2 Hz, 1 H), 3.56-3.38 (m, 4H), 3.10-3.00 (m, 4H), 1.48 (s, 9H).

### C. 4-(2-Amino-6-chloro-phenyl)-piperazine-1-carboxylic acid tert-butyl ester.

A solution of 342 mg (1 mmol) of 4-(2-chloro-6-nitro-phenyl)-piperazine-1-carboxylic acid tert-butyl ester in 5.0 mL of MeOH was treated with 630 mg (10 mmol, 10 eq) of ammonium formate and a catalytic amount of 10% Pd-C (34 mg). The reaction mixture was stirred at 65 °C for 30 min. The reaction mixture was then filtered through a pad of celite and concentrated to obtain a yellow solid. TLC (silica, 5% acetone/CH₂Cl₂): R_{*f*}=0.40. MS (electrospray): exact mass calculated for C₁₅H₂₂ClN₃O₂, 311.14; m/z found, 312.1 [M+H]⁺.

### D. 4-(2-Chloro-6-methanesulfonylamino-phenyl)-piperazine-1-carboxylic acid tert-butyl ester.

4-(2-Amino-6-chloro-phenyl)-piperazine-1-carboxylic acid tert-butyl ester (163 mg, 0.53 mmol) in CH₂Cl₂ was treated with 62 L (0.80 mmol, 1.5 eq) of methanesulfonyl chloride and 148 L (1.06 mmol, 2 eq) of triethylamine and the reaction mixture stirred at 25 °C for 1 h. EtOAc (40 mL) and sat. NaHCO₃ (20 mL) were added. The organic layer was separated and washed with H₂O (20 mL), brine (20 mL), dried over Na₂SO₄, and concentrated. Column chromatography (silica, 0-5% acetone/CH₂Cl₂) provided 145 mg (70%) of 4-(2-chloro-6-methanesulfonylamino-phenyl)-piperazine-1-carboxylic acid tert-butyl ester. TLC (silica, 5% acetone/CH₂Cl₂): R_{*f*} = 0.35. MS (electrospray): exact mass calculated for C₁₆H₂₄ClN₃O₄S, 389.12; m/z found, 388.1 (negative). ¹H NMR (400 MHz, CDCl₃): 7.41 (dd, J = 8.2, 1.6 Hz, 1 H), 7.11 (t, J = 8.2 Hz, 1 H), 6.99 (dd, J = 8.2, 1.6 Hz, 1 H), 4.25-3.91 (m, 2H), 3.66-3.52 (m, 2H), 3.01 (s, 3H), 3.01-2.84 (m, 2H), 2.70-2.56 (m, 2H), 2.55-2.43 (m, 2H), 1.44 (s, 9H).

### E. N-[3-Chloro-2-(4-{2-hydroxy-3-[5-methanesulfonyl-3-(4-trifluoromethylphenyl)-4,5,6,7-tetrahydro-pyrazolo[4,3-c]pyridin-1-yl]-propyl}-piperazin-1-yl)-phenyl]-methanesulfonamide.

4-(2-Chloro-6-methanesulfonylamino-phenyl)-piperazine-1-carboxylic acid tert-butyl ester (145 mg, 0.37 mmol) was dissolved in 3 mL of CH₂Cl₂ and treated with 1.5 mL of trifluoroacetic acid. The reaction mixture was stirred at 25 °C for 1 h before all volatiles were removed. The solid was treated with CH₂Cl₂ (20 mL) and aqueous KOH (4 *N,* 10 mL). The organic layer was separated, dried over Na₂SO₄, and concentrated. The crude oil (90 mg) was dissolved in absolute EtOH (1.0 mL) and treated with 96 mg (0.24 mmol) of 5-methanesulfonyl-1-oxiranylmethyl-3-(4-trifluoromethyl-phenyl)-4,5,6,7-tetrahydro-1H-pyrazolo[4,3-c]pyridine. The reaction mixture was refluxed at 85 °C for 3 h and then the solvent was removed. Column chromatography (silica, 0-5% MeOH/CH₂Cl₂) provided 138 mg (20% over 4 steps) of N-[3-chloro-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide. TLC (silica, 5% MeOH/CH₂Cl₂): R_{*f*} = 0.45. MS (electrospray): exact mass calculated for C₂₈H₃₄ClF₃N₆O₅S₂, 690.17; m/z found, 691.2 [M+H]⁺. ¹H NMR (400 MHz, CDCl₃): 8.28 (s, 1 H), 7.65 and 7.59 (AB pattern, J = 8.4 Hz, 4H), 7.36 (d, J = 8.1 Hz, 1 H), 7.07 (t, J = 8.2 Hz, 1 H), 6.95 (d, J = 8.2 Hz, 1 H), 4.54-4.44 (m, 2H), 4.21-3.94 (m, 3H), 3.77-3.52 (m, 4H), 3.41 (m, 2H), 2.96 (s, 3H), 2.81 (s, 3H), 3.05-2.73 (m, 4H), 2.66-2.20 (m, 4H).

### EXAMPLE 20

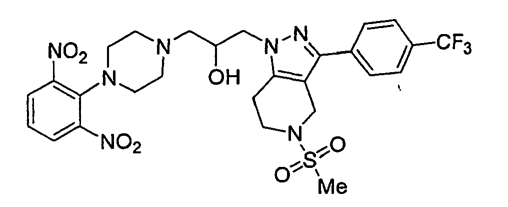

### 1-[4-(2,6-Dinitro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

### A. 4-(2,6-Dinitro-phenyl)-piperazine-1-carboxylic acid tert-butyl ester.

To a stirred solution of 1.01 g (5.0 mmol) of 1-chloro-2, 6-dinitrobenzene and 0.93 g (5.0 mmol) of 1-*tert*-butyloxycarbonylpiperazine in acetonitrile (5 mL) was added 1.38 g (10 mmol) of K₂CO₃. The mixture was heated to reflux for 48 h. The solvent was removed under reduced pressure. The crude product was partitioned between EtOAc (100 mL) and 20 mL of H₂O. The organic layer was washed with H₂O (2 x 20 mL), dried over Na₂SO₄ and concentrated. Column chromatography (silica, 10-20% EtOAc/hexanes) provided 1.31 g (85%) of 4-(2,6-dinitro-phenyl)-piperazine-1-carboxylic acid tert-butyl ester TLC (silica, 20% EtOAc/hexanes): R_{*f*} = 0.35. ¹H NMR (400 MHz, CDCl₃): 7.75 (d, J = 8.2 Hz, 2H), 7.25 (t, *J* = 8.2 Hz, 1 H), 3.30 (m, 4H), 2.95 (m, 2H), 1.44 (s, 9H).

### B. 1-[4-(2,6-Dinitro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

4-(2,6-Dinitro-phenyl)-piperazine-1-carboxylic acid tert-butyl ester (220 mg, 0.63 mmol) was dissolved in 5.0 mL of CH₂Cl₂ and treated with 3.0 mL of trifluoroacetic acid. The reaction mixture was stirred at 25 °C for 1 h before all volatiles were removed. The solid was treated with CH₂Cl₂ (20 mL) and aqueous KOH (4 *N*, 10 mL). The organic layer was separated, dried over Na₂SO₄, and concentrated. The crude oil (67 mg) was dissolved in absolute EtOH (1.2 mL) and treated with 141 mg (0.35 mmol, 1.3 eq) of 5-methanesulfonyl-1-oxiranylmethyl-3-(4-trifluoromethyl-phenyl)-4,5,6,7-tetrahydro-1 H-pyrazolo[4,3-c]pyridine. The reaction mixture was refluxed at 85 °C for 3 h and then the solvent was removed. Column chromatography purification (silica, 10-20% acetone/CH₂Cl₂) provided 150 mg (85%) of 1-[4-(2,6-dinitro-phenyl)-piperazin-1-yl]-3-[5-methanesulfonyl-3-(4-trifluoromethylphenyl)-4,5,6,7-tetrahydro-pyrazolo[4,3-c]pyridin-1-yl]-propan-2-ol. TLC (silica, 10% acetone/CH₂Cl₂): R_{f} = 0.3. MS (electrospray): exact mass calculated for C₂₇H₃₀F₃N₇O₇S, 653.19; *m*/*z* found, 654.2 [M+H]⁺. ¹H NMR (400 MHz, CDCl₃): 7.71 (d, J = 8.2 Hz, 2H), 7.64 and 7.58 (AB pattern, J = 8.4 Hz, 4H), 7.20 (t, J = 8.2 Hz, 1H), 4.54 (s, 2H), 4.29-4.12 (m, 2H), 3.66 (t, *J* = 5.3 Hz, 2H), 3.70-2.95 (m, 9H), 2.91 (s, 3H), 2.67-2.32 (m, 4H).

### EXAMPLE 21

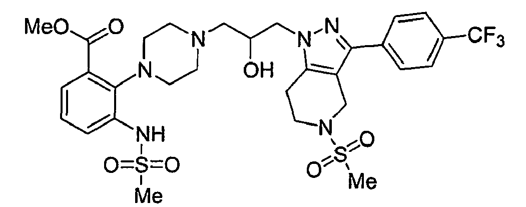

### 2-(4-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-3-methanesulfonylamino-benzoic acid methyl ester.

### A. 4-(2-Methoxycarbonyl-6-nitro-phenyl)-piperazine-1-carboxylic acid tert-butyl ester.

To a stirred solution of 736 mg (2.83 mmol) of ethyl 2-bromo-3-nitrobenzoate and 579 mg (3.1 mmol, 1.1 eq) of 1-*tert*-butyloxycarbonylpiperazine in n-butanol (6 mL) was added 330 mg (3.1 mmol, 1.1 eq) of Na₂CO₃. The mixture was heated to reflux for 4 h. The solvent was removed under reduced pressure. The crude product was partitioned between EtOAc (100 mL) and 20 mL of H₂O. The organic layer was washed with H₂O(2 x 20 mL), dried over Na₂SO₄ and concentrated. Column chromatography (silica, 10-20% EtOAc/hexanes) provided 744 mg (72%) of 4-(2-methoxycarbonyl-6-nitrophenyl)-piperazine-1-carboxylic acid tert-butyl ester. TLC (silica, 20% EtOAc/hexanes): R_{*f*}= 0.5. ¹H NMR (400 MHz, CDCl₃): 7.67 (dd, J = 8.2,1.4 Hz, 1 H), 7.62 (dd, J = 8.2, 1.4 Hz, 1 H), 7.16 (t, J = 8.2 Hz, 1 H), 3.86 (s, 3H), 3.44-3.36 (m, 4H), 3.03-2.95 (m, 4H), 1.48 (s, 9H).

### B. 2-(4-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1- yl)-3-methanesulfonylamino-benzoic acid methyl ester.

A solution of 1.0 g (2.73 mmol) of 4-(2-methoxycarbonyl-6-nitro-phenyl)-piperazine-1-carboxylic acid tert-butyl ester in 18 mL of MeOH was treated with 893 mg (13.7 mmol, 5 eq) of zinc dust and glacial acetic acid (8 mL). The reaction mixture was stirred at 25 °C for 1 h. The reaction mixture was then filtered through a pad of celite and concentrated to obtain a thick oil. The residue was taken up in EtOAc (200 mL) and sat. NaHCO₃ (100 mL). The organic layer was separated, washed with H₂O (2x 50 mL), dried over Na₂SO₄, and concentrated. Column chromatography (silica, 10-30% EtOAc/hexanes) provided the desired amine (844 mg, 92%). The amine (42 mg, 0.13 mmol) in CH₂Cl₂ (0.5 mL) was treated with 9.7 µL (0.13 mmol, 1.0 eq) of methanesulfonyl chloride and 34.9 µL (0.25 mmol, 2 eq) of triethylamine and the reaction mixture stirred at 25 °C for 1 h. EtOAc (20 mL) and sat. NaHCO₃ (10 mL) were added. The organic layer was separated and washed with H₂O (10 mL), brine (20 mL), dried over Na₂SO₄, and concentrated. The crude oil was dissolved in 2 mL of CH₂Cl₂ and treated with 0.5 mL of trifluoroacetic acid. The reaction mixture was stirred at 25 °C for 1 h before all volatiles were removed. The crude oil was dissolved in absolute EtOH (1.0 mL) and treated with 40 mg (0.1 mmol) of 5-methanesulfonyl-1-oxiranylmethyl-3-(4-trifluoromethyl-phenyl)-4,5,6,7-tetrahydro-1H-pyrazolo[4,3-c]pyridine and 200 µL of triethylamine. The reaction mixture was refluxed at 85 °C for 4 h and then the solvent was removed. Preparative TLC (silica, 7% MeOH/CH₂Cl₂) provided 35 mg (49% over 3 steps) of the title compound. TLC (silica, 5% MeOH/CH₂Cl₂): R_{*f*} = 0.30. MS (electrospray): exact mass calculated for C₃₀H₃₇F₃N₆O₇S₂, 714.21; *m*/*z* found, 715.2 [M+H]⁺. ¹H NMR (400 MHz, CDCl₃): 8.11 (s, 1 H), 7.74-7.59 (m, 5H), 7.30 (d, *J =* 8.1 Hz, 1H), 7.21 (t, *J =* 8.2 Hz, 1 H), 4.62-4.49 (m, 2H), 4.25-3.99 (m, 3H), 3.90 (s, 3H), 3.80-3.57 (m, 3H), 3.53-3.27 (m, 2H), 3.14-2.78 (m, 4H), 3.05 (s, 3H), 2.86 (s, 3H), 2.76-2.65 (m, 2H), 2.61-2.20 (m, 4H).

### EXAMPLE 22

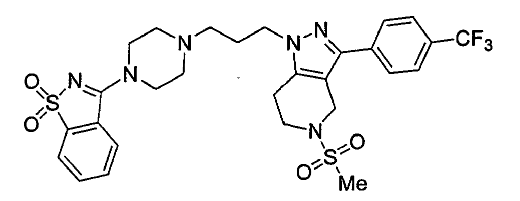

### 1-{3-[4-(1,1-Dioxo-1H-116-benzo[d]isothiazol-3-yl)-piperazin-1-yl]-propyl}-5-methanesulfonyl-3-(4-trifluoromethyl-phenyl)-4,5,6,7-tetrahydro-1 H-pyrazolo[4,3-c]pyridine.

### A. 3-Piperazin-1-yl-benzo[d]isothiazole 1.1-dioxide.

POCl₃ (10.2 mL, 109.2 mmol) was added to saccharin (5.0 g, 27.3 mmol) and heated at 120 °C for 20 h. The excess reagent was removed in a rotary evaporator and water (50 mL) was added to the residue to form a precipitate. The solid was filtered, washed with water (2 x 20 mL), and dried. A portion of the above crude material (2.0 g, 9.95 mmol) and piperazine (4.28 g, 49.75 mmol) was taken in dioxane (10 mL), and heated at 100 °C for 24 h. The reaction was allowed to cool to room temperature and poured into ice water (50 g), and neutralized by addition of 10% aqueous NaOH. The mixture was extracted with CH₂Cl₂ (3 x 25 mL) and the combined organic extracts were washed with brine, dried (Na₂SO₄) and concentrated. MPLC (silica, 5-20% MeOH/CH₂Cl₂) afforded the piperazinyl derivative (0.07 g, 4.2%). MS (electrospray): exact mass calculated for C₁₁H₁₃N₃O₂S, 251.07; m/z found, 252.1 [M+H]⁺. ¹H NMR (400 MHz, CDCl₃): 7.72 (dd, J = 0.8, 7.4 Hz, 1 H), 7.64 (d, J = 7.8 Hz, 1 H), 7.49 (dt, J = 0.8, 7.4 Hz, 1 H), 7.43 (dt, J = 1.2, 7.8 Hz, 1 H), 3.80 (s, 4H), 2.85 (br t, J = 5.0 Hz, 4H), 2.07 (br s, 1 H). ¹³C NMR (100 MHz, CDCl₃): 160.8, 145.3, 133.3, 133.0, 128.5, 125.9, 123.2, 49.8, 46.3.

### B. 1-{3-[4-(1,1-Dioxo-1H-1I6-benzo[d]isothiazol-3-yl)-piperazin-1-yl]-propyl}-5-methanesulfonyl-3-(4-trifluoromethyl-phenyl)-4,5,6,7-tetrahydro-1H-pyrazolo[4.3-c]pyridine.

To a stirred solution of 3-[5-methanesulfonyl-3-(4-trifluoromethyl-phenyl)-4,5,6,7-tetrahydro-pyrazolo[4,3-c]pyridin-1-yl]-propionaldehyde (0.040 g, 0.13 mmol) and 3-piperazin-1-yl-benzo[d]isothiazole 1,1-dioxide (0.050 g, 0.21 mmol) in CH₂Cl₂ (0.5 mL), glacial AcOH (12 µL, 0.21 mmol) was added and stirred for 15 min at room temperature. NaBH(OAc)₃ (0.058 g, 0.27 mmol) was added and stirred under nitrogen overnight. Saturated NaHCO₃ (0.5 mL) was then added and stirred for 15 min. The layers separated and the aqueous layer was extracted with CH₂Cl₂ (0.5 mL). MPLC (silica, 2-15% MeOH/CH₂Cl₂) afforded the desired product as a white solid (0.048 g, 76%). TLC (silica, 12% MeOH/CH₂Cl₂): R_{*f*} = 0.50. MS (electrospray): exact mass calculated for C₂₈H₃₁F₃N₆O₄S₂, 636.18; *m*/*z* found, 637.2 [M+H]⁺. ¹H NMR (400 MHz, CDCl₃): 7.94 (dd, J = 0.8, 7.6 Hz, 1 H), 7.86 (d, J = 7.8 Hz, 1 H), 7.75 (d, J = 8.3 Hz, 2H), 7.73-7.63 (m, 2H), 7.68 (d, J = 8.3 Hz, 2H), 4.57 (s, 2H), 4.17 (t, J = 6.9 Hz, 2H), 4.04 (br s, 4H), 3.69 (t, J = 5.7 Hz, 2H), 2.94 (s, 3H), 2.92 (t, J = 6.2 Hz, 2H), 2.62 (t, J = 5.0 Hz, 4H), 2.44 (t, J = 6.6 Hz, 2H), 2.13 (q, J = 6.6 Hz, 2H).

### EXAMPLE 23

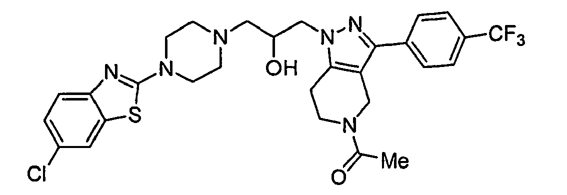

### 1-[1-{3-[4-(6-Chloro-benzothiazol-2-yl)-piperazin-1-yl]-2-hydroxy-propyl}-3-(4-trifluoromethyl-phenyl)-1,4,6,7-tetrahydro-pyrazolo[4,3-c]pyridin-5-yl]-ethanone

### A. 6-Chloro-2-piperazin-1-yl-benzothiazole.

To a stirred solution of 1.07 g (5.24 mmol) of 2,6-dichlorobenzothiazole in dry DMF (25 mL) was added 2.4 g of potassium carbonate (15.7 mmol) and 0.5 g of piperazine (5.8 mmol). The mixture was stirred at room temperature for 4 h. When the reaction was complete it was partitioned between EtOAc (150 mL) and water (50 mL) and separated. The aqueous layer was extracted with EtOAC (2 x 100 mL). The combined organic layers were then washed with water (2 x 25 mL), brine, dried over Na₂SO₄, and the solvent was removed under reduced pressure to give 1.33 g (100%) of desired product as a white solid. MS (electrospray): exact mass calculated for C₁₁H₁₂ClN₃S, 253.04; m/z found, 254.0 [M+H]⁺.

### B. 1-[1-{3-[4-(6-Chloro-benzothiazol-2-yl)-piperazin-1-yl)-2-hydroxy-propyl}-3-(4-trifluoromethyl-phenyl)-1,4,6,7-tetrahydro-pyrazolo[4 3-c]pyridin-5-yl]-ethanone.

To a stirred solution of 144 mg (0.39 mmol) of 1-[1-oxiranylmethyl-3-(4-trifluoromethyl-phenyl)-1,4,6,7-tetrahydro-pyrazolo[4,3-c]pyridin-5-yl]-ethanone in 4 mL of EtOH was added 100 mg (0.39 mmol) 6-chloro-2-piperazin-1-yl-benzothiazole. The solution was heated to 60 °C overnight. The solvent was then removed by rotary evaporation and the crude product was purified by column chromatography (silica, 0-10% MeOH/EtOAc) to afford 220 mg (90%) of a white solid. MS (electrospray): exact mass calculated for C₂₉H₃₀ClF₃N₆O₂S: 618.18; *m*/*z* found, 619.2 [M+H]⁺. HPLC (reverse phase conditions 40-90%): t_{R} = 8.27 min. ¹H NMR (CDCl₃, 400 MHz, a mixture of amide rotamers): 7.70 (d, *J* = 8.34 Hz, 1 H), 7.62 (m, 2H), 7.57 (d, J = 8.59 Hz, 1 H), 7.48 (d, J = 2.53 Hz, 1 H), 7.36 (d, *J =* 8.59 Hz, 1H), 7.16 (dd, *J =* 8.59, 2.53 Hz, 1 H), 4.80 and 4.68 (A and B of AB quartet, *J* = 15.92 Hz, 1H), 4.58 (s, 1H), 4.18-4.08 (m, 2H), 4.01-3.89 (m, 2H), 3.85-3.60 (m, 2H), 3.59-3.47 (m, 4H), 2.94-2.75 (m, 2H), 2.72-2.62 (m, 2H), 2.55-2.47 (m, 2H), 2.46-2.39 (m, 2H), 2.13 (s, 1.5H), 2.08 (s, 1.5H).

### EXAMPLE 24

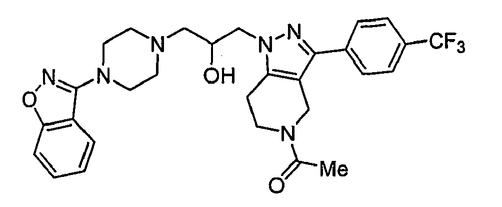

### 1-[1-[3-(4-Benzo[d]isoxazol-3-yl-piperazin-1-yl)-2-hydroxy-propyl]-3-(4-trifluoromethyl-phenyl)-1,4,6,7-tetrahydro-pyrazolo[4,3-c]pyridin-5-yl]-ethanone.

### A. 4-Benzo[d]isoxazol-3-yl-piperazine-1-carboxylic acid tert-butyl ester.

To a stirred solution of 100 mg (0.65 mmol) of 3-chloro-1,2-benzisoxazole in pyridine (1 mL) was added 145 mg of piperazine-1-carboxylic acid tert-butyl ester (0.78 mmol) and 0.18 mL of DBU (0.78 mmol). The mixture was stirred at 100 °C overnight and then partitioned between EtOAC (50 mL) and water (20 mL) and separated. The aqueous layer was extracted with EtOAC (2 x 30 mL). The combined organic layers were then washed with water (25 mL), brine, dried over Na₂SO₄, and the solvent was removed under reduced pressure to give crude product. Purification by column chromatography (silica, 60-100%CH₂Cl₂/hexanes) gave 82 mg (42%) of the desired product as a light yellow solid. MS (electrospray): exact mass calculated for C₁₆H₂₁N₃O₃, 303.16; *m*/*z* found, 326.1 [M+Na]⁺. ¹H NMR (CDCl₃, 400 MHz): 7.68 (dt, J = 8.02, 0.98 Hz, 1 H), 7.52-7.44 (m, 2H), 7.24 (ddd, J = 8.42, 6.46, 1.57 Hz, 1 H), 3.66-3.61 (m, 4H), 3.56-3.49 (m, 4H), 1.49 (s, 9H).

### B. 1-[1-[3-(4-Benzo[d]isoxazol-3-yl-piperazin-1-yl)-2-hydroxy-propyl]-3-(4-trifluoromethyl-phenyl)-1,4.6,7-tetrahydro-pyrazolo[4,3-c]pyridin-5-yl]-ethanone,

A solution of 82 mg (0.27 mmol) of 4-benzo[d]isoxazol-3-yl-piperazine-1-carboxylic acid tert-butyl ester in 2 mL of CH₂Cl₂was treated with trifluoroacetic acid (0.5 mL) at room temperature overnight. The solvent was then removed and the crude product dissolved in EtOH and stirred over 100 mg of sodium bicarbonate for 1 h, the solid was then filtered off and the filtrate concentrated. The crude piperazine was then dissolved in 4 mL EtOH and treated with 100 mg (0.27 mmol) of 1-[1-oxiranylmethyl-3-(4-trifluoromethyl-phenyl)-1,4,6,7-tetrahydro-pyrazolo[4,3-c]pyridin-5-yl]-ethanone. The solution was heated to 60 °C overnight. The solvent was then removed by rotary evaporation and the crude product was purified by column chromatography (silica, 0-10% MeOH/EtOAc) to afford 105 mg (68%) of a white solid. MS (electrospray), exact mass calculated for C₂₉H₃₁F₃N₆O₃, 568.24; *m*/*z* found, 569.2 [M+H]⁺. ¹H NMR (CDCl₃, 400 MHz, a mixture of amide rotamers): 7.77 (d, *J* = 8.41 Hz, 1 H), 7.69 (m, 2H), 7.67-7.62 (m, 2H), 7.50-7.44 (m, 1 H), 7.45-7.42 (m, 1 H), 7.23-7.18 (m, 1H), 4.93 (br m, 1 H), 4.87 and 4.75 (A and B of AB quartet, *J =* 15.65 Hz, 1H), 4.65 (br s, 1H), 4.27-4.15 (m, 2.3H), 4.09-3.95 (m, 1.7H), 3.91-3.82 (m, 0.7H), 3.81-3.66 (m, 1.3H), 3.62-3.49 (m, 4H), 3.01-2.85 (m, 1.5H), 2.85-2.74 (m, 2.5H), 2.71-2.60 (m, 2H), 2.58-2.45 (m, 2H), 2.20 (s, 1.5H), 2.15 (s, 1.5H).

### EXAMPLE 25

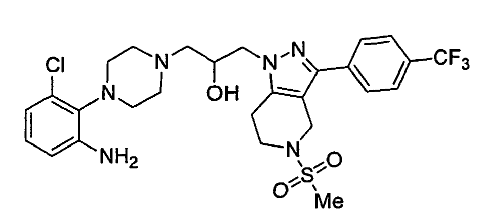

### 1-[4-(2-Amino-6-chloro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

### EXAMPLE 26

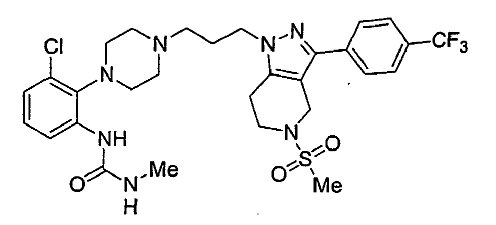

### 1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-3-methyl-urea.

### EXAMPLE 27

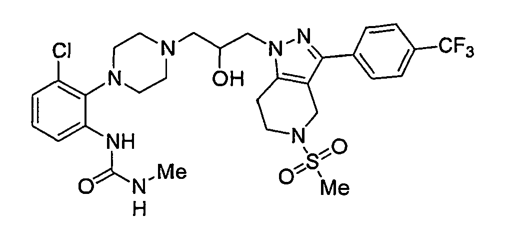

### 1-[3-Chloro-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-3-methyl-urea.

### EXAMPLE 28

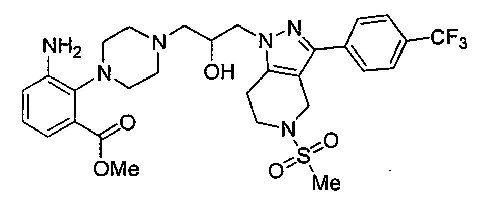

### 3-Amino-2-(4-{2-hydroxy-3-[5-methanesulfonyl-3-(4-trifluoromethyl-phenyl)-4,5,6,7-tetrahydro-pyrazolo[4,3-c]pyddin-1-yl]-propyl}-piperazin-1-yl)-benzoic acid methyl ester.

### EXAMPLE 29

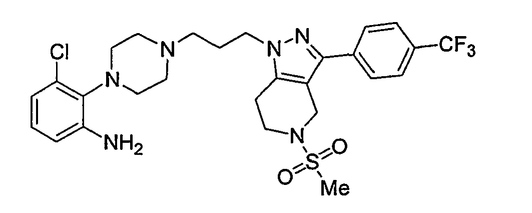

### 3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenylamine.

### EXAMPLE 30

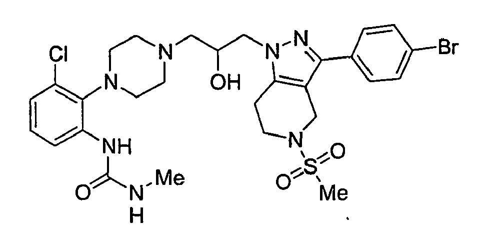

### 1-[2-(4-{3-[3-(4-Bromo-phenyl)-5-methanesulfonyl-4,5,6,7-tetrahydro-pyrazolo[4,3-c]pyridin-1-yl]-2-hydroxy-propyl}-piperazin-1-yl)-3-chloro-phenyl]-3-methyl-urea.

### EXAMPLE 31

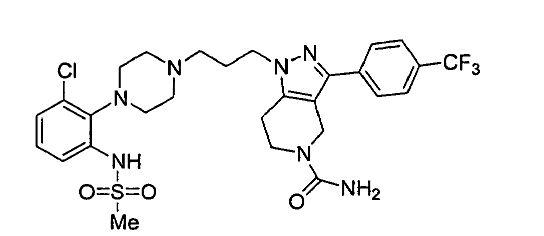

### 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-carboxylic acid amide.

### EXAMPLE 32

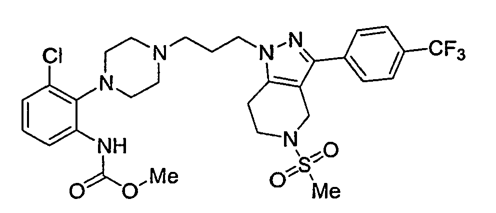

### [3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-carbamic acid methyl ester.

### EXAMPLE 33

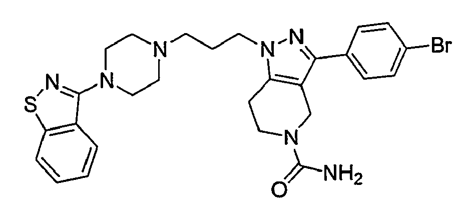

### 1-[3-(4-Benzo[d]isothiazol-3-yl-piperazin-1-yl)-propyl]-3-(4-bromo-phenyl)-1,4,6,7-tetrahydro-pyrazolo[4,3-c]pyridine-5-carboxylic acid amide.

### EXAMPLE 34

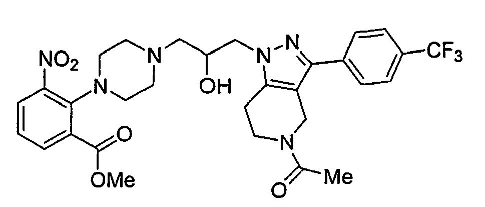

### 2-(4-{3-[5-Acetyl-3-(4-trifluoromethyl-phenyl)-4,5,6,7-tetrahydro-pyrazolo[4,3-c]pyridin-1-yl]-2-hydroxy-propyl}-piperazin-1-yl)-3-nitro-benzoic acid methyl ester.

### EXAMPLE 35

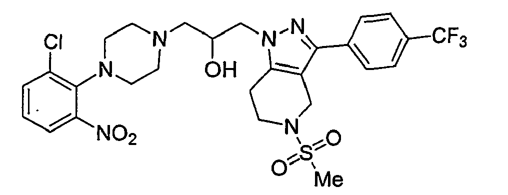

### 1-[4-(2-Chloro-6-nitro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol.

### EXAMPLE 36

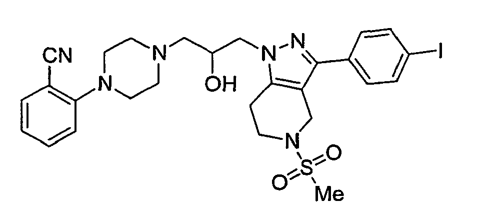

### 2-(4-{2-Hydroxy-3-[3-(4-iodo-phenyl)-5-methanesulfonyl-4,5,6,7-tetrahydro-pyrazolo[4,3-c]pyridin-1-yl]-propyl}-piperazin-1-yl)-benzonitrile.

### EXAMPLE 37

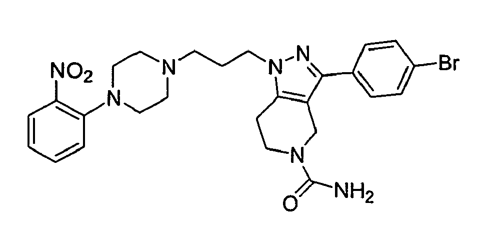

### 3-(4-Bromo-phenyl)-1-{3-[4-(2-nitro-phenyl)-piperazin-1-yl]-propyl}-1,4,6,7-tetrahydro-pyrazolo[4,3-c]pyridine-5-carboxylic acid amide.

### EXAMPLE 38

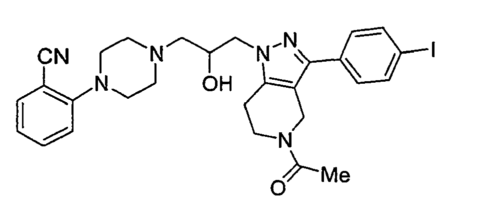

### 2-(4-{3-[5-Acetyl-3-(4-iodo-phenyl)-4,5,6,7-tetrahydro-pyrazolo[4,3-c]pyridin-1-yl]-2-hydroxy-propyl}piperazin-1-yl)-benzonitrile.

### EXAMPLE 39

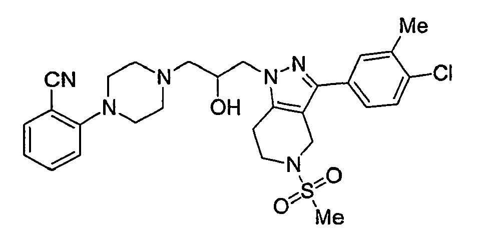

### 2-(4-{3-[3-(4-Chloro-3-methyl-phenyl)-5-methanesulfonyl-4,5,6,7-tetrahydro-pyrazolo[4,3-c]pyridin-1-yl]-2-hydroxy-propyl}-piperazin-1-yl)-benzonitrile.

### EXAMPLE 40

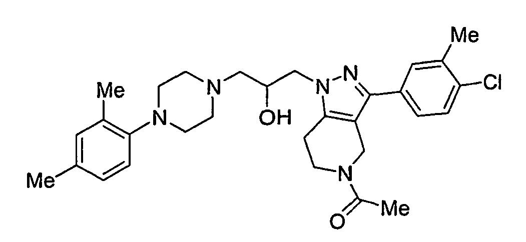

### 1-(3-(4-Chloro-3-methyl-phenyl)-1-{3-[4-(2,4-dimethyl-phenyl)-piperazin-1-yl]-2-hydroxy-propyl}-1,4,6,7-tetrahydro-pyrazolo[4,3-c]pyridin-5-yl)-ethanone.

### EXAMPLE 41

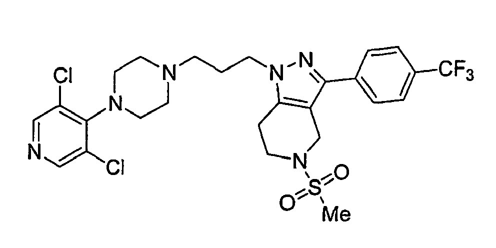

### 1-{3-[4-(3,5-Dichloro-pyridin-4-yl)-piperazin-1-yl]-propyl}-5-methanesulfonyl-3-(4-trifluoromethyl-phenyl)-4,5,6,7-tetrahydro-1H-pyrazolo[4,3-c]pyridine.

### EXAMPLE 42

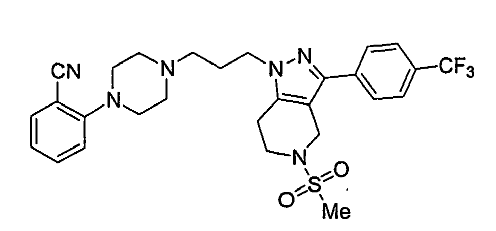

### 2-(4-{3-[5-Methanesulfonyl-3-(4-trifluoromethyl-phenyl)-4,5,6,7-tetrahydro-pyrazolo[4,3-c]pyridin-1-yl]-propyl}-piperazin-1-yl)-benzonitrile.

### EXAMPLE 43

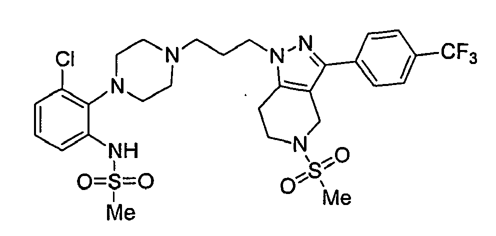

### N-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide.

### EXAMPLE 44

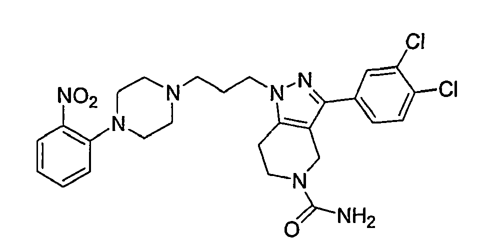

### 3-(3,4-Dichloro-phenyl)-1-{3-[4-(2-nitro-phenyl)-piperazin-1-yl]-propyl}-1,4,6,7-tetrahydro-pyrazolo[4,3-c]pyridine-5-carboxylic acid amide.

### EXAMPLE 45

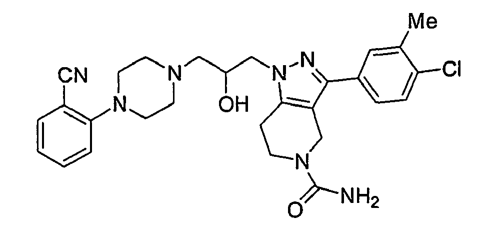

### 3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridine-5-carboxylic acid amide.

### EXAMPLE 46

### Cathepsin S Inhibition Assay.

Recombinant human cathepsin S (CatS) was expressed in the baculovirus system and purified in one step with a thiopropyl-sepharose column. 10-L yielded -700 mg of CatS and N-terminal sequencing confirmed identity. The assay is run in 100 mM sodium acetate pH 5.0 containing 1 mM DTT and 100 mM NaCl. The substrate for the assay is
(Aedens)EKARVLAEAA(Dabcyl)K-amide

The Kₘ for the substrate is around 5 µM but the presence of substrate inhibition makes kinetic analysis difficult. With 20 µM substrate the assay rate is linear over the range of 1-8 ng CatS in 100 µl reaction. Using 2 ng/well of CatS, the production of product is linear and yields ~7-fold signal after 20 min with only 20% loss of substrate. Primary assays are run by quenching the reaction after 20 min with 0.1 % SDS and then measuring the fluorescence. For other assays, measurements are taken every min for 20 min. The rate is calculated from the slope of the increase and the percent inhibition is calculated from this (See Tables 1 and 2 below).

**Table 1**

| EXAMPLE | IC₅₀ (µM) |
|---|---|
| 1 | 0.89 |
| 2 | 1.22 |
| 3 | 0.84 |
| 4 | 0.51 |
| 5 | 0.36 |
| 6 | 0.30 |
| 7 | 6.60 |
| 8 | 0.89 |
| 9 | 1.14 |
| 10 | 0.05 |
| 11 | 0.03 |
| 12 | 0.98 |
| 13 | 0.77 |
| 14 | 0.25 |
| 15 | 0.12 |
| 16 | 0.06 |
| 17 | 0.08 |
| 18 | 0.14 |
| 19 | 0.06 |
| 20 | 0.17 |
| 21 | 0.07 |
| 22 | 2.15 |
| 23 | 1.10 |
| 24 | 0.47 |
| 25 | 0.04 |
| 26 | 0.04 |
| 27 | 0.04 |
| 28 | 0.07 |
| 29 | 0.07 |
| 30 | 0.08 |
| 31 | 0.10 |
| 32 | 0.10 |
| 33 | 0.10 |
| 34 | 0.11 |
| 35 | 0.12 |
| 36 | 0.12 |
| 37 | 0.12 |
| 38 | 0.12 |
| 39 | 0.13 |
| 40 | 0.13 |
| 41 | 0.13 |
| 42 | 0.13 |
| 43 | 0.13 |
| 44 | 0.13 |
| 45 | 0.13 |

### Example 47

### Ex vivo inhibition by cathepsin S inhibitors of the allergenic response

The following assay demonstrates that cathepsin S inhibitors block the response of human T cells to crude allergen extracts.

### Materials and Methods.

Reagents. Glycerinated crude allergen extracts of house dust mites *(Dermataphagoides pteronyssinus, Dermataphagoides farinae)* and ragweed *[Ambrosia trifida* (giant), *Ambrosia artemisiifolia* (short)] were purchased from Hollister-Stier Laboratories (Minneapolis, MN). Concanavalin A (ConA) was purchased from Calbiochem (La Jolla, CA).

Donors. All allergic donors were prescreened for their specific allergies using RAST tests. The HLA class II haplotypes of these donors were determined using PCR.

Cell culture. Human peripheral blood mononuclear cells (PBMC) were purified from blood of allergic donors using Ficoll-Hypaque gradient followed by washes with phosphate buffered saline (PBS). PBMC were cultured in triplicate or duplicate at 0.5-1.0 x 10⁶ cells/well with titrated doses of allergen extracts, in the presence or absence of a known cathepsin S inhibitor, LHVS (morpholinurea-leucine-homo-phenylalanine-vinylsulfonephenyl) (Palmer et al. (1995), J. Med. Chem. 38:3193 and Riese et al. (1996), Immunity 4:357). Serial diluted stock solutions of LHVS were first made in 100% DMSO and then diluted 1:15 in 40% Hydroxypropynyl cyclodextrin (HPCD). Three microliters of LHVS in HPCD was added into PBMC cultures (200 µL/well). After 6 days of culture, 1 µCi/well of ³H-thymidine (TdR) was added. Eighteen hours later, cells were harvested using a Filtermate Harvester (Packard) and counted for ³H-TdR incorporation on Topcount (Packard).

### Inhibition of T cell proliferative responses to house dust mites.

About 10% of most populations are allergic to house dust mites (HDM) of the genus *Dermatophagoides* with *Dermatophagoides pteronyssinus* (Der p) and *D. farinae* (Der f) being the two major species present in varying proportions in most countries. The major clinical manifestations are asthma and perennial rhinitis.

Effect of cathepsin S inhibition on activation of HDM allergen-specific CD4 T cells was tested in an ex *vivo* human T cell-proliferation assay.
Culturing PBMC with crude extracts from either *Der p* or *Der f,* resulted in strong proliferation (Figure 1A). This proliferation consisted primarily of allergen-specific CD4 T cells. When cathepsin S activity was blocked by a specific cathepsin S inhibitor, LHVS (cf. Riese et al. (1996) Immunity 4:357) the proliferation was strongly inhibited (Figure 1 B). Inhibition by LHVS was specific for responses induced by HDM extracts since T cell proliferative responses induced by ConA, a pan-T cell mitogen, were not affected. Furthermore, this inhibition was observed for all four HDM-allergic donors tested regardless of the different HLA class II haplotypes (DR4; DR7, 15; DR11, 15; and DR4, 11).

This system is very similar to an *in vivo* situation. The allergic subject would be exposed to a crude mixture of allergens that would lead to the proliferation of T cells and an allergic response. The observation of inhibition of CD4 T cell activation by a cathepsin S inhibitor shows that such inhibitors can be effective in treating a generalized population of patients allergic to house dust mites.

### Inhibition of T cell proliferative responses to ragweed

About 10% of population in US are allergic to ragweed pollen, making it one of the most important allergens in terms of clinical diseases. Allergens from pollens are a common precipitant of rhinitis and asthma in this population.

The effect of cathepsin S inhibition on activation of ragweed allergen-specific CD4 T cells was tested in an ex *vivo* human T cell-proliferation assay. Culturing PBMC with crude extracts from both short and giant ragweed resulted in strong proliferation (Figure 2A). This proliferation consisted mainly of allergen-specific CD4 T cells. When cathepsin S activity was blocked by a specific cathepsin S inhibitor, LHVS (cf. Riese et al. (1996) Immunity 4:357) the proliferation was strongly inhibited (Figure 2B). Inhibition by LHVS was specific for responses induced by ragweed since T cell proliferative responses induced by ConA, a pan-T cell mitogen, were not affected. Furthermore, this inhibition was observed for the two ragweed-allergic donors tested regardless of the different HLA class II haplotypes (DR7, 15 and DR4, 11).

A similar experiment was run using two additional CatS inhibitors, compounds from Example 11 and Example 36 above, with the results shown in FIGS. 3A and 3B, respectively.

This system is very similar to an *in vivo* situation. The allergic subject would be exposed to a crude mixture of allergens that would lead to the proliferation of T cells and an allergic response. The observation of inhibition of CD4 T cell activation by a cathepsin S inhibitor shows that such inhibitors can be effective in treating a generalized population of patients allergic to ragweed.

### Example 48

### Monitoring cathepsin S inhibition in human blood.

The effect of *in vivo* administration of cathepsin S inhibitors, in a clinical trial setting, can be monitored by measuring accumulation of an intermediate degradation product of invariant chain (li), i.e. the p10li fragment, in blood of dosed subjects. After administration of a cathepsin inhibitor for a certain period of time, for example, between 0.01 and 50 mg/kg/day, to result in a blood concentration of between 1 nM-10 µM, for 16-30 h, blood is drawn and white blood cells are purified, e.g. either by lysis of red blood cells or by a Ficoll-Hypaque gradient centrifugation. Whole cell lysates of WBC are then made and analyzed by either a Westem blot assay or an ELISA assay. For the Western blot assay, cell lysates are first resolved on SDS-PAGE gels. After transferring to nitrocellulose membranes, li and its intermediate degradation products, including the p10li, can be detected using a mouse mAb against li, e.g. Pin1.1, or rabbit polyclonal antibodies specific for the C-terminus of the p10li fragment or against the entire p10li fragment. For ELISA assay, a pair of antibodies against li, including Pin1.1, and a rabbit polyclonal antibody or a mouse monoclonal antibody specific for p10li, can be used. The same assay can also be applied to monitor the effect of cathepsin S inhibitors *in vivo* in animal studies, for example in monkeys, dogs, pigs, rabbits, guinea pigs, and rodents.

In the present example PBMC from human blood were incubated with the cathepsin S inhibitor, LHVS (morpholinurea-leucine-homo-phenylalanine-vinylsulfonephenyl, also referred to as 4-morpholinecarboxamide, N-[(1S)-3-methyl-1-[[[(1S,2E)-1-(2-phenylethyl)-3-(phenylsulfonyl)-2-propenyl]amino]carbonyl]butyl]-. This compound has been described in US Patent No. 5,976,858 and in Palmer et al. (1995) J. Med. Chem. 38:3193 and Riese et al. (1996) Immunity 4:357. After incubation for 24 h the samples were run using standard SDS-PAGE protocols, transferred to nitrocellulose membranes and probed with an antibody that recognizes the invariant chain including the p10li fragment. In the presence of LHVS the p10li fragment was seen, representing a block in the degradation of li due to inhibition of cathepsin S.

### Example 49

### Monitoring in vivo inhibition of allergenic response by cathepsin S inhibitors.

To demonstrate the efficacy of cathepsin S inhibitors for suppressing allergic responses *in vivo,* allergic volunteers are dosed with cathepsin S inhibitors to levels where invariant chain degradation is inhibited. Allergens are deposited subcutaneously, and the size of the cutaneous reactions are determined at 15 min, 6 h and 24 h. Skin biopsies are performed at 24 h. The immediate weal and flare response is not mediated by a T cell response and is not expected to be influenced by cathepsin S inhibitors, while the late phase induration (noticeable at 6 hours, more pronounced at 24 hours) is characterized by activation and infiltration of CD4 T cells (as well as of eosinophils) and should be inhibited by administration of inhibitors of cathepsin S. The skin biopsies are used to determine the cellular composition in the induration, and cathepsin S treated subjects are expected to have fewer activated CD4 T cells present than placebo-treated subjects.

References for these procedures are provided in Eberlein-Konig et al. (1999) Clin. Exp. Allergy 29:1641-1647 and in Gaga et al. (1991) J. Immunol. 147:816-822.

As controls for the experiment, prednisone and cyclosporine A will be used. Prednisone will inhibit both the immediate and the late phase responses, while cyclosporin A will inhibit only the late phase response.

## Claims

1. The use of a compound in the manufacture of a pharmaceutical composition for treating a subject with an allergic condition, said compound being of formula (I) below: 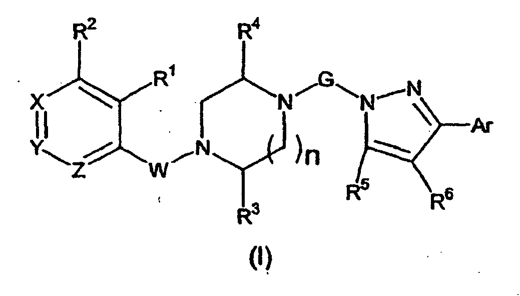 wherein:
R¹ is hydrogen, azido, halogen, C₁₋₅ alkoxy, hydroxy, C₁₋₅ alkyl, C₂₋₆ alkenyl, cyano, nitro, R⁷R⁸N, C₂₋₈ acyl, R⁹OC=O, R¹⁰R¹¹NC=O, or R¹⁰R¹¹NSO₂; or R¹ is taken together with W as described below;
R² is hydrogen, halogen, C₁₋₅ alkoxy, C₁₋₅ alkyl, C₂₋₅ alkenyl, C₁₋₅ haloalkyl, cyano, or R⁴⁶R⁴⁹N; alternatively, R¹ and R² can be taken together to form an optionally substituted 5- to 7- membered carbocyclic or heterocyclic ring, which ring may be unsaturated or aromatic;
each of R³ and R⁴ is independently hydrogen or C₁₋₅ alkyl;
each of R⁵ and R⁶ is independently hydrogen, C₁₋₅ alkyl, C₂₋₅ alkenyl, C₁₋₅ alkoxy, C₁₋₅ alkylthio, halogen, or a 4-7 membered carbocyclyl or heterocyclyl;
alternatively, R⁵ and R⁶ can be taken together to form an optionally substituted 5- to 7- membered carbocyclic or heterocyclic ring, which ring may be unsaturated or aromatic, and may be optionally substituted with between one and three substituents independently selected from halo, cyano, amino, nitro, R⁴⁰, R⁴⁰O-, R⁴⁰S-, R⁴⁰O(C₁₋₅alkylene)-, R⁴⁰O(C=O)-, R⁴⁰(C=O)-, R⁴⁰(C=S)-, R⁴⁰(C=O)O-, R⁴⁰O(C=O)(C=O)-, R⁴⁰SO₂, NHR⁶²(C=NH)-, NHR⁶²SO₂-, and NHR⁶²(C=O)-;
R⁴⁰ is H, C₁₋₅ alkyl, C₂₋₆ alkenyl, phenyl, benzyl, phenethyl, C₁₋₅ heterocyclyl, (C ₁₋₅ heterocyclyl)C₁₋₅ alkylene, amino, or mono- or di(C₁₋₅ alkyl)amino, or R⁵⁸OR⁵⁹-, wherein R⁵⁸ is H, C₁₋₅ alkyl, C₂₋₅ alkenyl, phenyl, benzyl, phenethyl, C₁₋₅ heterocyclyl, or (C₁₋₅ heterocyclyl)C₁₋₆alkylene and R⁵⁹ is C₁₋₅ alkylene, phenylene, or divalent C₁₋₅ heterocyclyl; and
R⁶² can be H in addition to the values for R⁴⁰;
R⁷ is hydrogen, C₁₋₅ alkyl. C₃₋₅ alkenyl, phenyl, naphthyl, C₁₋₅ heterocyclyl, C₂₋₈ acyl, aroyl, R²⁷OC=O, R²⁸R²⁹NC=O, R²⁷SO, R²⁷SO₂, or R²⁸R²⁹NSO₂;
R⁶ is hydrogen, C₁₋₅ alkyl, C₃₋₅ alkenyl, phenyl, or C₁₋₅ heterocyclyl; alternatively, R⁷ and R⁸ can be taken together to form an optionally substituted 4- to 7- membered heterocyclic ring, which ring may be saturated, unsaturated or aromatic;
R⁹ is C₁₋₅ alkyl, phenyl, naphthyl, or C₁₋₅ heterocyclyl;
R²¹ is hydrogen, C₁₋₅ alkyl, C₃₋₅ alkenyl, phenyl, naphthyl, C₁₋₅ heterocydyl, C₂₋₈ acyl, aroyl, R³⁰OC=O, R³¹R³²NC=O, R³⁰SO, R³⁰SO₂; or R³¹R³²NSO₂;
R²² is hydrogen, C₁₋₅ alkyl, C₃₋₅ alkenyl, phenyl, or C₁₋₅ heterocyclyl; alternatively, R²¹ and R²²can be taken together to form an optionally substituted 4- to 7-membered heterocyclic ring, which ring may be saturated, unsaturated or aromatic;
each of R²³, R²⁶, R²⁷, R³⁰, R³³, R⁴⁴, R⁴⁵, and R⁵⁰ is C₁₋₅ alkyl, phenyl, naphthyl, or C₁₋₅ heterocyclyl;
R²⁴ is hydrogen, C₁₋₅ alkyl, C₃₋₅ alkenyl, phenyl, naphthyl, C ₁₋₅ heterocyclyl, C₂₋₈ acyl, aroyl, R³³OC=O, R³⁴R³⁵NC=O, R³³SO, R³³SO₂, or R³⁴R³⁵NSO₂;
R²⁵ is hydrogen, C₁₋₅ alkyl, C₃₋₅ alkenyl, phenyl, or C₁₋₅ heterocyclyl; alternatively, R²⁴ and R²⁵ can be taken together to form an optionally substituted 4- to 7- membered heterocyclic ring, which ring may be saturated, unsaturated or aromatic;
each of R¹⁰ and R¹¹ is independently hydrogen, C₁₋₅ alkyl, C₂₋₅ alkenyl, phenyl, or C ₁₋₅ heterocyclyl; alternatively, R¹⁰ and R¹¹ or can be taken together to form an optionally substituted 4- to 7- membered heterocyclic ring, which ring may be saturated, unsaturated or aromatic;
each of R²⁸, R²⁹, R³¹, R³², R³⁴, R³⁵, R⁴⁶, R⁴⁷, R⁵¹ and R⁵² is independently hydrogen, C₁₋₅ alkyl, phenyl, or C ₁₋₅ heterocyclyl; alternatively, R²⁸ and R²⁹, R³¹ and R³², R³⁴ and R³⁵, R⁴⁶ and R⁴⁷, or R⁵¹ and R⁵², independently, can be taken together to form an optionally substituted 4- to 7- membered heterocyclic ring, which ring may be saturated, unsaturated or aromatic;
n is 1 or 2;
G represents C₃₋₆ alkenediyl or C₃₋₆ alkanediyl, optionally substituted with hydroxy, halogen, C₁₋₅alkyl, C₁₋₅ alkoxy, oxo, hydroximino, CO₂R⁶⁰, R⁶⁰R⁶¹NCO₂, (L)-C₁₋₄ alkylene-, (L)-C₁₋₅ alkoxy, N₃, or [(L)-C ₁₋₅ alkylene]amino;
each of R⁶⁰ and R⁶¹ is independently hydrogen, C₁₋₅alkyl, C₃₋₅ alkenyl, phenyl, benzyl, phenethyl, or C₁₋₅ heterocyclyl; alternatively R⁶⁰ and R⁶¹, can be taken together to form an optionally substituted 4- to 7- membered heterocyclic ring, which ring may be saturated, unsaturated or aromatic;
L is amino, mono- or di-C₁₋₅alkylamino, pyrrolidinyl, morpholinyl, piperidinyl homopiperidinyl, or piperazinyl, where available ring nitrogens may be optionally substituted with C₁₋₅ alkyl, benzyl, C₂₋₅ acyl, C₁₋₅ alkylsulfonyl or C₁₋₅ alkyloxycarbonyl;
X is nitrogen or R¹²C;
Y is nitrogen or R¹³C;
Z is nitrogen or R¹⁴C;
R¹² is hydrogen, halogen, C₁₋₅ alkoxy, C₁₋₆alkyl, C₂₋₅ alkenyl, cyano, nitro, R²¹R²²N, C₂₋₈ acyl, C₁₋₅ haloalkyl, C₁₋₅ heterocyclyl, (C₁₋₅ heterocyclyl)C ₁₋₅ alkylene, R²³OC=O, R²³O(C=O)NH-, R²³SO, R²²NHCO-, R²²NH(C=O)NH-, R²³(C₁₋₄ alkylene)NHCO-, R²³SO₂, or R²³SO₂NH-;
R¹³ is hydrogen, halogen, C₁₋₅ alkoxy, C₁₋₅ alkyl, C₂₋₅ alkenyl, cyano, nitro, R⁴²R⁴³N, C ₂₋₈ acyl, C₁₋₅ haloalkyl, C ₁₋₅ heterocyclyl, (C ₁₋₅ heterocyclyl)C ₁₋₅ alkylene, R⁴⁴OC=O, R⁴⁴O(C=O)NH-, R⁴⁴SO, R⁴³NHCO-, R⁴³NH(C=O)NH-, R⁴⁴(C₁₋₄ alkylene)NHCO-, R⁴⁴SO₂, or R⁴⁴SO₂NH-;
R¹⁴ is hydrogen, halogen, C₁₋₅ alkoxy, C₁₋₅ alkyl, C₂₋₅ alkenyl, cyano, nitro, R²⁴R²⁵N, C ₂₋₈ acyl, C₁₋₅ haloalkyl, C₁₋₅ heterocyclyl, (C₁₋₅ heterocyclyl)C_{1- 5} alkylene, R²⁶OC=O, R²⁶O(C=O)NH-, R²⁶SO, R²⁵NHCO-, R²⁵NH(C=O)NH-, R²⁶(C₁₋₄ alkylene)NHCO-, R²⁶SO₂, or R²⁶SO₂NH-; alternatively, R¹² and R¹³ or R¹² and R² or R¹³ and R¹⁴ can be taken together to form an optionally substituted 5- to 6- membered carbocyclic or heterocyclic ring, which ring may be unsaturated or aromatic;
Ar represents a monocyclic or bicyclic aryl or heteroaryl ring, optionally substituted with between 1 and 3 substituents selected from halogen, C₁₋₅ alkoxy, C₁₋₅ alkyl, C₂₋₅ alkenyl, cyano, azido, nitro, R¹⁵R¹⁶N, R¹⁷SO₂, R¹⁷S, R¹⁷SO, R¹⁷OC=O, R¹⁵R¹⁶NC=O, C₁₋₅ haloalkyl, C₁₋₅ haloalkoxy, C₁₋₅ haloalkylthio, and C₁₋₅alkylthio;
R¹⁵ is hydrogen, C₁₋₅alkyl, C₃₋₅ alkenyl, phenyl, benzyl, C₁₋₅ heterocyclyl, C ₂₋₈ acyl, aroyl, R⁵³OC=O, R⁵⁴R⁵⁵NC=O, R⁵³S, R⁵³SO, R⁵³SO₂, or R⁵⁴R⁵⁵NSO₂;
R¹⁶ is hydrogen, C₁₋₅ alkyl, C₃₋₅ alkenyl, phenyl, benzyl, or C₁₋₅ heterocyclyl; alternatively, R¹⁵ and R¹⁶ can be taken together to form an optionally substituted 4- to 7- membered heterocyclic ring, which ring may be saturated, unsaturated or aromatic;
each of R¹⁷ and R⁵³ is C₁₋₅alkyl, phenyl, or C₁₋₅ heterocyclyl;
each of R⁵⁴ and R⁵⁵ is independently hydrogen, C₁₋₅ alkyl, C₂₋₅ alkenyl, phenyl, benzyl, or C₁₋₅ heterocyclyl; alternatively, R⁵⁴ and R⁵⁵ can be taken together to form an optionally substituted 4- to 7- membered heterocyclic ring, which ring may be saturated, unsaturated or aromatic;
W represents SO₂, C=O, CHR²⁰, or a covalent bond; or W and R¹, taken together with the 6-membered ring to which they are both attached, form one of the following two formulae: 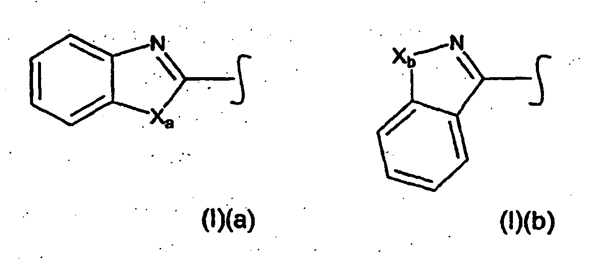 wherein Xₐ is O, S, or N; and X_{b} is O, S or SO₂;
R²⁰ is hydrogen, C₁₋₅ alkyl, phenyl, benzyl, naphthyl, or C₁₋₆ heterocyclyl;
R⁴² is hydrogen, C₁₋₅ alkyl, C₃₋₅ alkenyl, phenyl, naphthyl, C₁₋₅ heterocyclyl, C₂₋₈ acyl, aroyl, R⁴⁵OC=O, R⁴⁸R⁴⁷NC=O, R⁴⁶SO, R⁴⁵SO₂, or R⁴⁸R⁴⁷NSO₂;
R⁴³ is hydrogen, C₁₋₅ alkyl, C₃₋₅ alkenyl, phenyl, or C ₁₋₅ heterocyclyl; alternatively, R⁴² and R⁴³can be taken together to form an optionally substituted 4- to 7- membered heterocyclic ring, which ring may be saturated, unsaturated or aromatic;
R⁴⁴ is C₁₋₅ alkyl, C₂₋₅ alkenyl, phenyl, naphthyl, or C₁₋₆ heterocyclyl;
R⁴⁸ is hydrogen, C₁₋₅ alkyl, C₃₋₅ alkenyl, phenyl, naphthyl, C ₁₋₅ heterocyclyl, C ₂₋₈ acyl, aroyl, R⁵⁰OC=O, R⁵¹R⁵²NC=O, R⁵⁰SO, R⁵⁰SO₂, or R⁵¹R⁵²NSO₂;
R⁴⁹ is hydrogen, C₁₋₅ alkyl, C₃₋₆ alkenyl, phenyl, or C ₁₋₅ heterocyclyl; alternatively, R⁴⁸ and R⁴⁹ can be taken together to form an optionally substituted 4- to 7- membered heterocyclic ring, which ring may be saturated, unsaturated or aromatic; and
wherein each of the above hydrocarbyl or heterocarbyl groups, unless otherwise indicated, and in addition to any specified substituents, is optionally and independently substituted with between 1 and 3 substituents selected from methyl, halomethyl, hydroxymethyl, halo, hydroxy, amino, nitro, cyano, C₁₋₅ alkyl, C₁₋₅ alkoxy, -COOH, C₂₋₆, acyl, [di(C ₁₋₄ alkyl)amino]C₂₋₅ alkylene, [di(C ₁₋₄ alkyl)amino] C₂₋₅ alkyl-NH-CO-, and C₁₋₅ haloalkoxy;
or a pharmaceutically acceptable salt, ester, or amide thereof.

2. A use of claim 1, wherein each of R³ and R⁴ is hydrogen; Ar represents a six membered ring, optionally substituted with between 1 and 2 substituents selected from halogen, C₁₋₆ alkyl, cyano, nitro, R¹⁵R¹⁶N, CF₃ and OCF₃; R¹² is hydrogen, R²³SO, or R²³SO₂; R¹³ is hydrogen, R⁴⁴SO, or R⁴⁴SO₂; R¹⁴ is hydrogen, halogen, C₁₋₅ alkoxy, C₁₋₅ alkyl, cyano, nitro, or R²⁴R²⁵N; and G is C₃ alkanediyl, optionally substituted with hydroxy, (L)-C₁₋₅alkyloxy-, or (L)-C₁₋₅ alkylamino.

3. A use of claim 2, wherein Ar is phenyl.

4. A use of claim 1, wherein said compound is selected from:
1-[4-(2-Amino-6-chloro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol ;
1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-3-methyl-urea ;
1-[3-Chloro-2-(4-{2-hydroxy-3-[5-methanesulfonyl-3-(4-trifluoromethylphenyl)-4,5,6,7-tetrahydro-pyrazolo[4,3-c]pyridin-1-yl]-propyl}-piperazin-1-yl)-phenyl]-3-methyl-urea ;
3-Amino-2-(4-{2-hydroxy-3-[5-methanesulfonyl-3-(4-trifluoromethylphenyl)-4,5,6,7-tetrahydro-pyrazolo[4,3-c]pyridin-1-yl]-propyl}-piperazin-1-yl)-benzoic acid methyl ester ;
3-Chloro-2-(4-{3-[5-methanesulfonyl-3-(4-trifluoromethyl-phenyl)-4,5,6,7-tetrahydro-pyrazolo[4,3-c]pyrydin-1-yl]-propyl}-piperazin-1-yl)-phenylamine ;
1-[2-(4-{3-[3-(4-Bromo-phenyl)-5-methanesulfonyl-4,5,6,7-tetrahydro-pyrazolo[4,3-c]pyridin-1-yl]-2-hydroxy-propyl}-piperazin-1-yl)-3-chloro-phenyl]-3-methyl-urea ;
and 1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-carboxylic acid amide .

5. A use of claim 1, wherein said compound is selected from:
[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-carbamic acid methyl ester ;
1-[3-(4-Benzo[d]isothiazol-3-yl-piperazin-1-yl)-propyl]-3-(4-bromophenyl)-1,4,6,7-tetrahydro-pyrazolo[4,3-c]pyridine-5-carboxylic acid amide ;
2-(4-{3-[5-Acetyl-3-(4-trifluoromethyl-phenyl)-4,5,6,7-tetrahydro-pyrazolo[4,3-c]pyridin-1-yl]-2-hydroxy-propyl}-piperazin-1 yl)-3-nitro-benzoic acid methyl ester;
1-[4-(2-Chloro-6-nitno-phenyl)-piperazin-1-yl]-3-[5-methanesulfonyl-3-(4-trifluoromethyl-phenyl)-4,5,6,7-tetrahydro-pyrazolo[4,3-c]pyridin-9 -yl]-propan-2-ol;
2-(4-{2-Hydroxy-3-[3-(4-iodo-phenyl)-5-methanesulfonyl-4,5,6,7-tetrahydro-pyrazolo[4,3-c]pyridin-1-yl]-propyl}-piperazin-1-yl)-benzonitrile;
3-(4-Bromo-phenyl)-1-{3-[4-(2-nitro-phenyl)-piperazin-1-yl]-propyl}-1,4,6,7-tetrahydro-pyrazolo[4,3-c]pyridine-5-carboxylic acid amide ;
2-(4-{3-[5-Acetyl-3-(4-iodo-phenyl)-4,5,6,7-tetrahydro-pyrazolo[4,3-c]pyridin-1-yl]-2-hydroxy-propyl}-piperazin-1-yl)-benzonitrile ;
2-(4-{3-[3-(4-Chloro-3-methyl-phenyl)-5-methanesulfonyl-4,5,6,7-tetrahydro-pyrazolo[4,3-c]pyridin-1-yl]-2-hydroxy-propyl}-piperazin-1-yl)-benzonitrile;
1-(3-(4-Chloro-3-methyl-phenyl)-1-{3-[4-(2,4-dimethyl-phenyl)-piperazin-1-yl]-2-hydroxy-propyl}-1,4,6,7-tetrahydro-pyrazolo[4,3-c]pyridin-5-yl)-ethanone;
1-{3-[4-(3,5-Dichloro-pyridin-4-yl)-piperazin-1-yl]-propyl}-5-methanesulfonyl-3-(4-trifluoromethyl-phenyl)-4,5,6,7-tetrahydro-1H-pyrazolo[4,3-c]Pyridine ;
2-(4-{3-[5-Methanesulfonyl-3-(4-trifluoromethyl-phenyl)-4,5,6,7-tetrahydro-pyrazolo[4,3-c]pyridin-1-yl]-propyl}-piperazin-1-yl)-benzonitrile;
N-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide ;
3-(3,4-Dichloro-phenyl)-1-{3-[4-(2-nitro-phenyl)-piperazin-1-yl]-propyl}-1,4,6,7-tetrahydro-pyrazolo[4,3-c]pyridine-5-carboxylic acid amide;
and 3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridine-5-carboxylic acid amide.

6. A use of claim 1, wherein said compound is selected from:
1-(3-(4-Chloro-phenyl)-1-{3-[4-(2-fluoro-phenyl)-piperazin-1-yl]-propyl}-1,4,6,7-tetrahydro-pyrazolo[4,3-c]pyridin-5-yl)-ethanone;
1-{3-(4-Chloro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone;
1-{3-(4-Chloro-phenyl)-1-[2-methoxy-3-(4-o-tolyl-piperazin-1-yl)-propyl]-1,4,6,7-tetrahydro-pyrazolo[4,3-c]pyridin-5-yl}-ethanone;
1-[1-{2-Hydroxy-3-[4-(2-hydroxy-phenyl)-piperazin-1-yl]-propyl}-3-(4-iodophenyl)-1,4,6,7-tetrahydro-pyrazolo[4,3-c]pyridin-5-yl]-ethanone;
1-[1-[2-Hydroxy-3-(4-o-tolyl-piperazin-1-yl)-propyl]-3-(4-trifluoromethyl-phenyl)-1,4,6,7-tetrahydro-pyrazolo[4,3-c]pyridin-5-yl]-ethanone;
2-(4-{3-[5-Acetyl-3-(4-trifluoromethyl-phenyl)-4,5,6,7-tetrahydro-pyrazolo[4,3-c]pyridin-1-yl]-2-hydroxy-propyl}-piperazin-1-yl)-benzonitrile;
1-[3-(3,4-Dichloro-phenyl)-pyrazol-1 yl]-3-(4-o-tolyl-piperazin-1-yl)-propan-2-ol;
1-[1-[2-(2-Piperazin-1-yl-ethylamino)-3-(4-o-tolyl-piperazin-1-yl)-propyl]-3-(4-trifluoromethyl-phenyl)-1,4,6,7-tetrahydro-pyrazolo[4,3-c]pyridin-5-yl]-ethanone;
1-{3-[4-(2-Cyano-phenyl)-piperazin-1-yl]-2-hydroxy-propyl}-3-(4-iodo-phenyl)-1,4,6,7-tetrahydro-pyrazolo[4,3-c]pyridine-5-carboxylic acid tert-butyl ester;
1-{3-[4-(2-Cyano-phenyl)-piperazin-1-yl]-2-hydroxy-propyl}-3-(4-iodo-phenyl)-1,4,6,7-tetrahydro-pyrazolo[4,3-c]pyridine-5-carboxylic acid amide;
Carbamic acid 1-[5-carbamoyl-3-(4-iodo-phenyl)-4,5,6,7-tetrahydro-pyrazolo[4,3-c]pyridin-1-ylmethyl]-2-[4-(2-cyano-phenyl)-piperazin-1-yl]-ethyl ester;
1-{3-(3-Amino-4-chloro-phenyl)-1-[2-hydroxy-3-(4-o-tolyl-piperazin-1-yl)-propyl]-1,4,6,7-tetrahydro-pyrazolo[4,3-c]pyridin-5-yl}-ethanone;
(*R*)-1-(3-(4-Bromo-phenyl)-1-{3-[4-(5-chloro-2-methyl-phenyl)-piperazin-1-yl]-2-hydroxy-propyl}-1,4,6,7-tetrahydro-pyrazolo[4,3-c]pyridin-5-yl)-ethanone;
2-(4-{3-[5-Acetyl-3-(4-trifluoromethyl-phenyl)-4,5,6,7-tetrahydro-pyrazolo[4,3-c]pyridin-1-yl]-2-fluoro-propyl}-piperazin-1-yl)-benzonitrile;
(3-(4-Chloro-3-methyl-phenyl)-1-{3-[4-(2-cyano-phenyl)-piperazin-1-yl]-2-hydroxy-propyl}-1,4,6,7-tetrahydro-pyrazolo[4,3-c]pyridin-5-yl)-oxo-acetic acid methyl ester,
5-Methanesulfonyl-1-{3-[4-(2-nitro-phenyl)-piperazin-1-yl]-propyl}-3-(4-trifluoromethyl-phenyl)-4,5,6,7-tetrahydro-1H-pyrazolo[4,3-c]pyridine;
1-[3-Chloro-2-(4-{3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-urea;
1-{3-[4-(2-Chloro-6-methanesulfonylamino-phenyl)-piperazin-1-yl]-propyl}-3-(4-trifluoromethyl-phenyl)-1,4,6,7-tetrahydro-pyrazolo[4,3-c]pyridine-5-sulfonic acid amide;
N-[3-Chloro-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide;
1-[4-(2,6-Dinitro-phenyl)-piperazin-1-yl]-3-[5-methanesulfonyl-3-(4-trifluoromethyl-phenyl)-4,5,6,7-tetrahydro-pyrazolo[4,3-c]pyridin-1-yl]-propan-2-ol;
2-(4-{2-Hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-3-methanesulfonylamino-benzoic acid methyl ester;
1-{3-[4-(1,1-Dioxo-1H-116-benzo[d]isothiazol-3-yl)-piperazin-1-yl]-propyl}-5-methanesulfonyl-3-(4-trifluoromethyl-phenyl)-4,5,6,7-tetrahydro-1H-pyrazolo[4,3-c]pyridine;
1-[1-{3-[4-(6-Chloro-benzothiazol-2-yl)-piperazin-1-yl]-2-hydroxy-propyl}-3-(4-trifluoromethyl-phenyl)-1,4,6,7-tetrahydro-pyrazolo[4;3-c]pyridin-5-yl]-ethanone; and
1-[1-[3-(4-Benzo[d]isoxazol-3-yl-piperazin-1-yl)-2-hydroxy-propyl]3-(4-trifluoromethyl-phenyl)-1,4,6,7-tetrahydro-pyrazolo[4,3-c]pyridin-5-yl]-ethanone.

7. A use of claim 1, wherein said compound is selected from: N-[3-Chloro-2-(4-{2-hydroxy-3-[5-methanesulfonyl-3-(4-trifluoromethyl-phenyl)-4,5,6,7-tetrahydro-pyrazolo[4,3-c]pyridin-1-yl]-propyl}-piperazin-1-yl)-phenyl]-methanesulfonamide;
1-[3-(4-Benzo[d]isothiazol-3-yl-piperazin-1-yl)-propyl]-3-(4-bromophenyl)-1,4,6,7-tetrahydro-pyrazolo[4,3-c]pyridine-5-carboxylic acid amide; and
1-[3-Chloro-2-(4-{2-hydroxy-3-[5-methanesulfonyl-3-(4-trifluoromethylphenyl)-4,5,6,7 tetrahydro-pyrazoio[4,3-c]pyridin-1-yl]-propyl}-piperazin-1-yl)-phenyl]-3-rnethyl-urea.

8. A use of claim 1, wherein said pharmaceutical composition is formulated in a dosage amount appropriate for the treatment of an allergic condition.

## Patentansprüche

1. Verwendung einer Verbindung zur Herstellung einer pharmazeutischen Zusammensetzung zur Behandlung einer Person mit einem allergischen Zustand, wobei besagte Verbindung Formel (I) unten besitzt: 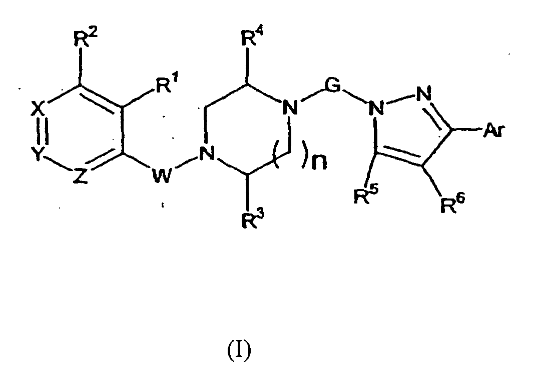 worin:
R¹ Wasserstoff, Azido, Halogen, C₁₋₅-Alkoxy, Hydroxy, C₁₋₅-Alkyl, C₂₋₅-Alkenyl, Cyano, Nitro, R⁷R⁸N, C₂₋₈-Acyl, R⁹OC=O, R¹⁰R¹¹NC=O oder R¹⁰R¹¹NSO₂ ist; oder R¹ mit W, wie unten beschrieben, zusammengenommen ist;
R² Wasserstoff, Halogen, C₁₋₅-Alkoxy, C₁₋₅-Alkyl, C₂₋₅-Alkenyl, C₁₋₅-Haloalkyl, Cyano oder R⁴⁸R⁴⁹ N ist;
alternativ R¹ und R² zusammengenommen sein können, um einen fakultativ substituierten 5- bis 7-gliedrigen carbocyclischen oder heterocyclischen Ring zu bilden, wobei dieser Ring ungesättigt oder aromatisch sein kann;
R³ und R⁴ jeweils unabhängig Wasserstoff oder C₁₋₅-Alkyl ist;
R⁵ und R⁶ jeweils unabhängig Wasserstoff, C₁₋₅-Alkyl, C₂₋₅-Alkenyl, C₁₋₅-Alkoxy, C₁₋₅-Alkylthio, Halogen oder ein 4- bis 7-gliedriges Carbocyclyl oder Heterocyclyl ist;
alternativ R⁵ und R⁶ zusammengenommen sein können, um einen fakultativ substituierten 5- bis 7-gliedrigen carbocyclischen oder heterocyclischen Ring zu bilden, wobei dieser Ring ungesättigt oder aromatisch sein kann und fakultativ mit zwischen einem und drei Substituenten substituiert sein kann, die unabhängig ausgewählt sind aus Halo, Cyano, Amino, Nitro, R⁴⁰, R⁴⁰O-, R⁴⁰S-, R⁴⁰O(C₁₋₅₋Alkylen)-, R⁴⁰O(C=O)-, R⁴⁰(C=O)-, R⁴⁰(C=S)-, R⁴⁰(C=O)O-, R⁴⁰O(C=O)(C=O)-, R⁴⁰SO₂, NHR⁶²(C=NH)-, NHR⁶²SO₂- und NHR⁶²(C=O)-;
R⁴⁰ H, C₁₋₅-Alkyl, C₂₋₅-Alkenyl, Phenyl, Benzyl, Phenethyl, C₁₋₅-Heterocyclyl, (C₁₋₅₋Heterocyclyl)-C₁₋₅-alkylen, Amino oder Mono- oder Di(C₁₋₅-alkyl)amino oder R⁵⁸OR⁵⁹ - ist, wobei R⁵⁸ H, C₁₋₅-Alkyl, C₂₋₅-Alkenyl, Phenyl, Benzyl, Phenethyl, C₁₋₅₋Heterocyclyl oder (C₁₋₅-Heterocyclyl)-C₁₋₆-alkylen ist und R⁵⁹ C₁₋₅-Alkylen, Phenylen oder zweiwertiges C₁₋₅-Heterocyclyl ist; und
R⁶² zusätzlich zu den Werten für R⁴⁰ H sein kann;
R⁷ Wasserstoff, C₁₋₅-Alkyl, C₃₋₅-Alkenyl, Phenyl, Naphthyl, C₁₋₅-Heterocyclyl, C₂₋₈₋Acyl, Aroyl, R²⁷OC=O, R²⁸R²⁹NC=O, R²⁷SO, R²⁷SO₂ oder R²⁸R²⁹NSO₂ ist;
R⁸ Wasserstoff, C₁₋₅-Alkyl, C₃-₅-Alkenyl, Phenyl oder C₁₋₅-Heterocyclyl ist;
alternativ R⁷ und R⁸ zusammengenommen sein können, um einen fakultativ substituierten 4- bis 7-gliedrigen heterocyclischen Ring zu bilden, wobei dieser Ring gesättigt, ungesättigt oder aromatisch sein kann;
R⁹ C₁₋₅-Alkyl, Phenyl, Naphthyl oder C₁₋₅-Heterocyclyl ist;
R²¹ Wasserstoff, C₁₋₅-Alkyl, C₃₋₅-Alkenyl, Phenyl, Naphthyl, C₁₋₅-Heterocyclyl, C₂₋₈₋Acyl, Aroyl, R³⁰OC=O, R³¹R³²NC=O, R³⁰SO, R³⁰SO₂ oder R³¹R³²NSO₂ ist;
R²² Wasserstoff, C₁₋₅-Alkyl, C₃₋₅-Alkenyl, Phenyl oder C₁₋₅-Heterocyclyl ist;
alternativ R²¹ und R²² zusammengenommen sein können, um einen fakultativ substituierten 4- bis 7-gliedrigen heterocyclischen Ring zu bilden, wobei dieser Ring gesättigt, ungesättigt oder aromatisch sein kann;
R²³, R²⁶, R²⁷, R³⁰, R³³, R⁴⁴, R⁴⁵ und R⁵⁰ jeweils C₁₋₅-Alkyl, Phenyl, Naphthyl oder C₁₋₅₋Heterocyclyl ist;
R₂₄ Wasserstoff, C₁₋₅-Alkyl, C₃₋₅-Alkenyl, Phenyl, Naphthyl, C₁₋₅-Heterocyclyl, C₂₋₈₋Acyl, Aroyl, R³³OC=O, R³⁴R³⁵NC=O, R³³SO, R³³SO₂ oder R³⁴R³⁵NSO₂ ist;
R²⁵ Wasserstoff, C₁₋₅-Alkyl, C₃₋₅-Alkenyl, Phenyl oder C₁₋₅-Heterocyclyl ist;
alternativ R²⁴ und R²⁵ zusammengenommen sein können, um einen fakultativ substituierten 4- bis 7-gliedrigen heterocyclischen Ring zu bilden, wobei dieser Ring gesättigt, ungesättigt oder aromatisch sein kann;
R¹⁰ und R¹¹ jeweils unabhängig Wasserstoff, C₁₋₅-Alkyl, C₂₋₅-Alkenyl, Phenyl oder C₁₋₅-Heterocyclyl ist;
alternativ R¹⁰ und R¹¹ zusammengenommen sein können, um einen fakultativ substituierten 4- bis 7-gliedrigen heterocyclischen Ring zu bilden, wobei dieser Ring gesättigt, ungesättigt oder aromatisch sein kann;
R²⁸ R²⁹ R³¹ R³², R³⁴ R³⁵, R⁴⁶, R⁴⁷ R⁵¹ und R⁵² jeweils unabhängig Wasserstoff, C₁₋₅₋Alkyl, Phenyl oder C₁₋₅-Heterocyclyl ist; jeweils unabhängig Wasserstoff, C₁₋₅₋Alkyl, Phenyl oder C₁₋₅-Heterocyclyl ist;
alternativ R²⁸ und R²⁹, R³¹ und R³², R³⁴ und R³⁵, R⁴⁶ und R⁴⁷ oder R⁵¹ und R¹² unabhängig zusammengenommen sein können, um einen fakultativ substituierten 4-bis 7-gliedrigen heterocyclischen Ring zu bilden, wobei dieser Ring gesättigt, ungesättigt oder aromatisch sein kann;
n 1 oder 2 ist;
G für C₃₋₆-Alkendiyl oder C₃₋₆-Alkandiyl steht, fakultativ substituiert mit Hydroxy, Halogen, C₁₋₅-Alkyl, C₁₋₅-Alkoxy, Oxo, Hydroximino, CO₂R⁶⁰, R⁶⁰R⁶¹NCO₂, (L)-C₁₋₄₋Alkylen-, (L)-C₁₋₅-Alkoxy, N₃ oder [(L)-C₁₋₅-Alkylen]amino;
R⁶⁰ und R⁶¹ jeweils unabhängig Wasserstoff, C₁₋₅-Alkyl, C₃₋₅-Alkenyl, Phenyl, Benzyl, Phenethyl oder C₁₋₅-Heterocyclyl ist;
alternativ R⁶⁰ und R⁶¹ zusammengenommen sein können, um einen fakultativ substituierten 4- bis 7-gliedrigen heterocyclischen Ring zu bilden, wobei dieser Ring gesättigt, ungesättigt oder aromatisch sein kann;
L Amino, Mono- oder Di-C₁₋₅-alkylamino, Pyrrolidinyl, Morpholinyl, Piperidinyl, Homopiperidinyl oder Piperazinyl ist, wobei verfügbare Ringstickstoffe fakultativ substituiert sein können mit C₁₋₅-Alkyl, Benzyl, C₂₋₅-Acyl, C₁₋₅-Alkylsulfonyl oder C₁₋₅-Alkyloxycarbonyl;
X Stickstoff oder R¹²C ist;
Y Stickstoff oder R¹³C ist;
Z Stickstoff oder R¹⁴C ist;
R¹² Wasserstoff, Halogen, C₁₋₅-Alkoxy, C₁₋₅-Alkyl, C₂₋₅-Alkenyl, Cyano, Nitro, R²¹R²²N, C₂₋₈-Acyl, C₁₋₅-Haloalkyl, C₁₋₅-Heterocyclyl, (C₁₋₅-Heterocyclyl)-C₁₋₅₋alkylen, R²³OC=O, R²³O(C=O)NH-, R²³SO, R²²NHCO-, R²²NH(C=O)NH-, R²³(C₁₋₄₋Alkylen)NHCO-, R²³SO₂ oder R²³SO₂NH- ist;
R¹³ Wasserstoff, Halogen, C₁₋₅-Alkoxy, C₁₋₅-Alkyl, C₂₋₅-Alkenyl, Cyano, Nitro, R⁴²R⁴³N C₂₋₈-Acyl, C₁₋₅-Haloalkyl, C₁₋₅-Heterocyclyl, (C₁₋₅-Heterocyclyl)-C₁₋₅₋alkylen, R⁴⁴OC=O, R⁴⁴O(C=O)NH-, R⁴⁴SO, R⁴³NHCO-, R⁴³NH(C=O)NH-, R⁴⁴(C₁₋₄₋Alkylen)NHCO-, R⁴⁴SO₂ oder R⁴⁴SO₂NH- ist;
R¹⁴ Wasserstoff, Halogen, C₁₋₅-Alkoxy, C₁₋₅-Alkyl, C₂₋₅-Alkenyl, Cyano, Nitro, R²⁴R²⁵N, C₂₋₈-Acyl, C₁₋₅-Haloalkyl, C₁₋₅-Heterocyclyl, (C₁₋₅-Heterocyclyl)-C₁₋₅₋alkylen, R²⁶OC=O, R²⁶O(C=O)NH-, R²⁶SO, R²⁵NHCO-, R²⁵NH(C=O)NH-, R²⁶(C₁₋₄₋Alkylen)NHCO-, R²⁶SO₂ oder R²⁶SO₂NH- ist;
alternativ R¹² und R¹³ oder R¹² und R² oder R¹³ und R¹⁴ zusammengenommen sein können, um einen fakultativ substituierten 5- bis 6-gliedrigen carbocyclischen oder heterocyclischen Ring zu bilden, wobei dieser Ring ungesättigt oder aromatisch sein kann;
Ar für einen monocyclischen oder bicyclischen Aryl- oder Heteroarylring steht, fakultativ substituiert mit zwischen 1 und 3 Substituenten, die ausgewählt sind aus Halogen, C₁₋₅-Alkoxy, C₁₋₅-Alkyl, C₂₋₅-Alkenyl, Cyano, Azido, Nitro, R¹⁵R¹⁶N, R¹⁷SO₂, R¹⁷S, R¹⁷SO, R¹⁷OC=O, R¹⁵R¹⁶NC=O, C₁₋₅-Haloalkyl, C₁₋₅-Haloalkoxy, C₁₋₅₋Haloalkylthio und C₁₋₅-Alkylthio;
R¹⁵ Wasserstoff, C₁₋₅-Alkyl, C₂₋₅-Alkenyl, Phenyl, Benzyl, C₁₋₅-Heterocyclyl, C₂₋₈₋Acyl, Aroyl, R⁵³OC=O, R⁵⁴R⁵⁵ NC=O, R⁵³ S, R⁵³SO, R⁵³SO₂ oder R⁵⁴R⁵⁵NSO₂ ist;
R¹⁶ Wasserstoff, C₁₋₅-Alkyl, C₃₋₅-Alkenyl, Phenyl, Benzyl oder C₁₋₅-Heterocyclyl ist;
alternativ R¹⁵ und R¹⁶ zusammengenommen sein können, um einen fakultativ substituierten 4- bis 7- gliedrigen heterocyclischen Ring zu bilden, wobei dieser Ring gesättigt, ungesättigt oder aromatisch sein kann;
R¹⁷ und R⁵³ jeweils C₁₋₅-Alkyl, Phenyl oder C₁₋₅-Heterocyclyl ist;
R⁵⁴ und R⁵⁵ jeweils unabhängig Wasserstoff, C₁₋₅-Alkyl, C₂₋₅-Alkenyl, Phenyl, Benzyl oder C₁₋₅-Heterocyclyl ist;
alternativ R⁵⁴ und R⁵⁵ zusammengenommen sein können, um einen fakultativ substituierten 4- bis 7-gliedrigen heterocyclischen Ring zu bilden, wobei dieser Ring gesättigt, ungesättigt oder aromatisch sein kann;
W für SO₂, C=O, CHR²⁰ oder eine kovalente Bindung steht; oder W und R¹, zusammengenommen mit dem 6-gliedrigen Ring, an den sie beide gebunden sind, eine der folgenden zwei Formeln bilden: 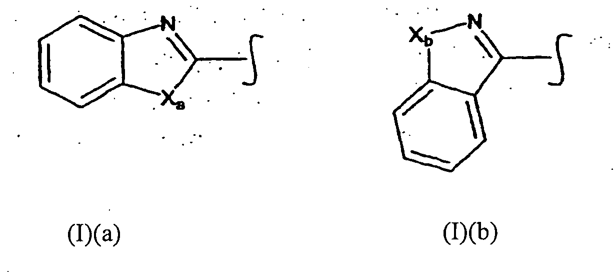
wobei Xₐ O, S oder N ist; und X_{b} O, S oder SO₂ ist;
R²⁰ Wasserstoff, C₁₋₅-Alkyl, Phenyl, Benzyl, Naphthyl oder C₁₋₅-Heterocyclyl ist;
R⁴² Wasserstoff, C₁₋₅-Alkyl, C₃₋₅-Alkenyl, Phenyl, Naphthyl, C₁₋₅-Heterocyclyl, C₂-₈₋Acyl, Aroyl, R⁴⁵OC=O, R⁴⁶R⁴⁷NC=O, R⁴⁵SO, R⁴⁵SO₂ oder R⁴⁶R⁴⁷NSO₂ ist;
R⁴³ Wasserstoff, C₁₋₅-Alkyl, C₃-₅-Alkenyl, Phenyl oder C₁₋₅-Heterocyclyl ist;
alternativ R⁴² und R⁴³ zusammengenommen sein können, um einen fakultativ substituierten 4- bis 7- gliedrigen heterocyclischen Ring zu bilden, wobei dieser Ring gesättigt, ungesättigt oder aromatisch sein kann;
R⁴⁴ C₁₋₅-Alkyl, C₂₋₅-Alkenyl, Phenyl, Naphthyl oder C₁₋₅-Heterocyclyl ist;
R⁴⁸ Wasserstoff, C₁₋₅-Alkyl, C₃₋₅-Alkenyl, Phenyl, Naphthyl, C₁₋₅-Heterocyclyl, C₂₋₈₋Acyl, Aroyl, R⁵⁰OC=O, R⁵¹R⁵²NC=O, R⁵⁰SO, R⁵⁰SO₂ oder R⁵¹R⁵²NSO₂ ist;
R⁴⁹ Wasserstoff, C₁₋₅-Alkyl, C₃₋₅-Alkenyl, Phenyl oder C₁₋₅-Heterocyclyl ist;
alternativ R⁴⁸ und R⁴⁹ zusammengenommen sein können, um einen fakultativ substituierten 4- bis 7-gliedrigen heterocyclischen Ring zu bilden, wobei dieser Ring gesättigt, ungesättigt oder aromatisch sein kann; und
wobei jede der obigen Hydrocarbyl- oder Heterocarbylgruppen, sofern nicht anders angegeben, und zusätzlich zu allen spezifizierten Substituenten, fakultativ und unabhängig mit zwischen 1 und 3 Substituenten substituiert sein kann, die ausgewählt sind aus Methyl, Halomethyl, Hydroxymethyl, Halo, Hydroxy, Amino, Nitro, Cyano, C₁₋₅-Alkyl, C₁₋₅-Alkoxy, -COOH, C₂₋₆-Acyl, [Di(C₁₋₄-alkyl)amino]-C₂₋₅-alkylen, [Di(C₁₋₄-alkyl)amino]-C₂₋₅-alkyl-NH-CO- und C₁₋₅-Haloalkoxy;
oder ein pharmazeutisch annehmbares Salz, Ester oder Amid derselben.

2. Verwendung nach Anspruch 1, **dadurch gekennzeichnet, daß** R³ und R⁴ jeweils Wasserstoff ist; Ar für einen sechsgliedrigen Ring steht, der fakultativ mit zwischen 1 und 2 Substituenten substituiert ist, die ausgewählt sind aus Halogen, C₁₋₅-Alkyl, Cyano, Nitro, R¹⁵R¹⁶N, CF₃ und OCF₃; R¹² Wasserstoff, R²³SO oder R²³SO₂ ist; R¹³ Wasserstoff, R⁴⁴SO oder R⁴⁴SO₂ ist; R¹⁴ Wasserstoff, Halogen, C₁₋₅-Alkoxy, C₁₋₅₋Alkyl, Cyano, Nitro oder R²⁴R²⁵N ist; und G C₃-Alkandiyl ist, fakultativ substituiert mit Hydroxy, (L)-C₁₋₅-Alkyloxy- oder (L)-C₁₋₅-Alkylamino.

3. Verwendung nach Anspruch 2, **dadurch gekennzeichnet, daß** Ar Phenyl ist.

4. Verwendung nach Anspruch 1, **dadurch gekennzeichnet, daß** besagte Verbindung ausgewählt ist aus:
1-[4-(2-Amino-6-chlor-phenyl)-piperazin-1-yl]-3-[5-methansulfonyl-3-(4-trifluormethyl-phenyl)-4,5,6,7-tetrahydro-pyrazolo[4,3-c]pyridin-1-yl]propan-2-ol;
1-[3-Chlor-2-(4-{3-[5-methansulfonyl-3-(4-trifluormethyl-phenyl)-4,5,6,7-tetrahydro-pyrazolo[4,3-c]pyridin-1-yl]-propyl}-piperazin-1-yl)-phenyl]-3-methyl-harnstoff;
1-[3-Chlor-2-(4-{2-hydroxy-3-[5-methansulfonyl-3-(4-trifluormethyl-phenyl)-4,5,6,7-tetrahydro-pyrazolo[4,3-c]pyridin-1-yl]-propyl}-piperazin-1-yl)-phenyl]-3-methylharnstoff;
3-Amino-2-(4-{2-hydroxy-3-[5-methansulfonyl-3-(4-trifluormethyl-phenyl)-4,5,6,7-tetrahydro-pyrazolo [4,3-c]pyridin-1-yl]-propyl} -piperazin-1-yl)-benzoesäuremethylester;
3-Chlor-2-(4-{3-[5-methansulfonyl-3-(4-trifluormethyl-phenyl)-4,5,6,7-tetrahydro-pyrazolo[4,3-c]pyridin-1-yl]-propyl} -piperazin-1-yl)-phenylamin;
1-[2-(4-{3-[3-(4-Brom-phenyl)-5-methansulfonyl-4,5,6,7-tetrahydro-pyrazolo[4,3-c]pyridin-1-yl]-2-hydroxy-propyl} -piperazin-1-yl)-3-chlor-phenyl]-3-methylharnstoff;
und 1-{3-[4-(2-Chlor-6-methansulfonylamino-phenyl)-piperazin-1-yl]-propyl}-3-(4-trifluormethyl-phenyl)-1,4,6,7-tetrahydro-pyrazolo[4,3-c]pyridin-5-carbonsäureamid.

5. Verwendung nach Anspruch 1, **dadurch gekennzeichnet, daß** besagte Verbindung ausgewählt ist aus:
[3-Chlor-2-(4-{3-[5-methansulfonyl-3-(4-trifluormethyl-phenyl)-4,5,6,7-tetrahydro-pyrazolo[4,3-c]pyridin-1-yl]-propyl} -piperazin-1-yl)-phenyl]-carbamidsäuremethylester;
1-[3-(4-Benzo [d]isothiazol-3-yl-piperazin-1-yl)-propyl]-3-(4-brom-phenyl)-1,4,6,7-tetrahydro-pyrazolo [4,3-c]pyridin-5-carbonsäure;
2-(4- {3-[5-Acetyl-3-(4-trifluormethyl-phenyl)-4,5,6,7-tetrahydro-pyrazolo[4,3-c]pyridin-1-yl]-2-hydroxy-propyl}-piperazin-1-yl)-3-nitro-benzoesäuremethylester;
1-[4-(2-Chlor-6-nitro-phenyl)-piperazin-1-yl]-3-[5-methansulfonyl-3-(4-trifluormethyl-phenyl)-4,5,6,7-tetrahydro-pyrazolo[4,3-c]pyridin-l-yl]-propan-2-ol;
2-(4-{2-Hydroxy-3-[3-(4-iod-phenyl)-5-methansulfonyl-4,5,6,7-tetrahydro-pyrazolo [4,3-c]pyridin-1-yl]-propyl}-piperazin-1-yl)-benzonitril;
3-(4-Brom-phenyl)-1-{3-[4-(2-nitro-phenyl)-piperazin-1-yl]-propyl}-1,4,6,7-tetrahydro-pyrazolo [4,3-c]pyridin-5-carbonsäureamid;
2-(4- {3-[5-Acetyl-3-(4-iod-phenyl)-4,5,6,7-tetrahydro-pyrazolo[4,3c]pyridin-1-yl]-2-hydroxy-propyl}-piperazin-1-yl)-benzonitril;
2-(4-{3-[3-(4-Chlor-3-methyl-phenyl)-5-methansulfonyl-4,5,6,7-tetrahydro-pyrazolo[4,3-c]pyridin-1-yl]-2-hydroxy-propyl}-piperazin-1-yl)-benzonitril;
1-(3-(4-Chlor-3-methyl-phenyl)-1-{3-[4-(2,4-dimethyl-phenyl)-piperazin-1-yl]-2-hydroxy-propyl}-1,4,6,7-tetrahydro-pyrazolo[4,3-c]pyridin-5-yl)-ethanon;
1-{3-[4-(3,5-Dichlor-pyridin-4-yl)-piperazin-1-yl]-propyl}-5-methansulfonyl-3-(4-trifluormethyl-phenyl)-4,5,6,7-tetrahydro-1H-pyrazolo[4,3-c]pyridin;
2-(4-{3-[5-Methansulfonyl-3-(4-trifluormethyl-phenyl)-4,5,6,7-tetrahydro-pyrazolo[4,3-c]pyridin-1-yl]-propyl}-piperazin-1-yl)-benzonitril;
N-[3-Chlor-2-(4- {3-[5-methansulfonyl-3-(4-trifluormethyl-phenyl)-4,5,6,7-tetrahydro-pyrazolo[4,3-c]pyridin-1-yl]-propyl} -piperazin-1-yl)-phenyl]-methansulfonamid;
3-(3,4-Dichlor-phenyl)-1-{3-[4-(2-nitro-phenyl)-piperazin-I-yl]-propyl]-1,4,6,7-tetrahydro-pyrazolo[4,3-c]pyridin-5-carbonsäureamid;
und 3-(4-Chlor-3-methyl-phenyl)-1-{3-[4-(2-cyano-phenyl)-piperazin-1-yl]-2-hydroxy-propyl}-1,4,6,7-tetrahydro-pyrazolo[4,3-c]pyridin-5-carbonsäureamid.

6. Verwendung nach Anspruch 1, **dadurch gekennzeichnet, daß** besagte Verbindung ausgewählt ist aus:
1-(3-(4-Chlor-phenyl)-1-{3-[4-(2-fluor-phenyl)-piperazin-1-yl]-propyl}-1,4,6,7-tetrahydro-pyrazolo[4,3-c]pyridin-5-yl)-ethanon;
1- {3-(4-Chlor-phenyl)-1-[2-hydroxy-3-(4-o-tolyl-piperazin-1-yl)-propyl]-1,4,6,7-tetrahydro-pyrazolo [4,3-c]pyridin-5-yl}-ethanon;
1-{3-(4-Chlor-phenyl)-1-[2-methoxy-3-(4-o-tolyl-piperazin-1-yl)-propyl]-1,4,6,7-tetrahydro-pyrazolo[4,3-c]pyridin-5-yl}-ethanon;
1-[1-{2-Hydroxy-3-[4-(2-hydroxy-phenyl)-piperazin-1-yl]-propyl}-3 -(4-iod-phenyl)-1,4,6,7-tetrahydro-pyrazolo [4,3-c]pyridin-5-yl]-ethanon;
1-[1-[2-Hydroxy-3-(4-o-tolyl-piperazin-1-yl)-propyl]-3-(4-trifluormethyl-phenyl)-1,4,6,7-tetrahydro-pyrazolo [4,3-c]pyridin-5-yl]-ethanon;
2-(4- {3-[5-Acetyl-3-(4-trifluormethyl-phenyl)-4,5,6,7-tetrahydro-pyrazolo[4,3c]pyridin-1-yl]-2-hydroxy-propyl) -piperazin-1-yl)-benzonitril;
1-[3-(3,4-Dichlor-phenyl)-pyrazol-1-yl]-3-(4-o-tolyl-piperazin-1-yl)-propan-2-ol;
1-[1-[2-(2-Piperazin-1-yl-ethylamino)-3-(4-o-tolyl-piperazin-1-yl)-propyl]-3-(4-trifluormethyl-phenyl)-1,4,6,7-tetrahydro-pyrazolo[4,3-c]pyridin-5-yl]-ethanon;
1-{3-[4-(2-Cyano-phenyl)-piperazin-1-yl]-2-hydroxy-propyl} -3-(4-iod-phenyl)-1,4,6,7-tetrahydro-pyrazolo[4,3-c]pyridin-5-carbonsäure-tert-butylester;
1-{3-4-(2-Cyano-phenyl)-piperazin-1-yl]-2-hydroxy-propyl}-3-(4-iod-phenyl)-1,4,6,7-tetrahydro-pyrazolo[4,3-c]pyridin-5-carbonsäureamid;
Carbamidsäure-1-[5-carbamoyl-3-(4-iod-phenyl)-4,5,6,7-tetrahydro-pyrazolo[4,3-c]pyridin-1-ylmethyl]-2-[4-(2-cyano-phenyl)-piperazin-1-yl]-ethylester;
1- { 3-(3-Amino-4-chlor-phenyl)-1-[2-hydroxy-3-(4-o-tolyl-piperazin-1-yl)-propyl]-1,4,6,7-tetrahydro-pyrazolo [4,3-c]pyridin-5-yl }-ethanon;
(R)-1-(3-(4-Brom-phenyl)-1-{3-[4-(5-chlor-2-methyl-phenyl)-piperazin-1-yl]-2-hydroxy-propyl} -1,4,6,7-tetrahydro-pyrazolo [4,3 -c]pyridin-5 -yl)-ethanon;
2-(4-{3-[5-Acetyl-3-(4-trifluormethyl-phenyl)-4,5,6,7-tetrahydro-pyrazolo [4,3c]pyridin-1-yl]-2-fluor-propyl}-piperazin-1-yl)-benzonitril;
(3-(4-Chlor-3-methyl-phenyl)-1-{3- [4-(2-cyano-phenyl)-piperazin-1-yl]-2-hydroxypropyl}-1,4,6,7-tetrahydro-pyrazolo [4,3-c]pyridin-5-yl)-oxo-essigsäuremethylester;
5-Methansulfonyl-1-{3-[4-(2-nitro-phenyl)-piperazin-1-yl]-propyl}-3-(4-trifluormethyl-phenyl)-4,5,6,7-tetrahydro-1H-pyrazolo [4,3-c]pyridin;
1-[3-Chlor-2-(4-{ 3-[5-methansulfonyl-3-(4-trifluormethyl-phenyl)-4,5,6,7-tetrahydro-pyrazolo [4,3-c]pyridin-1-yl]-propyl }-piperazin-1-yl)-phenyl]-harnstoff;
1-{ 3-[4-(2-Chlor-6-methansulfonylamino-phenyl)-piperazin-1-yl]-propyl -3-(4-trifluormethyl-phenyl)-1,4,6,7-tetrahydro-pyrazolo[4,3-c]pyridin-5-sulfonsäureamid; N-[3-Chlor-2-(4-{2-hydroxy-3-[5-methansulfonyl-3-(4-trifluoromethyl-phenyl)-4,5,6,7-tetrahydro-pyrazolo [4,3-c]pyridin-1-yl]-propyl }-piperazin-1-yl)-phenyl]-methansulfonamid;
1-[4-(2,6-Dinitro-phenyl)-piperazin-1-yl]-3-[5-methansulfonyl-3-(trifluormethylphenyl)-4,5,6,7-tetrahydro-pyrazolo [4,3-c]pyridin-1-yl]-propan-2-ol;
2-(4-{2-Hydroxy-3-[5-methansulfonyl-3-(4-trifluormethyl-phenyl)-4,5,6,7-tetrahydro-pyrazolo[4,3-c]pyridin-1-yl]-propyl}-piperazin-1-yl)-3-methansulfonylamino-benzoesäuremethylester;
1-{3-[4-(1,1-Dioxo-1H-1|6-benzo[d]isothiazol-3-yl)-piperazin-1-yl]-propyl}-5 - methansulfonyl-3-(4-trifluormethyl-phenyl)-4,5,6,7-tetrahydro-1H-pyrazolo[4,3-c]pyridin;
1-[1-{ 3-[4-(6-Chlor-benzothiazol-2-yl)-piperazin-1-yl]-2-hydroxy-propyl -3 -(4-trifluormethyl-phenyl)-1,4,6,7-tetrahydro-pyrazolo [4,3-c]pyridin-5-yl]-ethanon;
und 1-[1-[3-(4-Benzo[d]isoxazol-3-yl-piperazin-1-yl)-2-hydroxy-propyl]-3-(4-trifluormethyl-phenyl)-1,4,6,7-tetrahydro-pyrazolo [4,3-c]pyridin-5-yl]-ethanon.

7. Verwendung nach Anspruch 1, **dadurch gekennzeichnet, daß** besagte Verbindung ausgewählt ist aus:
N-[3-Chlor-2-(4-{2-hydroxy-3-[5-methansulfonyl-3-(4-trifluormethyl-phenyl)-4,5,6,7-tetrahydro-pyrazolo[4,3-c]pyridin-1-yl]-propyl}-piperazin-1-yl)-phenyl]-methansulfonamid;
1-[3-(4-Benzo[d]isothiazol-3-yl-piperazin-1-yl)-propyl]-3-(4-brom-phenyl)-1,4,6,7-tetrahydro-pyrazolo[4,3-c]pyridin-5-carbonsäureamid; und
1-[3-Chlor-2-(4-{2-hydroxy-3-[5-methansulfonyl-3-(4-trifluormethylphenyl)-4,5,6,7-tetrahydro-pyrazolo[4,3-c]pyridin-1-yl]-propyl}-piperazin-1-yl)-phenyl]-3-methylharnstoff.

8. Verwendung nach Anspruch 1, **dadurch gekennzeichnet, daß** besagte pharmazeutische Zusammensetzung in einer Dosierungsmenge formuliert wird, die geeignet ist zur Behandlung eines allergischen Zustandes.

## Revendications

1. Utilisation d'un composé dans la fabrication d'une composition pharmaceutique destinée à traiter un sujet atteint d'un état allergique, ledit composé répondant à la formule (I) ci-dessous : 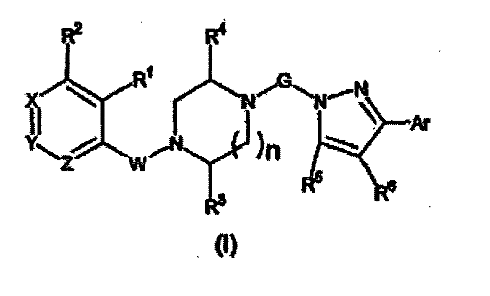 dans laquelle :
R¹ est un atome d'hydrogène, un groupe azido, halogène, alcoxy en C_{1 à 5}, hydroxy, alkyle en C_{1 à 5}, alcényle en C_{2 à 5}, cyano, nitro, R⁷R⁸N, acyle en C_{2 à 8}, R⁹OC=O, R¹⁰R¹¹NC=O, ou R¹⁰R¹¹NSO₂ ; ou R¹ est pris conjointement avec W comme décrit ci-dessous ;
R² est un atome d'hydrogène, d'halogène, un groupe alcoxy en C_{1 à 5}, alkyle en C_{1 à 5}, alcényle en C_{2 à 5}, halogénoalkyle en C_{1 à 5}, cyano ou R⁴⁶R⁴⁹ N*;* en variante, R¹ et R² peuvent être pris ensemble pour former un cycle carboxylique ou hétérocyclique à 5 à 7 chaînons facultativement substitué, lequel cycle peut être insaturé ou aromatique ;
chacun de R³ et R⁴ est indépendamment un atome d'hydrogène ou un groupe alkyle en C_{1 à 5} ;
chacun de R⁵ et R⁶ est indépendamment un atome d'hydrogène, un groupe alkyle en C_{1 à 5}, alcényle en C₂ à 5,, alcoxy C_{1 à 5}, alkylthio en C_{1 à 5}, halogène, ou carbocyclyle ou hétérocyclyle à 4 à 7 chaînons ;
en variante, R⁵ et R⁶ peuvent être pris ensemble pour former un cycle carboxylique ou hétérocyclique à 5 à 7 chaînons facultativement substitué, lequel cycle peut être insaturé ou aromatique, et peut être facultativement substitué par entre un et trois substituants indépendamment choisis parmi un groupe halogéno, cyano, amino, nitro, R⁴⁰, R⁴⁰O-, R⁴⁰S-, R⁴⁰O(alkylène en C_{1 à 5})-, R⁴⁰O (C=O) -, R⁴⁰(C=O) -, R⁴⁰(C=S)-, R⁴⁰ (C=O) O-, R⁴⁰O(C=O) (C=O) **-.** R⁴⁰SO_{2'} NHR⁶²( C=NH)-, NHR⁸²SO₂-, et NHR⁶² ( C=O)-;
R⁴⁰ est H, un groupe alkyle en C_{1 à 5}, alcényle en C_{2 à 6}, phényle, benzyle, phénéthyle, hétérocyclyle en C_{1 à 5}, (hétérocyclyle en C_{1 à 5}) alkylène en C_{1 à 5}, amino, ou mono- ou di (alkyle en C_{1 à 5})amino, ou R⁶⁸OR⁶⁹-, dans laquelle R⁶⁸ est H, un groupe alkyle en C_{1 à 5}, alcényle en C_{2 à 5}, phényle, benzyle, phénéthyle, hétérocyclyle en C_{1 à 5}, ou (hétérocyclyle en C_{1 à 5}) alkylène en C_{1 à 5} et R⁶⁹ est un groupe alkylène en C_{1 à 5}, phénylène, ou hétérocyclyle en C_{1 à 5} divalent ; et
R⁶² peut être H en plus des valeurs pour R⁴⁰ ;
R⁷ est un atome d'hydrogène, un groupe alkyle en C_{1 à 5}, alcényle en C_{3 à 5}, phényle, naphtyle, hétérocyclyle en C_{1 à 5}, acyle en C_{2 à 8}, aroyle, R²⁷ OC=O, R²⁸R²⁹NC=O, R²⁷ SO, R²⁷SO₂ ou R²⁸R²⁹NSO₂ ;
R⁸ est un atome d'hydrogène, un groupe alkyle en C_{1 à 5}, alcényle en C_{3 à 5}, phényle ou hétérocyclyle en C_{1 à 5} ; en variante, R⁷ et R⁸ peuvent être pris ensemble pour former un cycle hétérocyclique à 4 à 7 chaînons facultativement substitué, cycle qui peut être saturé, insaturé ou aromatique ;
R⁹ est un groupe alkyle en C_{1 à 5}, phényle, naphtyle, ou hétérocyclyle en C_{1 à 5} ;
R²¹ est un atome d'hydrogène, un groupe alkyle en C_{1 à 5}, alcényle en C_{3 à 5}, phényle, naphtyle, hétérocyclyle en C₁ à ₅, acyle en C_{2 à 6}, aroyle, R⁹⁰OC=O, R³¹R³²NC=O, R³⁰SO, R³⁰SO₂, ou R³¹R³²NSO₂ ;
R²² est un atome d'hydrogène, un groupe alkyle en C_{1 à 5}, alcényle en C_{3 à 5}, phényle, ou hétérocyclyle en C₁ à ₅ ; en variante, R²¹ et R²² peuvent être pris ensemble pour former un cycle hétérocyclique à 4 à 7 chaînons facultativement substitué, cycle qui peut être saturé, insaturé ou aromatique ;
chacun de R²³, R²⁶, R²⁷, R³⁰, R³³, R⁴⁴, R⁴⁵ et R⁵⁰ est un groupe alkyle en C_{1 à 5}, phényle, naphtyle ou hétérocyclyle en C_{1 à 5} ;
R²⁴ est un atome d'hydrogène, un groupe alkyle en C_{1 à 5}, alcényle en C_{3 à 5}, phényle, naphtyle, hétérocyclyle en C₁ à ₅, acyle en C_{2 à 8}, aroyle, R³³OC=O, R³⁴R³⁵NC=O, R³³SO, R³³SO₂, ou R³⁴R³⁵NSO₂,
R²⁵ est un atome d'hydrogène, un groupe alkyle en C_{1 à 5}, alcényle en C_{3 à 5}, phényle ou hétérocyclyle en C_{1 à 5} ; en variante, R²⁴ et R²⁵ peuvent être pris ensemble pour former un cycle hétérocyclique à 4 à 7 chaînons facultativement substitué, cycle qui peut être saturé, insaturé ou aromatique ;
chacun de R¹⁰ et R¹¹ est indépendamment un atome d'hydrogène, un groupe alkyle en C_{1 à 5}, alcényle en C_{2 à 5}, phényle ou hétérocyclyle en C_{1 à 5} ; en variante, R¹⁰ et R¹¹ peuvent être pris ensemble pour former un cycle hétérocyclique à 4 à 7 chaînons facultativement substitué, cycle qui peut être saturé, insaturé ou aromatique ;
chacun de R²⁸, R²⁹, R³¹, R³², R³⁴, R³⁵, R⁴⁶, R⁴⁷, R⁵¹ et R⁵² est indépendamment un atome d'hydrogène, un groupe alkyle en C_{1 à 5}, phényle, ou hétérocyclyle en C_{1 à 5} ; en variante, R²⁸ et R²⁹, R³¹ et R³² R³⁴ et R³⁵, R⁴⁶ et R⁴⁷, ou R⁵¹ et R⁵², indépendamment, peuvent être pris ensemble pour former un cycle hétérocyclique à 4 à 7 chaînons facultativement substitué, cycle qui peut être saturé, insaturé ou aromatique ;
n vaut 1 ou 2 ;
G représente un groupe alcènediyle en C_{3 à 6} ou alcanediyle en C_{3 à 6}, facultativement substitué par un groupe hydroxy, halogène, alkyle en C_{1 à 5}, alcoxy en C_{1 à 5}, oxo, hydroximino, CO₂R⁸⁰, R⁶⁰R⁶¹NCO₂, (L)-alkylène en C_{1 à 4}, (L) -alcoxy en C_{1 à 5}, N₃, ou [(L)-alkylène] en C_{1 à 5} amino ;
chacun de R⁶⁰ et R⁶¹ est indépendamment un atome d'hydrogène, un groupe alkyle en C_{1 à 5}, alcényle en C_{3 à 5}, phényle, benzyle, phénéthyle ou hétérocyclyle en C_{1 à 5} ; en variante R⁶⁰ et R⁶¹ peuvent être pris ensemble pour former un cycle hétérocyclique à 4 à 7 chaînons facultativement substitué, cycle qui peut être saturé, insaturé ou aromatique ;
L est un groupe amino, mono- ou di-(alkyle en C_{1 à 5}) amino, pyrrolidinyle, morpholinyle, pipéridinyle, homopipéridinyle ou pipérazinyle, ou les atomes d'azote disponibles du cycle peuvent être facultativement substitués par un groupe alkyle en C_{1 à 5}, benzyle, acyle en C_{2 à 5}, alkylsulfonyle en C_{1 à 5} ou alkyloxycarbonyle en C_{1 à 5} ;
X est un atome d'azote ou R¹²C ;
Y est un atome d'azote ou R¹³C;
Z est un atome d'azote ou R¹⁴C;
R¹² est un atome d'hydrogène, d'halogène, un groupe alcoxy en C_{1 à 5}, alkyle en C_{1 à 5}, alcényle en C_{2 à 5}, cyano, nitro, R²¹R²²N, acyle en C_{2 à 8}, haloalkyle en C_{1 à 5}, hétérocyclyle en C_{1 à 5}, (hétérocyclyl en C_{1 à 5})alkylène en C_{1 à 5}, R²³ OC=O, R²³O(C=O)NH-, R²³SO, R²²NHCO-, R²² NH (C=O) NH-, R²³ (alkylène en C_{1 à 4}) NHCO-, R²³SO₂ ou R²³ SO₂NH- ;
R¹³ est un atome d'hydrogène, d'halogène, un groupe alcoxy en C_{1 à 5,} alkyle en C_{1 à 5,} alcényle en C_{2 à 5}, cyano, nitro, R⁴²R⁴³ N, acyle en C_{2 à 8}, halogénoalkyle en C_{1 à 5,} hétérocyclyle en C_{1 à 5}, (hétérocyclyle en C_{1 à 5})alkylène en C_{1 à 5}, R⁴⁴OC=O, R⁴⁴O(C=O)NH-, R⁴⁴SO, R⁴³NHCO-, R⁴³NH (C=O) NH-, R⁴⁴ (alkylène en C_{1 à 4}) NHCO-, R⁴⁴SO₂ ou R⁴⁴ SO₂NH- ;
R¹⁴ est un atome d'hydrogène, d'halogène, un groupe alcoxy en C_{1 à 5}, alkyle en C_{1 à 5}, alcényle en C_{2 à 5}, cyano, nitro, R²⁴ R²⁵N, acyle en C_{2 à 8}, halogénoalkyle en C_{1 à 5}, hétérocyclyle en C_{1 à 5}, (hétérocyclyl en C_{1 à 5}) alkylène en C_{1 à 5}, R²⁶OC=O, R²⁸O (C=O) NH-, R²⁶SO, R²⁵NHCO-, R²⁵NH (C=O) NH-, R²⁶ (alkylène en C_{1 à 4}) NHCO-, R²⁶SO₂, ou R²⁶SO₂NH- ; en variante, R¹² et R¹³ ou R¹² et R² ou R¹³ et R¹⁴ peuvent être pris ensemble pour former un cycle carboxylique ou hétérocyclique à 5 à 6 chaînons facultativement substitué, cycle qui peut être insaturé ou aromatique ;
Ar représente un cycle aryle ou hétéroaryle monocyclique ou bicyclique, facultativement substitué par entre 1 et 3 substituants choisis parmi un atome d'halogène, un groupe alcoxy en C_{1 à 5}, alkyle en C_{1 à 5}, alcényle en C_{2 à 5}, cyano, azido, nitro, R¹⁵R¹⁶N, R¹⁷SO₂, R¹⁷S, R¹⁷SO, R¹⁷OC=O, R¹⁵R¹⁶NC=O, haloalkyle en C_{1 à 5}, halogénoalcoxy en C_{1 à 5}, halogénoalkylthio en C_{1 à 5}, et alkylthio en C_{1 à 5} ;
R¹⁵ est un atome d'hydrogène, un groupe alkyle en C_{1 à 5}, alcényle en C_{2 à 5}, phényle, benzyle, hétérocyclyle en C_{1 à 5}, acyle en C_{2 à 8}, aroyle, R⁵³OC=O, R⁵⁴R⁵⁵NC=O, R⁵³S, R⁵³SO, R⁵³SO₂, ou R⁵⁴R⁵⁵NSO₂ ;
R¹⁶ est un atome d' hydrogène, un groupe alkyle en C_{1 à 5}, alcényle en C_{2 à 5}, phényle, benzyle ou hétérocyclyle en C_{1 à 5} ; en variante, R¹⁵ et R¹⁶ peuvent être pris ensemble pour former un cycle hétérocyclique à 4 à 7 chaînons facultativement substitué, cycle qui peut être saturé, insaturé ou aromatique ;
chacun de R¹⁷ et R⁵³ est un groupe alkyle en C_{1 à 5}, phényle ou hétérocyclyle en C₁ à ₅ ;
chacun de R⁵⁴ et R⁵⁵ est indépendamment un atome d' hydrogène, un groupe alkyle en C_{1 à 5}, alcényle en C_{2 à 5}, phényle, benzyle ou hétérocyclyle en C_{1 à 5} ; en variante, R⁴⁴ et R⁵⁵ peuvent être pris ensemble pour former un cycle hétérocyclique à 4 à 7 chaînons facultativement substitué, cycle qui peut être saturé, insaturé ou aromatique ;
W représente SO₂, C=O, CHR²⁰, ou une liaison covalente ; ou W et R¹, pris ensemble avec le cycle à 6 chaînons auquel ils sont tous deux attachés, forment l'une des deux formules suivantes : 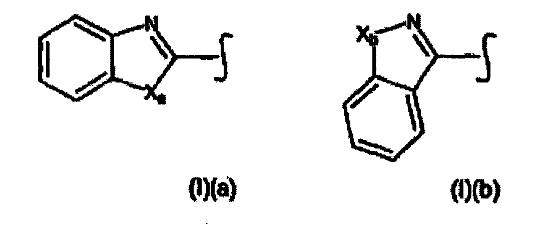 dans lesquelles Xₐ est 0, S ou N ; et X_{b} est 0, S ou SO₂ ;
R²⁰ est un atome d'hydrogène, un groupe alkyle en C_{1 à 5}, phényle, benzyle, naphtyle ou hétérocyclyle en C_{1 à 5};
R⁴² est un atome d'hydrogène, un groupe alkyle en C_{1 à 5}, alcényle en C_{3 à 5}, phényle, naphtyle, hétérocyclyle en C₁ à ₅, acyle en C_{2 à 8}, aroyle, R⁴⁵OC=O, R⁴⁶R⁴⁷NC=O, R⁴⁶SO, R⁴⁵SO₂ ou R⁴⁶R⁴⁷NSO₂;
R⁴³ est un atome d' hydrogène, un groupe alkyle en C_{1 à 5}, alcényle en C_{3 à 5}, phényle ou hétérocyclyle en C₁ à ₅ ; en variante, R⁴² et R⁴³ peuvent être pris ensemble pour former un cycle hétérocyclique à 4 à 7 chaînons facultativement substitué, cycle qui peut être saturé, insaturé ou aromatique ;
R⁴⁴ est un groupe alkyle en C_{1 à 5}, alcényle en C_{2 à 5}, phényle, naphtyle ou hétérocyclyle en C_{1 à 5} ;
R⁴⁸ est un atome d'hydrogène, un groupe alkyle en C_{1 à 5}, alcényle en C_{3 à 5}, phényle, naphtyle, hétérocyclyle en C_{1 à 5,} acyle en C_{2 à 8}, aroyle, R⁵⁰OC=O, R⁵¹R⁵²NC=O, R⁵⁰SO, R⁵⁰SO₂ ou R⁵¹R⁵²NSO₂ ;
R⁴⁹ est un atome d' hydrogène, un groupe alkyle en C_{1 à 5,} alcényle en C_{3 à 5}, phényle ou hétérocyclyle en C_{1 à 5} ; en variante, R⁴⁸ et R⁴⁹ peuvent être pris ensemble pour former un cycle hétérocyclique à 4 à 7 chaînons facultativement substitué, cycle qui peut être saturé, insaturé ou aromatique ; et
dans lesquels chacun des groupes hydrocarbyle et hétérocarbyle ci-dessus, sauf indication contraire, et en plus de tout substituant spécifié, est substitué facultativement et indépendamment par entre 1 et 3 substituants choisis parmi le méthyle, halogénométhyle, hydroxyméthyle, halogéno, hydroxy, amino, nitro, cyano, alkyle en C_{1 à 5}, alcoxy en C_{1 à 5}, -COOH, acyle en C_{2 à 6}, [di (alkyle en C_{1 à 4}) amino] alkylène en C_{2 à 5}, [di (alkyle en C_{1 à 4}) amino] alkyle en C_{2 à 5}-NHCO- et halogénoalcoxy en C_{1 à 5};
ou un sel, ester ou amide pharmaceutiquement acceptable de celui-ci.

2. Utilisation selon la revendication 1, dans laquelle chacun de R³ et R⁴ est un atome d'hydrogène ; Ar représente un cycle à six chaînons, facultativement substitué par entre 1 et 2 substituants choisis parmi un atome d'halogène, un groupe alkyle en C_{1 à 5}, cyano, nitro, R¹⁵R¹⁶N, CF₃ et OCF₃ ; R¹² est un atome d' hydrogène, R²³SO ou R²³ SO₂ ; R¹³ est un atome d' hydrogène, R⁴⁴SO ou R⁴⁴SO₂ ; R¹⁴ est un atome d'hydrogène, d'halogène, un groupe alcoxy en C_{1 à 5}, alkyle en C_{1 à 5}, cyano, nitro ou R²⁴R²⁵N ; et G est un groupe alkanediyle en C₃, facultativement substitué par un groupe hydroxy, (L)-alkyloxy en C_{1 à 5}, ou (L)-alkylamino en C_{1 à 5}.

3. Utilisation selon la revendication 2, dans laquelle Ar est un groupe phényle.

4. Utilisation selon la revendication 1, dans laquelle ledit composé est choisi parmi :
le 1-[4-(2-amino-6-chloro-phényl)-pipérazin-1-yl]-3-[5-méthanesulfonyl-3-(4-trifluorométhyl-phényl)-4,5,6,7-tétrahydro-pyrazolo[4,3-c]pyridin-1-yl]-propan-2-ol ;
la 1-[3-chloro-2-(4-{3-[5-méthanesulfonyl-3-(4-trifluorométhyl-phényl)-4,5,6,7-tétrahydro-pyrazolo[4,3-c]pyridin-1-yl]-propyl}-pipérazin-1-yl)-phényl]-3-méthyl-urée ;
la 1-[3-chloro-2-(4-(2-hydroxy-3-[5-méthane-sulfonyl-3-(4-trifluorométhyl-phényl)-4,5,6,7-tétrahydro-pyrazolo[4,3-c]pyridin-1-yl]-propyl}-pipérazin-1-yl)-phényl]-3-méthyl-urée ;
l'ester de méthyle d'acide 3-amino-2-(4-{2-hydroxy-3-[5-méthanesulfonyl-3-(4-trifluorométhylphényl)-4,5,6,7-tétrahydro-pyrazolo[4,3-c]pyridin-1-yl]-propyl}-pipérazin-1-yl)benzoïque ;
la 3-chloro-2-(4-{3-[5-méthanesulfonyl-3-(4-trifluorométhyl-phényl)-4,5,6,7-tétrahydro-pyrazolo[4,3-c]-pyridin-1-yl]-propyl}-pipérazin-1-yl)-phénylamine ;
la 1-[2-(4-{3-[3-(4-bromo-phényl)-5-méthanesulfonyl-4,5,6,7-tétrahydro-pyrazolo[4,3-c]pyridin-1-yl]-2-hydroxy-propyl}-pipérazin-1-yl)-3-chloro-phényl]-3-méthyl-urée ;
et l'amide d'acide 1-{3-[4-(2-chloro-6-méthanesulfonylamino-phényl)-pipérazin-1-yl]-propyl}-3-(4-trifluorométhyl-phényl)-1,4,6,7-tétrahydro-pyrazolo[4,3-c]pyridine-5-carboxylique.

5. Utilisation selon la revendication 1, dans laquelle ledit composé est choisi parmi :
l'ester de méthyl d'acide [3-chloro-2-(4-{3-[5-méthanesulfonyl-3-(4-trifluorométhyl-phényl)-4,5,6,7-tétrahydro-pyrazolo[4,3-c]pyridin-1-yl]-propyl}-pipérazin-1 yl)-phényl]-carbamique ;
l'amide d'acide 1-[3-(4-benzo[d]isothiazol-3-yl-pipérazin-1-yl)-propyl]-3-(4-bromophényl)-1,4,6,7 tétrahydro-pyrazolo[4,3-c]pyridine-5-carboxylique ;
l'ester de méthyle d'acide 2-(4-{3-[5-acétyl-3-(4-trifluorométhyl-phényl)-4,5,6,7-tétrahydro-pyrazolo[4,3-c]pyridin-1-yl]-2-hydroxy-propyl}-pipérazin-1-yl)-3-nitro-benzoïque ;
le 1-[4-(2-chloro-6-nitro-phényl)-pipérazin-1-yl]-3-[5-méthanesulfonyl-3-(4-trifluorométhyl-phényl)-4,5,6,7-tétrahydro-pyrazolo[4,3-c]pyrdin-1-yl]-propan-2-ol ;
le 2-(4-{2-Hydroxy-3-[3-(4-iodo-phényl)-5-méthanesulfonyl-4,5,6,7-tétrahydro-pyrazolo[4,3-c]pyridin-1-yl]-propyl}-pipérazin-1-yl)-benzonitrile ;
l'amide d'acide 3-(4-bromo-phényl)-1-{3-[4-(2-nitro-phényl)-pipérazin-1-yl]-propyl}-1,4,6,7-tétrahydro-pyrazolo[4,3-c]pyridine-5-carboxylique ;
le 2-(4-{3-[5-acétyl-3-(4-iodo-phényl)-4,5,6,7-tétrahydro-pyrazolo[4,3-c]pyridin-1-yl]-2-hydroxy-propyl}-pipérazin-1-yl)-benzonitrile ;
le 2-(4-{3-[3-(4-chloro-3-méthyl-phényl)-5-méthanesulfonyl-4,5,6,7-tétrahydro-pyrazolo[4,3-c]pyridin-1-yl]-2-hydroxy-propyl}-pipérazin-1-yl)-benzonitrile ;
la 1-(3-(4-chloro-3-méthyl-phényl)-1-{3-[4-(2,4-diméthyl-phényl)-pipérazin-1-yl]-2-hydroxy-propyl}-1,4,6,7-tétrahydro-pyrazolo[4,3-c]pyridin-5-yl)-éthanone ;
la 1-{3-[4-(3,5-dichloro-pyridin-4-yl)-pipérazin-1-yl]-propyl)-5-méthanesulfonyl-3-(4-trifluorométhyl-phényl)-4,5,6,7-tétrahydro-1H-pyrazolo[4,3-c]pyridine ;
le 2-(4-(3-[5-méthanesulfonyl-3-(4-trifluo-rométhyl-phényl)-4,5,6,7-tétrahydro-pyrazolo[4,3-c]pyridin-1-yl]-propyl}-pipérazin-1-yl)-benzonitrile ;
le N-[3-chloro-2-(4-{3-[5-méthanesulfonyl-3-(4-trifluorométhyl-phényl)-4,5,6,7-tétrahydro-pyrazolo [4,3-c]pyridin-1-yl]-propyl}-pipérazin-1-yl)-phényl]-méthanesulfonamide ;
l'amide d'acide 3-(3,4-dichloro-phényl)-1-{3-[4-(2-nitro-phényl)-pipérazin-1-yl]-propyl}-1,4,6,7 tétrahydro-pyrazolo[4,3-c]pyridine-5-carboxylique ;
et l'amide d'acide 3-(4-chloro-3-méthyl-phényl)-1-{3-[4-(2-cyano-phényl)-pipérazin-1-yl]-2-hydroxy-propyl}-1,4,6,7-tétrahydro-pyrazolo[4,3-c]pyridine-5-carboxylique.

6. Utilisation selon la revendication 1, dans laquelle ledit composé est choisi parmi :
la 1-(3-(4-chloro-phényl)-1-{3-[4-(2-fluoro-phényl)-pipérazin-1-yl]-propyl}-1,4,6,7-tétrahydro-pyrazolo[4,3-c]pyridin-5-yl)-éthanone ;
la 1-{3-(4-chloro-phényl)-1-[2-hydroxy-3-(4-o-tolyl-pipérazin-1-yl-propyl]-1,4,6,7-tétrahydro-pyrazolo[4,3-c]pyridin-5-yl}-éthanone ;
la 1-{3-(4-chloro-phényl)-1-[2-méthoxy-3-(4-o-tolyl-pipérazin-1-yl)-propyl]-1,4,6,7-tétrahydro-pyrazolo[4,3-c]pyridin-5-yl}-éthanone ;
la 1-[1-{2-hydroxy-3[4-(2-hydroxy-phényl)-pipérazin-1-yl]-propyl}-3-(4-iodophényl)-1,4,6,7-tétrahydro-pyrazolo[4,3-c]pyridin-5-yl]-éthanone ;
la 1-[1-[2-hydroxy-3-(4-o-tolyl-pipérazin-1-yl)-propyl]-3-(4-trifluorométhyl-phényl)-1,4,6,7-tétrahydro-pyrazolo[4,3-c]pyridin-5-yl]-éthanone ;
le 2-(4-{3-[5-acétyl-3-(4-trifluorométhyl-phényl)-4,5,6,7-tétrahydro-pyrazolo[4,3-c]pyridin-1-yl]-2-hydroxy-propyl}-pipérazin-1-yl)-benzonitrile ;
le 1-[3-(3,4-dichloro-phényl)-pyrazol-1-yl]-3-(4-0-tolyl-pipérazin-1-yl)-propan-2-ol ;
la 1-[1-[2-(2-pipérazin-1-yl-éthylamino)-3-(4-o-tolyl-pipérazin-1-yl)-propyl]-3-(4-trifluorométhyl-phényl)-1,4,6,7-tétrahydro-pyrazolo[4,3-c]pyridin-5-yl]-éthanone ;
l'ester de tert-butyle d'acide 1-{3-[4-(2-cyano-phényl)-pipérazin-1-yl]-2-hydroxy-propyl}-3-(4-iodo-phényl)-1,4,6,7-tétrahydro-pyrazolo[4,3-c]pyridine-5-carboxylique ;
l'amide d'acide 1-{3-[4-(2-cyano-phényl)-pipérazin-1-yl]-2-hydroxy-propyl}-3-(4-iodo-phényl)-1,4,6,7-tétrahydro-pyrazolo[4,3-c]pyridine-5-carboxylique ;
l'ester de 1-[5-carbamoyl-3-(4-iodo-phényl)-4,5,6,7-tétrahydropyrazolo[4,3-c]pyridin-1-ylméthyl]-2-[4-(2-cyano-phényl)-pipérazin-1-yl]-éthyle d'acide carbamique,
la 1-{3-(3-amino-4-chloro-phényl)-1-[2-hydroxy-3-(4-o-tolyl-pipérazin-1-yl)-propyl]-1,4,6,7-tétrahydro-pyrazolo[4,3-c]pyridin-5-yl}-éthanone ;
la (*R*)-1-(3-(4-bromo-phényl)-1-{3-[4-(5-chloro-2-méthyl-phényl)-pipérazin-1-yl]-2-hydroxy-propyl}-1,4,6,7-tétrahydro-pyrazolo[4,3-c]pyridin-5-yl)-éthanone ;
le 2-(4-{3-[5-acétyl-3-(4-trifluorométhyl-phényl)-4,5,6,7-tétrahydro-pyrazolo[4,3-c]pyridin-1-yl]-2 fluoro-propyl}-pipérazin-1-yl)-benzonitrile ;
l'ester de méthyle d'acide (3-(4-chloro-3-méthyl-phényl)-1-{3-[4-(2-cyano-phényl)-pipérazin-1-yl]-2-hydroxy-propyl}-1,4,6,7-tétrahydro-pyrazolo[4,3-c]pyridin-5-yl)-oxo-acétique ;
la 5-méthanesulfonyl-1-{3-[4-(2-nitro-phényl)-pipérazin-1-yl]-propyl}-3-(4-trifluorométhyl-phényl)-4,5,6,7-tétrahydro-1H-pyrazolo[4,3-c]pyridine ;
la 1-[3-chloro-2-(4-{3-[5-méthanesulfonyl-3-(4 trifluorométhyl-phényl)-4,5,6,7-tétrahydro-pyrazolo[4,3-c]pyridin-1-yl]-propyl}-pipérazin-1-yl)-phényl]-urée ;
l'amide d'acide 1-{3-[4-(2-chloro-6-méthane-sulfonylamino-phényl)-pipérazin-1-yl]-propyl}-3-[4-trifluorométhyl-phényl]-1,4,6,7-tétrahydro-pyrazolo[4,3-c]pyridine-5-sulfonique ;
le N-[3-chloro-2-(4-{2-hydroxy-3-[5-méthanesulfonyl-3-(4-trifluorométhyl-phényl)-4,5,6,7-tétrahydro-pyrazolo[4,3-c)pyridin-1-yl]-propyl}-pipérazin-1-yl)-phényl]-méthanesulfonamide ;
le 1-[4-(2,6-dinitro-phényl)-pipérazin-1-yl]-3-[5-méthanesulfonyl-3-(4-trifluorométhyl-phényl)-4,5,6,7-tétrahydro-pyrazolo[4,3-c]pyridin-1 -yl]-propan-2-ol ;
l'ester de méthyle d'acide 2-(4{2-hydroxy-3-[5-méthanesulfonyl-3-(4-trifluorométhyl-phényl)-4,5,6,7-tétrahydro-pyrazolo[4,3-c]pyridin-1-yl]-propyl}-pipérazin-1-yl)-3-méthanesulfonylamino-benzoïque,
la 1-{3-[4-(1,1-dioxo-1H-116-benzo[d]isothiazol-3-yl)-pipérazin-1-yl]-propyl}-5-méthanesulfonyl-3-(4-trifluorométhyl-phényl)-4,5,6,7-tétrahydro-1H-pyrazolo[4,3-c]pyridine ;
la 1-[1-{3-[4-(6-chloro-benzothiazol-2-yl)-pipérazin-1-yl]-2-hydroxy-propyl}-3-(4-trifluorométhyl-phényl)-1,4,6,7-tétrahydro-pyrazolo[4,3-c]pyridin-5-yl]-éthanone ; et
la 1-[1-[3-(4-benzo[d]isoxazol-3-yl-pipérazin-1-yl)-2-hydroxy-propyl]-3-(4-trifluorométhyl-phényl)-1,4,6,7-tétrahydro-pyrazolo[4,3-c]pyridin-5-yl]-éthanone.

7. Utilisation selon la revendication 1, dans laquelle ledit composé est choisi parmi :
le N-[3-chloro-2-(4-{2-hydroxy-3-[5-méthanesulfonyl-3-(4-trifluorométhyl-phényl)-4,5,6,7-tétrahydro-pyrazolo[4,3-c]pyridin-1-yl]-propyl}-pipérazin-1-yl)-phényl]-méthanesulfonamide ;
l'amide d'acide 1-[3-(4-benzo[d]isothiazol-3-yl-pipérazin-1-yl)-propyl]-3-(4-bromo-phényl)-1,4,6,7-tétrahydro-pyrazolo[4,3-c]pyridine-5-carboxylique ; et
la 1-[3-chloro-2-(4-(2-hydroxy-3-[5-méthanesulfonyl-3-(4-trifluorométhyl-phényl)-4,5,6,7-tétrahydro-pyrazolo[4,3-c]pyridin-1-yl]-propyl}-pipérazin-1-yl)-phényl]-3-méthyl-urée.

8. Utilisation selon la revendication 1, dans laquelle ladite composition pharmaceutique est formulée en une quantité posologique appropriée pour le traitement d'un état allergique.
